**Europäisches Patentamt**

**European Patent Office**

**Office européen des brevets**

(19)

(11) Veröffentlichungsnummer: **0 004 258**
**B1**

(12) **EUROPÄISCHE PATENTSCHRIFT**

(45) Veröffentlichungstag der Patentschrift: **03.02.82**

(51) Int. Cl.³: **G 06 F 15/20, A 61 B 6/02**

(21) Anmeldenummer: **78100622.6**

(22) Anmeldetag: **07.08.78**

(54) **Computer-Tomograph zur Durchstrahlung eines Körpers.**

(30) Priorität: **15.08.77 US 824632**

(43) Veröffentlichungstag der Anmeldung:
**03.10.79 Patentblatt 79/20**

(45) Bekanntmachung des Hinweises auf die Patenterteilung:
**03.02.82 Patentblatt 82/5**

(84) Benannte Vertragsstaaten:
**DE FR GB**

(56) Entgegenhaltungen:
**US - A - 4 038 551**
**US - A - 4 066 903**
**Electromedica Siemens (Erlangen) Nr. 3/4 1977,
Jass und anderen "Somatom-ein schneller
ganzkörper-Computertomograph mit
unmittelbarer Bildwiedergabe", Seiten 118 bis
122**

**IEEE Transactions on Computers, März 1977,
New York, Wang "Cross section reconstruction
with a Fan-Beam scanning geometry" Seiten
264 bis 268**

(73) Patentinhaber: **SIEMENS AKTIENGESELLSCHAFT
Berlin und München
Postfach 22 02 61
D-8000 München 22 (DE)**

(72) Erfinder: **Gordon, Bernard M.
136 Hesperus Avenue
Magnolia Massachusetts 09130/USA (US)**
Erfinder: **Neumann, Leopold
26 Winchester Drive
Lexington Massachusetts 02173/USA (US)**
Erfinder: **Dobbs, John Mc.G.
188 Mystic Valley Parkway
Arlington Massachusetts 02174/USA (US)**

(56) Entgegenhaltungen:
**Medical Physics, Vol. 4, Nr. 2 März/April 1977,
New York, McCullough "X-ray transmission
computed tomography", Seiten 85 bis 98**

Courier Press, Leamington Spa, England.

**0 004 258**

## Computer-Tomograph zur Durchstrahlung eines Körpers

Die Erfindung bezieht sich auf einen Computer-Tomographen zur Durchstrahlung eines Körpers für die Erzeugung eines Querschnittsbildes aus Bilddaten, die repräsentativ für die Dichte des Körpers in einer Querschnittsebene sind, mit einer Quelle für die Erzeugung der den Körper durchdringenden Strahlung, einem Detektor zum Empfangen der Strahlung, die in der betreffenden Querschnittsebene durch den Körper längs einer Vielzahl von Pfaden verläuft, und zur Bildung von für die empfangene Strahlungsintensität repräsentativen Ausgangssignalen, einer Einrichtung zur gemeinsamen Rotation der Strahlenquelle und des Detektors in bezug auf den Körper und zur periodischen Aussendung der Strahlung während der Rotation für die Abtastung unter einer Vielzahl von Projektionen mit einer entsprechenden Vielzahl von Projektionswinkeln, sowie einem Tomographieprozessor zur Bildung eines Bildes der durchstrahlten Schicht aus den Ausgangssignalen des Detektors, bestehend aus einem Korrektor zur Korrektur der Ausgangssignale des Detektors für die Bildung von korrigierten Daten, die repräsentativ für Linienintegrale der Dichte des Körpers längs der von der Strahlung durchsetzten Pfade sind, einem ersten Speicher für die Speicherung der korrigierten Daten und zum Zwecke der Weiterverarbeitung der Daten zu einem späteren Zeitpunkt, einem Faltungsrechner für die Faltung der korrigierten Daten mit einer die Bildschärfe erhöhenden Funktion, um eine Vielzahl von gefalteten Datenpunkten zu erzeugen, einem zweiten Speicher, der mit dem Faltungsrechner verbunden ist, um die gefalteten Datenpunkte zu speichern für die Verarbeitung derselben zu einem späteren Zeitpunkt, einem Rekonstruktor, der mit dem zweiten Speicher gekoppelt ist und dessen Daten so verarbeitet, daß die gefalteten Datenpunkte der Projektionen durch eine entsprechende Zuordnung zu ihren Plätzen im Querschnittsbild sowie durch Bewichtung mit einem vorgegebenen Bewichtungsfaktor mit nachfolgender Aufsummierung in einem Summationskreis rückprojiziert werden, sowie einem Bildspeicher mit einer Vielzahl von Speicherplätzen, die mit einer entsprechenden Vielzahl von Bereichen des Körpers in der entsprechenden Querschnittsebene übereinstimmt und in denen die rückprojizierten Datenpunkte abgespeichert werden.

Die Entwicklung von mathematischen Verfahren für die Rekonstruktion von Bildern der inneren Struktur eines Körpers geht zurück bis zum Beginn dieses Jahrhunderts. Im allgemeinen beinhalten tomographische Systeme eine Strahlungsquelle, die Röntgenstrahlung oder andere Strahlung durch einen Teil eines Körpers sendet und einen oder mehr Sensoren, die die durch den Körper gedrungene Strahlung empfangen und ein Signal bilden, das repräsentativ für die empfangene Strahlungsintensität ist. Die Quelle und die zugeordneten Sensoren sind relativ zum Körper bewegbar, um eine Mehrzahl von Projektionen während einer Abtastung zu ermöglichen und dabei Daten in den verschiedenen Projektionen zu erzeugen. Verschiedene Techniken der Bildrekonstruktion sind vorgeschlagen worden, durch die Daten der Projektionen in ein Endbild umgewandelt werden. Solche Techniken haben iterative algebraische Rekonstruktion, Fouriertransformation und Faltung benutzt.

Während der letzten Jahre ist großes Interesse an der Computer-Tomographie entstanden, bei der ein Digitalcomputer zur Bildrekonstruktion benutzt wird. Die gegenwärtigen Tomographiesysteme beinhalten im allgemeinen kostenaufwendige, für allgemeine Zwecke vorgesehene Computer für das Rekonstruktionsverfahren und arbeiten aus einer Anzahl von Gründen nicht vollkommen zufriedenstellend, z.B. wegen der Kosten, einer beschränkten Auflösung und des Erfordernisses einer langen Zeitperiode, die nach der Vervollständigung einer Abtastung vor der Fertigstellung eines rekonstruierten Bildes vergeht. Um die große Anzahl von komplexen Rechenvorgängen durchzuführen, die zur Erzeugung eines endgültigen Bildes erforderlich sind, ist ein hochentwickelter Hochgeschwindigkeitscomputer erforderlich, aber selbst mit solch einem Computer ist eine beachtliche Zeit für die Erzeugung des endgültigen Bildes erforderlich.

Einige tomographische Verarbeitungssysteme weisen separate, spezielle Prozessoren auf, die durch einen zentralen Computer gesteuert sind, um eine oder mehrere der Korrektur-, Faltungs- und Bildrekonstruktionsfunktionen auszuüben. Jedoch sind in diesen Systemen die speziellen Prozessoren unabhängig voneinander und die Daten zwischen diesen speziellen Prozessoren werden über den zentralen Computer übertragen, welcher jeden der Prozessoren steuert und mit denen jeder der Prozessoren verbunden ist. Diese Konfiguration reduziert etwas die Komplexität des Computers, aber die Geschwindigkeit des Systems ist beschränkt durch die Eingangs/Ausgangsgeschwindigkeit des Computers, und für ein Hochgeschwindigkeitssystem ist nach wie vor ein teurer Hochgeschwindigkeitscomputer erforderlich.

Um die Zeit zu reduzieren, die zur Erzeugung eines endgültigen Bildes erforderlich ist, oder um den Rechneraufwand zu reduzieren, sind Näherungen in dem Algorithmus vorgenommen worden, der in den oben beschriebenen Systemen angewandt wird, damit die Rechenzeit reduziert wird. Jedoch haben diese Näherungen zu schlechteren als den optimalen Bildresultaten geführt.

In der US - A - 40 38 551 ist ein Computer-Tomograph der eingangs genannten Art beschrieben, bei dem die Bilddaten sämtlicher Projektionen erst abgespeichert und dann insgesamt dem Faltungsvorgang unterzogen werden, so daß dem Bildspeicher gleich diejenigen Bilddatenpunkte angeliefert werden, aus denen sich das endgültige Computerbild zusammensetzt. Aufgrund dieses Verarbeitungsprinzipes muß die Datenverarbeitungskapazität der verschiedenen Prozessorstufen groß sein.

2

**0 004 258**

Der vorliegenden Erfindung liegt die Aufgabe zugrunde, den bekannten Computer-Tomographen der gattungsgemäßen Art bezüglich der Verarbeitungsweise seiner Daten dahingehend zu verändern, daß die verschiedenen Prozessorstufen viel einfacher aufgebaut werden können und eine geringere Kapazität bezüglich der aufzunehmenden und zu verarbeitenden Daten benötigen.

Diese Aufgabe ist erfindungsgemäß dadurch gelöst, daß der Korrektor, Faltungsrechner und Rekonstruktor sowie die als Zwischenspeicher ausgebildeten Speicher des Tomographieprozessors mit einer Steuereinrichtung verbunden sind, welche die Prozessorstufen so steuert, daß jede Stufe die ankommenden Daten einer Projektion sofort verarbeitet und nach ihrer Verarbeitung der jeweils nachfolgenden Prozessorstufe zuführt, und daß der Summationskreis zwei Eingänge besitzt, von denen der erste Eingang mit dem zweiten Speicher und der zweite Eingang mit dem Bildspeicher in Verbindung steht, derart, daß der Summationskreis die vom zweiten Speicher angelieferten Datenpunkte einer Projektion mit den im Bildspeicher bereits vorher abgespeicherten Datenpunkten aufsummiert und die auf diese Weise aufsummierten Datenpunkte den Dateneingängen des Bildspeichers zuführt, in welchem jeweils diese Datenpunkte die vorher abgespeicherten Datenpunkte ersetzen.

Bei dem erfindungsgemäßen Computer-Tomographen werden also die einzelnen Prozessorstufen durch die betreffende Steuereinrichtung so gesteuert, daß jede Stufe die ankommenden Daten einer Projektion sofort verarbeitet und nach ihrer Verarbeitung der jeweils nachfolgenden Prozessorstufe zuführt. Die verschiedenen Prozessorstufen brauchen demgemäß im Vergleich zum Stand der Technik eine relativ geringe Datenverarbeitungskapazität zu besitzen. Das System beinhaltet zur Durchführung der erforderlichen Rechenvorgänge spezielle Hochgeschwindigkeitsverarbeitungsstufen, die mit einem Computer niedriger Kosten zusammenarbeiten, der das System steuert. Diese Verarbeitungsstufen bilden einen mehrstufigen Pipeline-Prozessor, der geeignet ist, die Korrektur-, Faltungs-, Interpolations- und Rückprojektionsfunktionen durchzuführen, die erforderlich sind, um das gewünschte endgültige Bild zu rekonstruieren. Die Erfindung macht Gebrauch von einem Rekonstruktions-Algorithmus, der speziell dem beschriebenen und beanspruchten Gerät angepaßt ist.

Kurz ausgedrückt, empfängt das Datenerfassungs- und Verarbeitungssystem digitale Daten, die die Intensität einer Röntgenstrahlung oder anderen Strahlung die auf einem Detektor-Array auftrifft, repräsentieren und führt gewisse Korrekturen durch, um korrigierte Daten zu erzeugen, die für das Integral über die Schwächungskoeffizienten (Dichte) längs der betrachteten Strahlenwege im Körper repräsentativ sind. Die korrigierten Daten werden dann gefaltet zur Vorbereitung der Rückprojektionsoperation. Darauf werden die Daten jeder Projektion vorinterpoliert, um der Notwendigkeit, Interpolationswerte für jedes individuelle Bildelement (Pixel) des endgültigen Bildes berechnen zu müssen, vorzubeugen. Dann wird eine Koordinatentransformation durchgeführt, aus der sehr einfache Rechenvorgänge für das Rückprojektionsverfahren resultieren. Die Rückprojektion wird durch Selektion von geeigneten Daten für jedes Bildelement des endgültigen Bildes und Multiplikation dieser Daten mit einem Bewichtungsfaktor durchgeführt. In dem transformierten Koordinatensystem sind die Selektions- und Bewichtungsprozesse unabhängig vom Projektionswinkel. Das endgültige Bild wird in einem Speicher mit einem Speicherplatz für jedes Pixel gespeichert. Jeder Platz in dem Bildspeicher wird mit den entsprechenden Daten während jeder Projektion beaufschlagt. Der Endwert ist die Summe aller Datenbeiträge, die während jeder Projektion erzeugt werden.

Die Steuerung des Systems wird durchgeführt durch einen relativ einfachen und billigen Steuerrechner. Es sind Vorkehrungen getroffen, um die Korrektur- und Faltungs- verfahren zu modifizieren, indem gewisse Parameter über den Steuercomputer variiert werden. In einer bevorzugten Ausführungsform wird ein 256×256 Bildelemente-Bild erzeugt, und zwar während der 5 Sekunden-360°-Abtastung. Die Bildberechnung ist um Bruchteile einer Sekunde nach der Abtastung beendet.

Weiterbildungen der Erfindung ergeben sich aus den Unteransprüchen. Die Erfindung ist nachfolgend anhand der Zeichnungen näher erläutert.

Fig. 1 und 1a zeigen den tomographischen Scanner mit seinem Rahmen, seiner Röntgenstrahlenquelle und seinem Detektor-Array;

Fig. 2 ist ein Blockdiagramm des tomographischen Prozessors;

Fig. 3 und 3A zeigen die Datenerfassungsanordnung;

Fig. 4 ist ein Flußdiagramm der Rechenvorgänge in dem Korrektor;

Fig. 5 zeigt das Blockdiagramm des Korrektors;

Fig. 6 ist ein Blockdiagramm des C/C-Speichers und eines Teiles des Faltungskreises;

Fig. 6A ist eine Skizze einer typischen Faltungsfunktion;

Fig. 7 ist ein Blockdiagramm für den Rest des Faltungsrechners und die Interpolationsanordnung.

Fig. 8 zeigt ein Pixel-Array und seine Zuordnung zu dem Röntgen-Fächerstrahl;

Fig. 9 zeigt eine Darstellung zur Erläuterung der Arbeitsweise des Interpolators;

Fig. 10 zeigt die Beziehung zwischen Punkten in dem rekonstruierten Bild und der rotierenden Röntgenstrahlenquelle und dem Detektor-Array in dem Patienten-Koordinaten-System;

Fig. 11 erläutert die Berechnung der Bewichtungs- und Suchfunktionen in dem Projektions-Koordinaten-System;

3

# 0 004 258

Fig. 12 erläutert, wie der Bildspeicher während jeder Projektion gefüllt wird;

Fig. 13 ist ein Blockdiagramm der Bildrekonstruktionseinrichtung;

Fig. 14 zeigt den Koordinaten-Transformationsteil der Bildrekonstruktionseinrichtung;

Fig. 15 zeigt den Koordinatentransformationssteuerkreis gemäß Fig. 14;

Fig. 16 sind Kurven zur Erläuterung der Wirkungsweise des Koordinatentransformationsteils;

Fig. 17 zeigt den Rückprojektionsteil der Bildrekonstruktionseinrichtung;

Fig. 18 zeigt, wie das endgültige Bild für die Berechnung von Bewichtungs- und Suchfunktionen geteilt wird;

Fig. 19 zeigt den Bildspeicher;

Fig. 20 zeigt den Zwischenkreis gemäß Fig. 2.

Detaillierte Beschreibung der bevorzugten Ausführung Beschreibung des Gesamtsystems

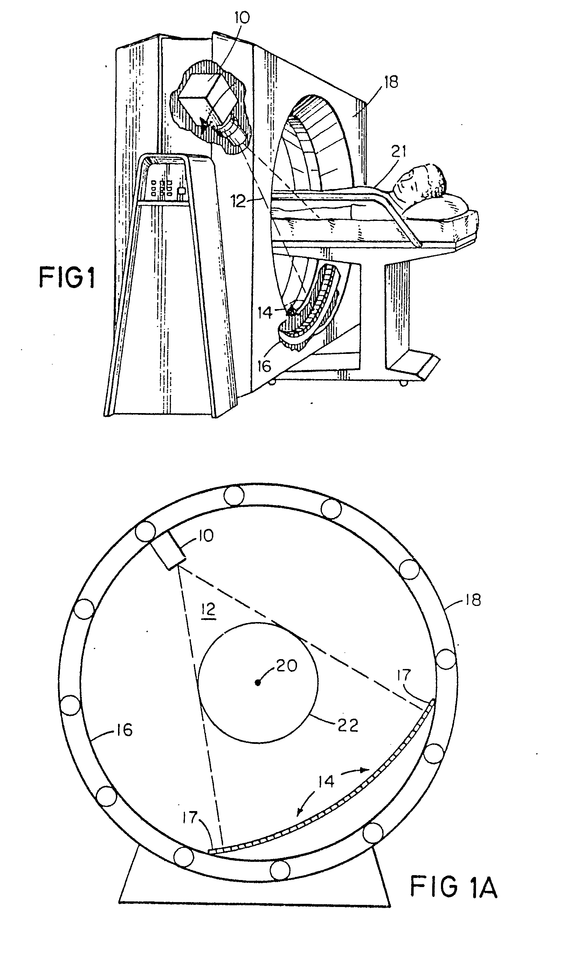

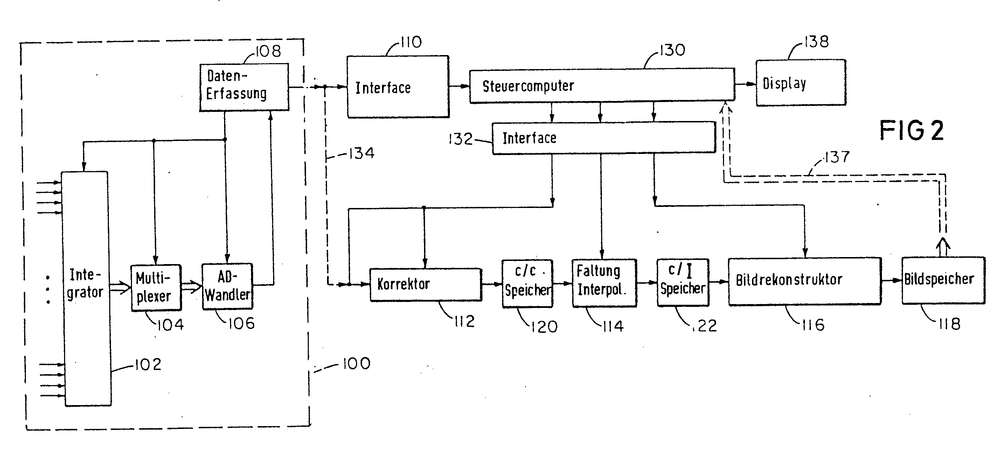

Vor einer Erläuterung der vorliegenden Erfindung im Detail sei das gesamte Tomographie-System mit einer kurzen Zusammenfassung der Funktionen, die von jedem der großen Teile der Datenverarbeitungseinrichtung ausgeführt werden, beschrieben. Die Fig. 1 und 1A zeigen den mechanischen Teil des Systems, in dem der Patient der Röntgenstrahlung ausgesetzt wird, und die Fig. 2 ist ein Blockdiagramm, das die Funktionsteile der Datenverarbeitungselektronik darstellt.

Eine Röntgenröhre 10 oder eine andere Strahlenquelle sendet ein fächerförmiges Röntgenstrahlenbündel 12 aus, welches auf einem Detektor-Array 14 auftrifft. Das Detektor-Array 14 besteht aus einer Vielzahl von in einer Reihe angeordneten Detektoren. Die Detektoren sind auf einem Kreis angeordnet, dessen Mittelpunkt in der Röntgenstrahlenquelle 10 liegt. Bei der bevorzugten Ausführungsform sind 256 Detektoren vorgesehen, welche Daten erzeugen, die zur Bildung des endgültigen Bildes dienen und vier Monitordetektoren 17, von denen jeweils zwei an einem Ende des Detektor-Arrays angeordnet sind, welche ungeschwächte Strahlung der Röntgenstrahlenquelle 12 in der unten noch näher beschriebenen Weise empfangen. Dies geht aus der Fig. 1, welche den Röntgenaufnahmemechanismus zeigt, und aus der Fig. 1A hervor, welche eine schematische Darstellung dieses Mechanismusses ist.

Der Röntgenstrahler 10 und das Detektor-Array 14 sind auf einem inneren Ring 16 einer Rahmenstruktur angeordnet, so daß der Röntgenstrahler 10 und das Detektor-Array 14 in einer festen Zuordnung zueinander gehalten sind. Der innere Ring 16 ist drehbar in einem äußeren Ring 18 angeordnet, so daß der innere Ring 16 mit dem Röntgenstrahler 10 und dem Detektor-Array 14 um eine Achse 20 gedreht werden kann, die senkrecht zu der Ebene des fächerförmigen Röntgenstrahlenbündels 12 liegt. Das fächerförmige Röntgenstrahlenbündel 12 durchstrahlt einen kreisförmigen Bereich, der durch den Kreis 22 definiert ist, wenn der innere Ring 16 des Rahmen um 360° gedreht wird, wobei der Winkel der Durchstrahlung sich während der Rotation des Röntgenstrahlers 10 und des Detektor-Arrays 14 ändert. Der Körper 21, der abgetastet wird, ist in dem Kreis 22 angeordnet, und die Röntgenstrahlen, die den Kreis 22 durchdringen, treffen auf die 256 Datendetektoren des Detektor-Arrays 14 auf. Die Monitordetektoren 17 sind so angeordnet, daß Röntgenstrahlen, die auf ihnen auftreffen, außerhalb des Kreises 22 verlaufen. Auf diese Weise geben die Röntgenstrahlen, die auf den Monitordetektoren 17 auftreffen, einen Hinweis auf die Röntgenstrahlenintensität des Röntgenstrahlers 10 für jede Projektion.

Der Durchstrahlungswinkel des Röntgenstrahlers 10 im Kreis 22 im Hinblick auf den stationären Körper 21 heißt der Projektionswinkel. Durch Aufnahme der Daten der Detektoren im Detektor-Array 14 unter einer Vielzahl von verschiedenen Projektionswinkeln erfolgt eine tomographische Abtastung eines Patienten oder eines anderen Objektes, das im Kreis 22 angeordnet ist, und durch Verarbeitung der Daten der Datendetektoren kann ein Bild berechnet werden, das repräsentativ für die Dichte eines Patienten oder eines anderen Objektes im Querschnittsbereich, der in der Ebene des fächerförmigen Röntgenstrahlenbündels 12 liegt, ist.

Das oben beschriebene Verfahren wird während der Rotation der Röntgenstrahlenquelle und des Detektors um den stationären Patienten mehrmals ausgeführt. Bei der beschriebenen Ausführungsform macht der Rahmen eine komplette Umdrehung in ungefähr fünf Sekunden. Eine Projektion der zu messenden Objektschicht wird bei jedem Grad der Rotation gemacht, so daß 360 separate Sätze mit je 256 Daten bei einer Abtastung erzeugt werden. Diese Daten werden in der unten beschriebenen Weise zur Erzeugung des endgültigen Bildes verarbeitet.

Gemäß Fig. 2 ist die Verarbeitungselektronik in zwei Abschnitte geteilt. Der erste Abschnitt enthält die Datenerfassungselektronik und ist auf dem Rahmen angeordnet, so daß er mit dem Detektor-Array und dem Röntgenstrahler rotiert. Der andere Abschnitt enthält die Verarbeitungselektronik, welche stationär und nicht auf dem Rahmen angeordnet ist. Die Datenerfassungselektronik, die in dem gestrichelten Bereich 100 in Fig. 2 dargestellt ist, sollte so nah wie möglich am Detektor-Array 14 angeordnet werden, um Störsignale und andere Störquellen so gering wie möglich zu halten.

Der Ausgang jedes Detektors im Detektor-Array 14 ist zu einem zugeordneten Integrator 102 geführt. Es sind 256 Datendetektorintegratoren und vier Monitordetektorintegratoren vorhanden. Die Ausgänge der Integratoren sind zu einem Mehrkanal-Analogmultiplexer 104 geführt. Der Multiplexer 104 verbindet selektiv die Integratorausgänge mit einem logarithmischen Analog-Digital-Wandler 106.

4

**0 004 258**

Die Integratoren 102, der Multiplexer 104 und der Analog-Digital-Wandler 106 werden von Signalen von einer Datenerfassungslogik 108 gesteuert. Die digitalen Daten vom Wandler 106, die die Detektorausgangssignale repräsentieren, werden der Logik 108 zugeführt, welche diese Daten über ein Kabel überträgt, das die Rahmenelektronik im Bereich 100 mit den verbleibenden Teilen der Tomographie-Verarbeitungseinrichtung verbindet.

Nachdem die Daten jedes Detektors in digitale Form umgewandelt und übertragen worden sind, werden die verschiedenen Operationen, welche mit diesen Daten durchgeführt werden müssen, um das endgültige Bild zu formen, in einem Hochgeschwindigkeits-Pipelineprozessor vorgenommen. Der Pipelineprozessor besteht aus vier Funktionseinheiten: einem Korrektor 112, einem Faltungsrechner/Interpolator 114, einem Bildrekonstruktionsrechner 116 und einem Bildspeicher 118. Der detaillierte Aufbau jeder dieser Stufen ist in den folgenden Abschnitten beschrieben. Verschiedene dieser Stufen haben selbst eine interne Pipeline-Verarbeitungs-Konfiguration. Durch schrittweise Ausführung der erforderlichen Rechenoperationen in jeder der Pipeline-Stufen ist der Prozessor in der Lage, seine Funktionen in der Zeit der Datenaufnahme auszuführen.

Bei der dargestellten Ausführungsform werden die Daten vom Bereich 100 dem Rahmen/Computerinterface 110 zugeführt. Dieses Interface prüft die Daten vom Bereich 100 bezüglich ihres Formats und speichert sie in den Speicher eines Steuercomputers 130 über einen direkten Speicherzugang ein (DMA). Wenn der Korrektor 112 für neue Daten bereit ist, werden diese Daten dem Korrektor 112 vom Computer 130 über das Computer/Prozessor-interface 132 zugeführt und im Korrektor 112 für die Verarbeitung gespeichert. Der Datentransfer vom Bereich 100 zum Korrektor 112 erfolgt über den Computer 130, um die Speicherung von Rohdaten zu erleichtern, wenn dies gewünscht wird, und um Kalibrierberechnungen zuzulassen, welche durch den Computer 130 entsprechend Kalibrierdaten durchgeführt werden, wie dies unten beschrieben ist. In anderen Einrichtungen, wo es unerwünscht sein kann, daß die Daten vom Bereich 100 durch den Speicher des Computers 130 gehen, können die Daten vom Bereich 100 direkt dem Korrektor 112 zugeführt werden, wie dies durch die gestrichelte Linie 134 dargestellt ist.

Die dem Korrektor 112 zugeführten Daten bestehen aus Digitalwerten, die repräsentativ für den Logarithmus der Detektorausgangssignale sind. Der Korrektor führt mehrere verschiedene Operationen mit diesen Daten aus. Um Variationen in der Detektorempfindlichkeit und Kanalverstärkung, Offsets in der Elektronik und Variationen in der Röntgenstrahlenintensität von Projektion zu Projektion auszugleichen, werden Daten ohne Objekt zwischen Röntgenröhre 12 und Detektor-Array 14 aufgenommen. Aus diesen Daten berechnet der Computer Kalibrierwerte, welche gespeichert und später dem Korrektor über den Zwischenkreis 132 zugeführt werden. Die Detektor-Ausgangsdaten sind nicht eine lineare Funktion der Dichte des Körpers, durch den die Röntgenstrahlen passieren, und zwar wegen des Strahlenaufhärtungseffekts. Deshalb wird eine Aufhärtungskorrektur ebenfalls durch den Korrektor 112 durchgeführt. Schließlich werden die korrigierten Signale mit dem Kosinus des Winkels zwischen dem speziellen Detektor und dem zentralen Detektor multipliziert. Dies ist erforderlich wegen der mathematischen Funktion, die die gemessenen Daten dem endgültigen Bild zuordnet, wie dies im Detail in der folgenden mathematischen Beschreibung beschrieben wird.

Die korrigierten Daten werden in dem C/C-Speicher 120 gespeichert. Die Daten einer Projektion sind, nachdem sie korrigiert und im C/C-Speicher 120 gespeichert sind, für die Verarbeitung im Faltungsrechner 114 zugänglich. Der Faltungsrechner faltet die Serie der korrigierten Eingangsdaten mit einer Verwischungen beseitigenden Funktion, um die Daten für die Rückprojektion vorzubereiten. Die Faltungsfunktion kann durch die Bedienperson variiert werden, und zwar in Abhängigkeit von den Charakteristiken, die das endgültige Bild haben soll. Für diesen Zweck sind im Computer 130 eine Vielzahl von Faltungsfunktionen gespeichert. In Abhängigkeit von der Wahl einer dieser Funktionen durch die Bedienperson überträgt der Computer 130 Daten, die repräsentativ für diese Funktion sind, zu dem Faltungsrechner über den Zwischenkreis 132. Dies erfolgt vor dem Beginn einer tomographischen Abtastung. Die Verwischungen beseitigende Funktion (Faltungsfunktion). die vom Computer zum Faltungsrechner übertragen wird, besteht aus 256 Worten mit je 24 bits.

Das Ausgangssignal des Faltungsrechners besteht aus 256 Datenpunkten. Der Interpolator nimmt diese Information auf und liefert acht Punkte interpolierter Daten von jedem der 256 Datenpunkte. Dies erfolgt mittels sieben zusätzlicher Datenpunkte für jeden der Original-Datenpunkte. Diese zusätzlichen Datenpunkte werden durch lineare Interpolation zwischen jedem der originalen Datenpunkte berechnet.

Die gefalteten und interpolierten Daten werden im C/I-Speicher 122 gespeichert, wo sie für den Bildrekonstruktor 116 zur Verfügung stehen, welcher die Daten von jeder der Aufnahmen in den Bildspeicher 118 rückprojiziert, um das endgültige Bild zu bilden. Bei der vorliegenden Erfindung ist eine neuartige Methode zur Durchführung der Rückprojektion benutzt mit einem Hochgeschwindigkeits-Pipeline-Prozessor zur Ausführung der Rückprojektionsrechnungen in außerordentlich kurzer Zeit, so daß das endgültige Bild unmittelbar nach einem Abtastvorgang vorliegt.

Der Bildrechner erfordert, daß gewisse Konstanten vor der Durchführung der Bildrekonstruktionsrechnungen für jede Abtastung gespeichert oder berechnet werden. In typischer Weise werden diese Projektionskonstanten meistens im Computer 130 gespeichert und von diesem berechnet und dann zum Bildrekonstruktor 116 übertragen.

5

Während jeder Projektion bestimmt der Bildrechner für jeden der Punkte in der 256×256 Bildmatrix die entsprechenden Daten und multipliziert sie mit einer Bewichtungsfunktion. Das Ausgangssignal des Bildrekonstruktors 116 für jede Projektion sind soviel Worte wie Bildpunkte vorhanden sind. Diese Werte werden zu den Teilbilddaten addiert, die vorher im Bildspeicher 118 gespeichert worden sind. Der Bildspeicher wird gefüllt mit 256×256 Worten zu je 16 bits. Nachdem eine Abtastung beendet worden ist, sind die Daten, die im Bildspeicher 118 gespeichert sind, repräsentativ für die Dichte des Querschnittsbereichs, der abgetastet worden ist.

Die Bilddaten vom Bildspeicher 118 können auf verschiedenen Wegen angezeigt werden. Bei einer bevorzugten Ausführungsform werden diese Daten vom Computer 130 gelesen, wie dies durch die gestrichelte Linie 137 gezeigt ist, wobei gewisse Funktionen mit diesen Daten durch den Computer ausgeführt werden können, z.B um den Dynamikbereich der Grauskala im dem Bild zu verkleinern oder zu vergrößern. Der Computer führt dann die bearbeiteten Daten einem Anzeige-Display 138 zu.

Die Daten werden zwischen dem Korrektor, dem Faltungsrechner/Interpolator und den Bildrekonstruktionsstufen des Pipeline-Prozessors mittels zweier Zweibank-Speicher übertragen. Zwischen dem Korrektor und dem Faltungsrechner/Interpolator liegt ein Korrektor/Faltungsrechner-Speicher oder C/C-Speicher 120. Während der Korrektor 112 korrigierte Daten einer Projektion in eine Bank des C/C-Speichers 120 einschreibt, liest der Faltungsrechner 114 Daten der vorhergehenden Projektion von der anderen Bank des C/C-Speichers 120 aus. Während jeder Abtastung werden 256 Worte zu je 15 bits in eine Bank des C/C-Speichers 120 eingeschrieben oder von einer Bank ausgelesen. Zwischen dem Faltungsrechner/Interpolator und dem Bildrekonstruktor 116 liegt ein Faltungsrechner/Bildrekonstruktor-Speicher oder C/I-Speicher 122. Der C/I-Speicher ist ein Zweibankspeicher ähnlich dem C/C-Speicher. Der Interpolator erzeugt acht Zwischenwerte für jeden gefalteten Datenpunkt, und die 18 signifikantesten bits des Resultats jeder Faltung und Interpolation werden jeweils gehalten. Auf diese Weise werden 2048 Worte zu je 16 bits während jeder Projektion in jede der zwei Speicherbänke des C/I-Speichers 112 eingeschrieben und von dort ausgelesen.

Datenerfassungselektronik

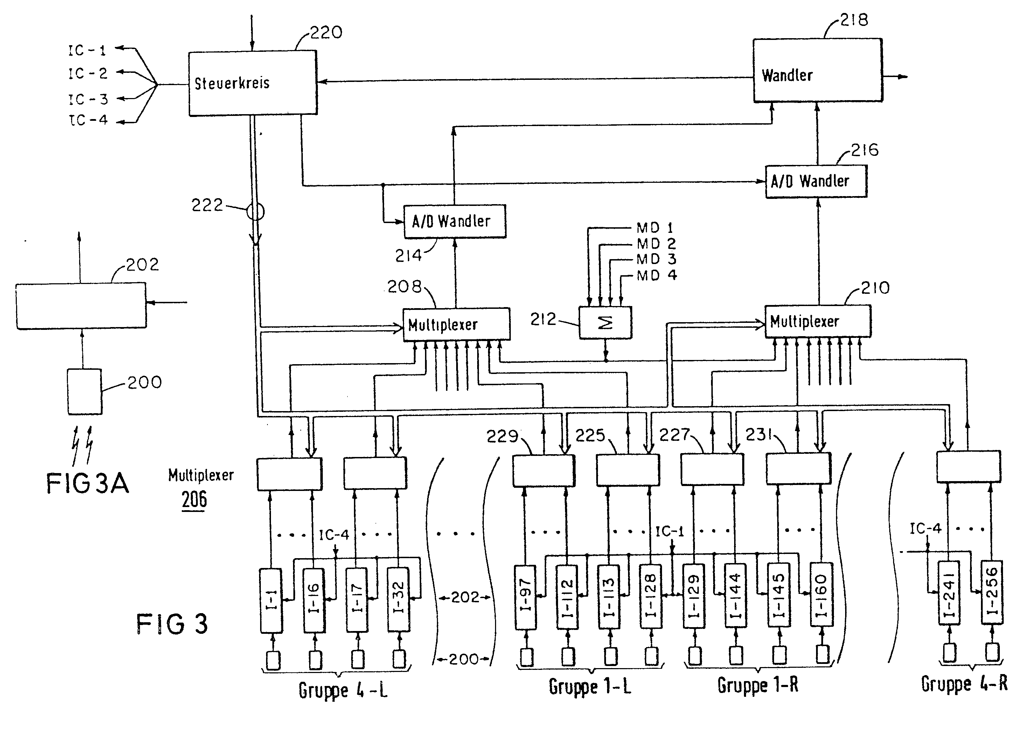

Die 256 Detektoren im Detektor-Array 14 sind mit einem zugeordneten Integrator verbunden. Dies ist im Blockdiagramm in der Fig. 3A gezeigt, wo der Ausgang eines der Detektoren 200 im Detektor-Array 14 mit einem Rückstellintegrator 202 verbunden ist. Ein Integrator-Steuersignal stellt den Integrator 202 vor jedem Puls der Röntgenstrahlenquelle 10 zurück. Während der Zeit, während der der Patient vom fächerförmigen Röntgenstrahlenbündel durchstrahlt wird, wird das Ausgangssignal des Detektors 200 durch den Integrator 202 integriert. Das Ausgangssignal des Integrators 202 wird dem Multiplexerkreis zugeführt, welcher in der Fig. 3 gezeigt und nachfolgend beschrieben ist.

Nachdem eine Röntgenaufnahme beendet ist, wird das integrierte Detektorsignal aus jedem der Integratoren aufeinanderfolgend durch den Multiplexerkreis ausgelesen. Nachdem die Integratoren gelesen sind, setzt das Integratorsteuersignal die Integratoren vor dem nächsten Röntgenstrahlenpuls der Röntgenröhre 10 zurück. Zwischen dem Zeitpunkt, in dem die Daten von den Integratoren 202 gelesen werden und dem Beginn des nächsten Röntgenstrahlenpulses liegt ein Intervall. Die Genauigkeit des Tomographiesystems kann erhöht werden, indem dieses Intervall zur automatischen Eichung der rückstellbaren Integratoren herangezogen wird. Dies ist nachfolgend näher beschrieben.

Die Fig. 3 zeigt den Datenerfassungskreis, der die Daten von jedem der rückstellbaren Integratoren 202 multiplext und verarbeitet. Das Detektor-Array 14 ist in zwei gleiche Abschnitte geteilt, einen rechten und einen linken Abschnitt. Jeder dieser zwei Abschnitte ist weiter in vier Gruppen geteilt, und diese Gruppen seien mit 1-L, 2-L bis 4-L für den linken Abschnitt und 1-R bis 4-R für den rechten Abschnitt bezeichnet. Jede dieser acht Gruppen besteht aus 32 Detektoren, so daß sich 256 Detektoren im Array 14 ergeben. Die ersten Gruppen in jeder der rechten und linken Abschnitte, die Gruppen 1-L und 1-R, sind zu beiden Seiten des Zentrums des Detektor-Arrays 14 angeordnet und die folgenden Gruppen 2-L, 2-R usw. folgen darauf, wobei die Gruppen 4-L und 4-R am linken und rechten Rand des Detektor-Arrays angeordnet sind, jedoch die Monitor-Detektoren ausschließen. Der Grund für diese Ordnung ist nachfolgend beschrieben.

Jedes der Ausgangssignale der rückstellbaren Integratoren 202 ist einer Serie von Multiplexern zugeführt, welche aufeinanderfolgend jeden dieser Ausgänge zwei logarithmischen Analog-Digital-Wandlern zuführen. Diese Multiplex-Operation wird in zwei Niveaus durchgeführt. Im ersten Niveau werden die 128 individuellen Ausgangssignale von jedem der rechten und linken Abschnitte in acht Signale für jeden Abschnitt umgeformt und in dem zweiten Niveau wählen zwei 9- zu 1-Multiplexer eines unter diesen acht Signalen und ein Signal von den Moni-tordetektoren. Jedes dieser Ausgangssignale ist einem logarithmischen A/D-Wandler zugeführt, welcher eine digitale Darstellung des inversen Logarithmus des Signals erzeugt.

Die insgesamt mit 206 bereichneten Multiplexer des ersten Niveaus empfangen die Signale von den rückstellbaren Integratoren 202, die jedem der Detektoren zugeordnet sind. Bei der bevorzugten Ausführungsform gemäß Fig. 3 sind die Multiplexer des ersten Niveaus von 16 Stck. 16-Kanalmultiplexern 206 gebildet. Die acht Ausgänge der Multiplexer des linken Abschnitts sind einem Multiplexer 208 des zweiten Niveaus zugeführt. Analog dazu empfangen acht Multiplexer 206 des ersten Niveaus die Signale von den Integratoren des rechten Abschnitts, und ihre Ausgänge sind einem Multiplexer 210 des zweiten Niveaus zugeführt. Die vier Ausgangssignale der vier Integratoren der Monitor-Detektoren, die mit MD1 bis MD4 bezeichnet sind, sind einem Summationskreis 212 zugeführt, in dem sie kombiniert werden, um ein Signal zu erzeugen, das repräsentativ für die durchschnittliche Intensität der Röntgenstrahlen ist, welche auf den Monitor-Detektoren auftreffen. Der Ausgang des Summationskreises 212 ist sowohl dem Multiplexer 208 des zweiten Niveaus für den linken Abschnitt als auch dem Multiplexer 210 des zweiten Niveaus für den rechten Abschnitt zugeführt. Jeder der Multiplexer 208 und 210 des zweiten Niveaus wählt ein Ausgangssignal von den neun Eingangssignalen.

Das Ausgangssignal von jedem der Multiplexer 208 und 210 des zweiten Niveaus ist zwei logarithmischen A/D-Wandlern 214 und 216 zugeführt. Jeder dieser Wandler formt die aufeinanderfolgenden analogen Signale, die ihm durch die oben beschriebene Multiplexerstruktur zugeführt werden, in eine 15-bit Digitalrepräsentation des Logarithmus des reziproken Analogwertes des Signales um, so daß ein Ausgangssignal erzeugt wird, das proportional der Strahlenschwächung ist.

Die digitalen Ausgänge der zwei logarithmischen Wandler 214 und 216 sind einem Parallel-Serien-Wandler 218 zugeführt. Entsprechend der Bewegung des Rahmens mit dem Detektor-Array 14 und dem Datenerfassungskreis sind zur Übertragung von Signalen von dem rotierenden Rahmen zu dem übrigen Signalverarbeitungskreis, welcher stationär ist, Schleifringe, bewgbare Kabel oder andere Mittel vorgesehen, die die Übertragung der Signale bei der Bewegung des Rahmens erlauben. Für diesen Zweck ist es wünschenswert, die Zahl der aktuellen Signallinien auf einem Minium zu halten. Der Wandler 218 nimmt die digitalen Daten von jedem der logarithmischen A/D-Wandler 214 und 216 auf und wandelt diese Daten in einen einzigen Fluß serieller Daten um, der über ein einziges Kabel vom Rahmen zum Computer 40 und den verbleibenden Teilen der Signalverarbeitungselektronik übertragen wird.

Die Wirkung des oben beschriebenen Datenerfassungskreises wird von einem Multiplexer und einem Integrationssteuerkreis 220 gesteuert. Der Steuerkreis 220 erzeugt vier Integrationssteuersignale, die mit IC-1 bis IC-4 bezeichnet sind. Jedes dieser Integrationssteuersignale wird den entsprechenden linken und rechten Gruppen zugeführt, so daß die Gruppen 1-L und 1-R durch IC-1, die Gruppen 2-L und 2-R durch IC-2 gesteuert werden usw. Die Integrationssteuersignale wählen aus, ob die Integratoren im Integrations-Mode sind, bei dem das Ausgangssignal des entsprechenden Detektors 200 integriert wird oder im automatischen Rücksetzbetrieb, bei dem der Integrator auf Null zurückgesetzt wird, wobei automatisch Drifts, Offsets und andere Fehler in dem Integrator kompensiert werden. Der Steuerkreis 220 führt auch die geeigneten Signale 222 dem ersten und zweiten Niveaumultiplexer zu, um diese zu veranlassen, daß die Integratorausgangssignale den A/D-Wandlern 214 und 216 zugeleitet werden, wie dies unten beschrieben ist.

Der Röntgenstrahlenquelle 10 wird ein Triggerpuls zugeführt, mit dem ein Strahlungspuls ausgelöst wird. Mit der Führungsflanke dieses Pulses schaltet der Steuerkreis 220 jeden der Integratoren 202 von dem automatischen Rücksetzbetrieb in den Integrations-Mode über die Integrator-Steuersignale um. Darauf erzeugt die Röntgenstrahlenquelle 10 einen Puls von Röntgenstrahlen von einer ungefähren Länge von 2 ms. Der Puls des Röntgensteuerkreises endet dann, was bedeutet, daß die Röntgenaufnahme beendet ist, und der Multiplexer- und Integratorsteuerkreis 220 bewirkt darauf, daß die Ausgangssignale der Detektoren 202 in der folgenden Weise verarbeitet werden.

Zunächst bewirkt der Steuerkreis 220, daß die Multiplexer 208 und 210 des zweiten Niveaus das entsprechende Ausgangssignal aus der Gruppe 1-L und 1-R der Multiplexer des ersten Niveaus auswählen. Dann bewirkt der Steuerkreis 220, daß die zu der gesamten Multiplexeranordnung 206 gehörenden Einzel-Multiplexer 225 und 227 der Gruppen 1-L und 1-R aufeinanderfolgend die Ausgänge der Integratoren I-128 bis I-97 und I-129 bis I-160 auswählen. Wenn jeder dieser Integratoren angewählt und die Signale zu dem entsprechenden Wandler 214 oder 216 durch den Multiplexer 208 und 210 geführt sind, werden die Signale in digitale Form umgewandelt und zum Speicher des Computers 120 übertragen.

Wenn jeder der Integratoren in den Gruppen 1-L und 1-R gelesen worden ist, geht das Integrator-Steuersignal IC-1 zurück und bewirkt, daß die Gruppenintegratoren 1-L und 1-R zurückgesetzt werden und daß ein neuer automatischer Rücksetzzyklus beginnt. Darauf werden die Multiplexer 229 und 231 der Multiplexeranordnung 206 befähigt, die Signale von den Integratoren in den Gruppen 2-L und 2-R für eine Umwandlung auszuwählen. Wenn alle der Integratoren in den Gruppen 2-L und 2-R gelesen worden sind, geht das Integrator-Steuersignal IC-2 zurück und bewirkt, daß diese Integratoren einen neuen automatischen Rücksetzzyklus beginnen. Dieser Ablauf wird wiederholt, bis jeder der Integratoren gelesen worden ist und die entsprechenden digitalen Daten zum Computer 120 übertragen worden sind.

Nachdem die Daten von den Meßdetektoren umgewandelt worden sind, führen die Multiplexer 208 und 210 des zweiten Niveaus das Ausgangssignal vom Summationskreis 212, an dem die Monitordetektoren mit den zugehörigen Integratoren angeschlossen sind, den A/D-Wandlern 214 und 216 zu. Ein Umwandlungssignal wird jedem dieser Wandler durch den Steuerkreis 220 zugeführt, und die digitalen Ausgangssignale jedes der Wandler werden zum Computer 120 über einen Parallel-Serien-Wandlerkreis 218 übertragen.

7

**0 004 258**

Die oben beschriebene Datenerfassung arbeitet also derart, daß die Detektoren und Integratoren vom Mittelpunkt des Detektor-Arrays 14 beginnend der Reihe nach bis zu den Rändern des Detektor-Arrays gelesen werden. Trotz Vorsichtsmaßnahmen werden die Integratoren 202 einige geringe Fehler haben, die aus Offsets und Drifts resultieren. Aufgrund der Natur der Integratoren haben einige dieser Fehler die Tendenz, mit der Zeit zuzunehmen. Detektoren, die näher dem Zentrum des Detektor-Arrays liegen, erzeugen Daten, die für den Bildrekonstruktionsprozeß wichtiger sind als die Signale der Detektoren, die näher an den Rändern des Arrays liegen. Indem die Integratorausgänge der Detektoren im Zentrum des Detektor-Arrays zuerst gelesen werden, werden die Integratorfehler der mehr im Zentrum gelegenen Detektoren auf einem Minimum gehalten, woraus ein klareres und schärferes Bild resultiert.

Korrektor

Der Korrektor nimmt die Daten von jedem individuellen Detektor, die in logarithmischer Form im Computer 130 gespeichert sind, auf und führt mit diesen Daten mathematische Operationen durch, welche Fehler korrigieren, die durch Offsets in der Elektronik, Differenzen in der Verstärkung der Detektorkanäle oder Fehler in der Elektronik und durch Strahlaufhärtungseffekte verursacht sind. Der Korrektor multipliziert auch die Daten mit dem Cosinus des Detektorwinkels. Für die Durchführung dieser Korrekturen fragt der Korrektor verschiedene im Computer 130 gespeicherte Datentabellen ab. Eine Offset-Tabelle enthält 256 Worte von Offset-Daten für die 256 Detektoren. Eine Eichtabelle enthält in ähnlicher Weise 256 Worte von Eichfaktoren. Eine Strahlaufhärtungs-Korrekturtabelle mit 96 Worten enthält Daten, die repräsentativ für eine stückweise lineare Annäherung der Strahlaufhärtungs-Korrekturfunktion sind. Die den Korrekturprozessen zu unterwerfenden Detektor-Daten werden von der Datenerfassungselektronik in einer von zwei Datentabellen zu je 258 Worten in dem Computerspeicher gespeichert.

Die Offset-Korrekturtabelle und die Eichtabelle werden durch das tomographische System vor der Ausführung einer tomographischen Abtastung eines zu untersuchenden Körpers bestimmt. Bei der bevorzugten Ausführungsform wird eine komplette Abtastung mit 360 Projektionen ohne Objekt oder mit einem Objekt bekannter, gleichförmiger Dichte in dem Scanner durchgeführt. Für jeden der Detektoren werden die Ausgangssignale, die während jeder Projektion erzeugt werden, summiert und gemittelt. Aufgrund dieser Daten wird ein Eichfaktor für jeden Detektor bestimmt, der die Differenzen in den Empfindlichkeiten der Detektoren und die Differenzen in der Verstärkung der Elektronik jedes Detektorkanals berücksichtigt. Dieser Eichfaktor wird im Hinblick auf die Röntgenstrahlenintensität der Röntgenstrahlenquelle normiert, indem die gemittelten Ausgangssignale jedes der Bilddetektoren durch die gemittelten Ausgangssignale der Monitordetektoren dividiert werden, so daß der aktuelle Wert des Korrekturfaktors gebildet wird.

Die Offset-Eichung zur Erfassung von Fehlern der dem jeweiligen Detektor zugeordneten Integratorschaltung wird durchgeführt, indem eine Präzisionsvorspannung dem Eingang jedes der Integratoren 202 anstelle des Detektor-Ausgangssignals zugeführt wird. Das dem bekannten, präzisen Eingangssignal entsprechende Detektorausgangssignal wird zur Messung des OffsetFehlers in der dem Detektor zugeordneten Elektronik, welcher positiv oder negativ sein kann, verarbeitet. Dieser Offset-Fehler wird in einer Offset-Tabelle gespeichert und zur Korrektur der Daten benutzt, die während einer tomographischen Abtastung aufgenommen werden, wie dies unten näher erläutert ist.

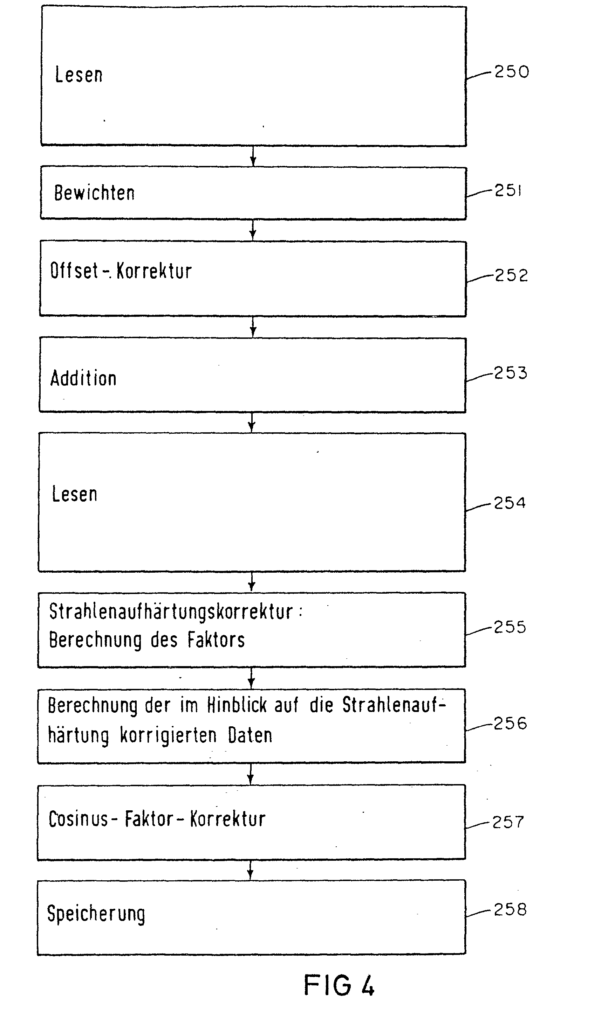

Die Fig. 4 ist ein Flußdiagramm, das die vom Korrektor durchgeführten Operationen zeigt, während er die oben beschriebenen Verfahrensschritte ausführt. Der Korrektor beginnt nicht zu arbeiten, so lange nicht alle Daten von jedem der Detektoren für die erste Projektion in einem der Daten-Puffer in einem Computerspeicher gespeichert sind. Die vom Korrektor durchgeführten Stufen zur Korrektur der Rohdaten des n-ten Detektors sind in der Fig. 4 gezeigt. Am Anfang einer Korrekturprozedur liest der Korrektor (direct memory access DMA) aus dem Computerspeicher aus: Das unkorrigierte Digitalsignal des n-ten Detektors $D_N$, das in logarithmischer Form gespeichert ist, die Offset-Korrektur $OC_N$, welche dem n-ten Detektor entspricht, und den Eichkorrekturfaktor $C_N$ für den n-ten Detektor. Zusätzlich wird für den ersten Detektor in jedem der rechten und linken Abschnitte des Detektor-Arrays ein Korrekturfaktor M, der von den Monitor-Detektordaten abgeleitet und im entsprechenden A/D-Wandler umgewandelt worden ist, gelesen und gespeichert. Da die Monitor-Detektordaten dieselben für alle Datendetektoren während jeder Projektion sind, braucht dieser Wert nur zweimal gelesen zu werden, einmal für jeden Monitor-Detektor. Diese Stufen sind in Block 250 der Fig. 4 gezeigt.

Als nächstes folgt die Offset-Korrektur. Aufgrund der logarithmischen Natur der Daten kann der Offset-Korrekturwert nicht einfach zu den Daten addiert werden. Ermuß mit einem Faktor bewichtet werden, der von der Neigung der logarithmischen Kurve abhängt und so eine Funktion der Größe der Detektor-Ausgangsdaten $D_N$ ist. Bei der bevorzugten Ausführungsform steuern die neun signifikantesten bits der Rohdaten $D_N$ einen 8-bit-Bewichtungsfaktor $SF(D_N)$, gemäß Block 251. Dieser Bewichtungsfaktor wird in einer 512×8-bit-Tabelle in dem Read-Only-Memory (ROM) des Korrektors gespeichert. Die Offset-Korrektur erfolgt, indem der vorher bestimmte Bewichtungsfaktor SF mit dem Offset-Korrekturfaktor für den n-ten Detektor $OC_N$ multipliziert wird, und der zuletzt genannte Offset-Korrekturfaktor zu den rohen Detektordaten addiert wird, um die Offsetkorrigierten Daten $D_{OC}$ gemäß Block 252 zu erzeugen.

8

**0 004 258**

Nach der Offset-Korrektur entsprechen die Daten $D_N$ dem Wert

$$k \ln \frac{U_{REF}}{U_N} ,$$

wobei k ein für alle Kanäle gleicher Faktor ist und $U_N$ die um die Offsets korrigierte Signalspannung am Ausgang des n-ten Integrators. $U_{REF}$ ist eine konstante Referenzspannung. Entsprechend ist der Monitorwert M gleich

$$k \ln \frac{U_{REF}}{U_M} .$$

Bei der Eichung wird nun ohne Objekt der Wert

$$C_N = k \ln \frac{U_M}{U_{NO}}$$

bestimmt, mit dem festgelegt ist, wie groß das Signal im n-ten Kanal ohne Objekt gewesen wäre ($U_{NO}$), wenn als Monitorsignal $U_M$ gemessen wird.

Für die Bildberechnung braucht man

$$K \ln \frac{U_{NO}}{U_N} .$$

Dies ergibt sich aus

$$D_N - M - C_N = k \ln \frac{U_{REF}}{U_N} - k \ln \frac{U_{REF}}{U_M} - k \ln \frac{U_M}{U_{NO}} = k \ln \frac{U_{NO}}{U_N} .$$

Die Operation $D_N - M$ nennt man Normierung der Daten, die Subtraktion von $C_N$ heißt Kalibrierung.

Als nächstes wird die Verstärkungskorrektur zur Erfassung von Fehlern in den Verstärkerschaltungen durchgeführt. Da die gemessenen Daten logarithmisch sind, wird eine Verstärkungskorrektur durchgeführt, indem ein Korrekturwert zu den Offset-korrigierten Detektordaten addiert wird, wobei die Addition zu den Logarithmen der Meßsignale der Multiplikation der Meßsignale mit Faktoren äquivalent ist. Die Monitordaten repräsentieren Variationen in der Intensität der von der Röntgenstrahlenquelle 10 ausgesandten Röntgenstrahlen, welche auf alle Detektoren gleichmäßig einwirken. Der Eichkorrekturfaktor CN für den n-ten Detektor muß zu den Monitordaten M für die jeweilige Projektion addiert werden, um eine Eichkorrektur zu bewirken, die für die Röntgenstrahlenintensität der jeweiligen Projektion normiert ist. (Dies erfolgt wegen der Tatsache, daß die Eichkorrekturdaten $C_N$ durch Division des Eichkorrekturfaktors durch die Monitordaten für den Eich-Scan berechnet worden sind—was gleichwertig ist der Subtraktion des Logarithmus der Monitordaten von dem Eichkorrekturfaktor). Die kalibrierten Daten $D_C$ werden berechnet, indem die Offset-korrigierten Daten zum Eichkorrekturfaktor und den Monitordaten gemäß Block 253 addiert werden.

Der Korrektor führt auch die Strahlenaufhärtungs-Korrektur durch und die Multiplikation mit dem Cosinus des Detektorwinkels. Aufgrund der Tatsache, daß die Strahlenaufhärtungs-Korrektur sich mit verschiedenen Anlagenparametern ändert, wird die Strahlenaufhärtungs-Korrekturfaktortabelle vorteilhafterweise in dem Speicher des Computers 130 gespeichert, so daß diese Tabelle erforderlichenfalls geändert werden kann. Die Strahlenaufhärtungs-Korrekturtabelle bei dem bevorzugten Ausführungsbeispiel ist eine stückweise lineare Annäherung, bei der jedes Segment aus einem Wert und einer Neigung besteht. Auf diese Weise besteht das Strahlenaufhärtungs-Korrektursignal aus einer 10-bit-Größe $V(D_C)$, welche eine Funktion der geeichten Daten ist und 6 bits, die die Neigung des Strahlenaufhärtungs-Korrekturfaktors $SL(D_C)$ als eine Funktion der kalibrierten Daten festlegen. Die Strahlenaufhärtungs-Korrektur wird in 96 Abschnitte geteilt und durch die sieben signifikantesten bits von $D_C$ adressiert. Die Strahlenaufhärtungs-Korrekturfaktoren und der Cosinus des Detektorwinkels für den n-ten Detektor werden aus dem Speicher 128 des Computers durch den Korrektor ausgelesen, wie dies in Block 254 gezeigt ist.

Der Strahlenaufhärtungs-Korrekturfaktor wird berechnet, indem der Neigungsfaktor SL mit den unteren acht bits der kalibrierten Daten, $D_{C(lower)}$, multipliziert wird und indem dieser Wert zu der Größe V des Strahlenaufhärtungs-Korrekturfaktors addiert wird, um den Strahlenaufhärtungs-Korrekturfaktor

9

$C_{BH}$ gemäß Block 255 zu erzeugen. Darauf wird der Strahlenaufhärtungs-Korrekturfaktor von den kalibrierten Daten subtrahiert, um die im Hinblick auf die Strahlenaufhärtung korrigierten Daten $D_{BHC}$ gemäß Block 256 zu erzeugen. Schließlich werden die im Hinblick auf Strahlenaufhärtung korrigierten Daten mit dem Cosinus des Winkels des n-ten Detektors multipliziert, um die Ausgangsdaten des Korrektors gemäß Block 257 zu erzeugen. Die korrigierten Daten werden dann in der n-ten Adresse des C/I-Speichers 120 gemäß Block 258 gespeichert.

Der Korrektor kann auf verschiedene Arten ausgeführt sein. Wenn nur ein Satz von Korrekturen pro Projektion ausgeführt zu werden braucht, muß das oben beschriebene Verfahren nur 256 mal während jeder Projektion wiederholt werden und in einigen Anwendungen kann der Computer 130 in der Lage sein, alle Korrekturrechnungen in der erforderlichen Zeit durchzuführen. (Man beachte, daß im Gegensatz dazu der Faltungsrechner und der Bildrekonstruktor 256 mal 256 individuelle Operationen pro Projektion ausführen müssen, von denen jede aus vielen arithmetischen Stufen besteht.) Alternativ kann der Korrektor als separater Block ausgeführt sein, der besonders ausgebildet ist, um die oben beschriebenen Operationen auszuführen. Bei der bevorzugten Ausführungsform ist die letztere Alternativ gewählt und der Korrektor ist von einem eigenen Prozessorsystem gebildet, das programmiert ist, um die oben beschriebenen Rechenvorgänge auszuführen.

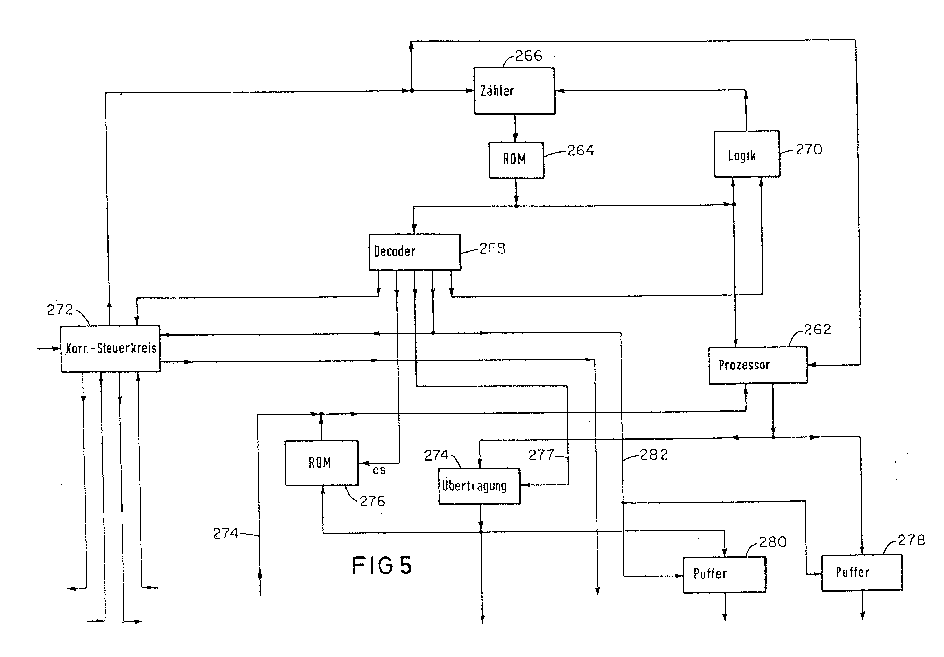

Fig. 5 ist ein Blockdiagramm des Korrekturkreises, der bei der bevorzugten Ausführungsform benutzt ist. Die Rechenvorgänge, die vom Korrektor ausgeführt werden und die Steuerfunktionen für den verbleibenden Korrekturkreis werden in einem Prozessor 262 durchgeführt. Die Funktionen des Prozessors 262 können durch verschiedene Typen von digitalen Datenverarbeitungseinrichtungen ausgeübt werden, einschließlich von Mikroprozessoren. Bei der bevorzugten ausführungsform besteht der Prozessor 262 aus bipolaren, stückweise integrierten Kreisen, die einen 16-bit Hochgeschwindigkeitsmikroprozessor bilden.

Die digitalen Daten, die die Programmschritte festlegen, welche vom Prozessor 262 bei der Ausführung seiner Rechnungen gemäß Fig. 4 gemacht werden, sind in einem ROM 264 gespeichert. In typischer Weise kann das ROM 264 fünf 256 mal 4 bit ROM's enthalten. Die Adresseneingänge zum ROM 264 werden von einem 8-bit-Programmzähler 266 geliefert. Entsprechend den Daten im Programmzähler 266, welche einen Adressenplatz kennzeichnen, erzeugt das ROM 264 Daten, welche den Programmeingängen des Prozessors 262 zugeführt werden, um den Prozessor zu veranlassen, die gewünschten Operationen auszuführen. 3 bits der Ausgangsdaten des ROM 264 werden einem einsaus-acht-Decoder 268 zugeführt. An ausgewählten Punkten des Programms erzeugt der Decoder 268 entsprechend den Ausgangssignalen des ROM 264 eines von verschiedenen Steuersignalen, die unten näher erläutert sind.

Das Verfahren, das in Verbindung mit Fig. 4 beschrieben ist, und das der Prozessor 262 ausübt, erfordert nur wenige Zweige in dem Programm. Diese Verzweigungspunkte können gebildet werden, indem die geeigneten Adressendaten den vorgewählten Eingängen des Programmzählers 266 zugeführt werden und indem der Zähler entsprechend diesem Wert zu den entsprechenden Zeiten voreingestellt wird. Dies ist erreicht, indem die Daten vom ROM 264 und die Signale vom Decoder 268 der Sprunglogik 270 zugeführt werden. Entsprechend den Signalen vom ROM 264, die anzeigen, daß ein Sprung zu einer vorbestimmten Adresse durchgeführt werden soll, führt die Sprunglogik 270 die entsprechenden Eingangssignale den Vorwähleingängen des Programmzählers 266 zu und stellt dann den Zähler auf diese Adresse ein.

Für den Beginn einer Operation des Korrektors veranlaßt das Zwischenglied (Interface) 132 ein Korrektursteuersignal, von einem niedrigen zu einem hohen Niveau überzugehen.

Dementsprechend führt der Korrektursteuerkreis 272 dem Programmzähler 266 und dem Prozessor 262 ein Zeitsignal zu. Nachdem die Korrektur aller Daten einer Projektion beendet worden ist, wird ein Signal für die letzte Stufe des Decoders 268 erzeugt, welches dem Korrektorsteuerkreis 272 zugeführt wird. Dementsprechend sendet der Korrektorsteuerkreis 272 ein Korrektur-beendet-Signal dem Zwischenglied 132 zu und löscht das Zeitsignal des Programmzählers 266 und des Prozessors 262. Entsprechend dem Korrektur-beendet-Signal schaltet das Zwischenglied 133 das Korrektursteuersignal auf einen niedrigen Wert zurück.

Für das Lesen des Detektorausgangs und der Korrekturdaten des Computers 130 führt der Prozessor 262 Daten einer Übertragungsschaltung 274 zu, die repräsentativ für die Adressen dieser Daten im Computer 132 sind. Diese Daten werden in die Übertragungsschaltung 274 durch ein Zeitsignal des Decoders 268 eingegeben. Der Korrektorsteuerkreis 272 sendet dann ein DMA-Anforderungssignal zum Computer 130 über das Zwischenglied 132, das die Zeitsignale zum Programmzähler 266 und zum Prozessor 262 unterbricht, während der Korrektor auf die Daten wartet. Entsprechend der DMA-Anforderung speisen der Computer 130 und das Zwischenglied 132 die geeigneten Daten auf Leitungen 274, die zu den Dateneingängen des Prozessors 262 führen und überlagern dem Korrektorsteuersignal ein DMA-erledigt-Signal. Der Korrektorsteuerkreis 272 führt dann Zeitsignale auf den Programmzähler 266 und den Prozessor 262 zurück, damit der Korrektor seine Tätigkeit wieder aufnimmt.

Die Offset-Daten werden in einem Offset-ROM 276 gespeichert. Die Adresseneingangssignale des ROM 276 werden von den Ausgangssignalen der Übertragungsschaltung 274 gebildet. Um die Offset-Information aus dem ROM 276 auszulesen, führt der Prozessor 262 die geeignete Adressen-

**0 004 258**

information der Übertragungsschaltung 274 zu, welche in die Übertragungsschaltung 274 durch ein Zeitsignal 277 vom Decoder 268 übertragen wird. Darauf erzeugt der Prozessor 262 die korrigierten Daten auf seinen Datenausgangsleitungen. Diese Daten werden den Eingängen der Puffer 278 zugeführt. Die C/C-Speicheradressendaten in der Übertragungsschaltung 274 werden in ähnlicher Weise den Eingängen der Puffer 280 zugeführt. Darauf erzeugt der Decoder 268 ein positives Signal auf der Leitung 282, welches die drei Zustände aufweisenden Puffer 278 und 280 derart beeinflußt, daß die Adressendaten und die korrigierten Detektordaten dem C/C-Speicher 120 zugeführt werden. Dieses Signal auf der Leitung 282 wird auch dem Korrektorsteuerkreis 272 zugeführt, welcher einen Schreibtakt 283 an den C/C-Speicher 120 legt und den Programmzähler 266 veranlaßt, beim nächsten Zeitimpuls zu springen, um die Korrekturrechnungen für den nächsten Datenpunkt zu beginnen. Nachdem alle Datenpunkte einer Projektion im Korrektor korrigiert worden sind, erzeugt der Steuerkreis 272 ein Korrektur-beendet-Signal, das dem Zwischenglied 132 zugeführt wird, wobei der Korrektor leer bleibt, bis er einen Hinweis vom Computer 130 über das Zwischenglied 132 erhält, daß die Daten der nächsten Projektion für die Verarbeitung zur Verfügung stehen.

Faltungsrechner

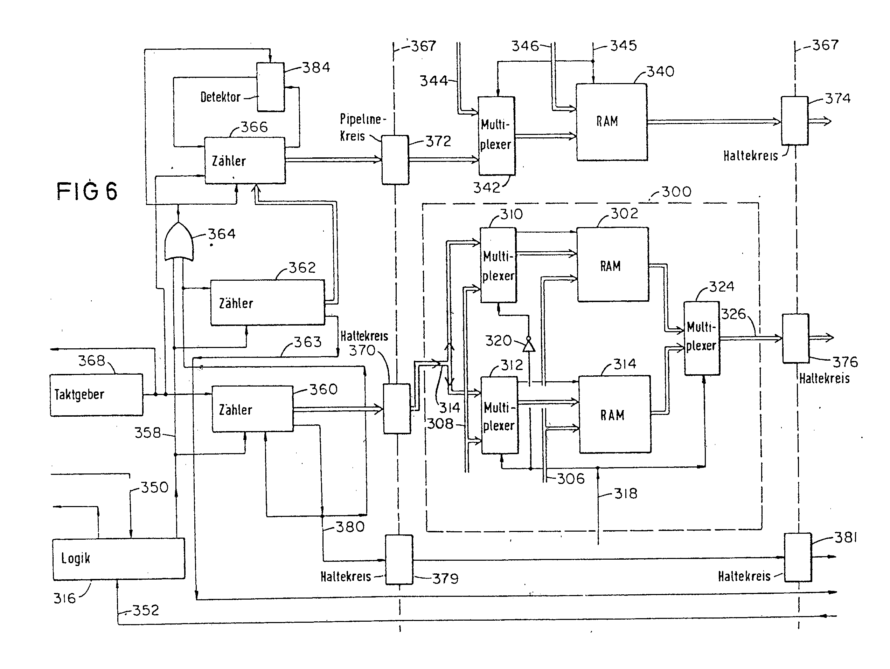

Wenn die korrigierten Daten durch den Korrektor erzeugt sind, werden sie in dem Zweibank-Korrektor/Faltungsrechner oder C/C-Speicher 120 gespeichert. Der C/C-Speicher ist in der Fig. 6 in der gestrichelt gezeichneten Umrandung 300 dargestellt und arbeitet in der folgenden Weise. Wenn jeder Datenpunkt berechnet ist, wird er in dem Korrektor in einer Bank des C/C-Speichers gespeichert. Gleichzeitig werden die in der anderen Bank des C/C-Speichers gespeicherten Daten durch den Faltungsrechner abgefragt, und er führt die nachfolgend beschriebenen Faltungsrechnungen durch. Am Ende der Verarbeitung der Daten jeder Projektion, wenn sowohl der Korrektor als auch der Faltungsrechner leer sind, werden die Speicherbänke elektronisch vertauscht. Die vom Korrektor während der vorherigen Abtastung erzeugten Daten stehen nun dem Faltungsrechner zur Verfügung und die voher gefalteten Daten werden durch die neuen Daten des Korrektors überschrieben, wenn diese Daten in dem C/C-Speicher gespeichert werden.

Wesentlich für die Wirkung des C/C-Speichers sind zwei Random Access Memories (RAM's) 302 und 304, die die zwei unabhängigen Bänke des Speichers bilden. Bei der dargestellten Ausführungsform ist jeder dieser Speicher ein $256 \times 15$-bit-Speicher. Die Daten vom Korrektor werden über Leitungen 306 den Dateneingängen jeder Speicherbank zugeführt. Die Adressendaten vom Korrektor, die anzeigen, welcher Detektor den Daten, die dann auf den Leitungen 306 liegen, entspricht, und das Schreibtaktsignal werden auf Leitungen 308 zwei Zweikanalmultiplexern 310 und 312 zugeführt. Die anderen Eingänge der Multiplexer 310 und 312 sind mit Leitungen 314 verbunden, die vom Faltungskreis kommen, wie dies unten näher erläutert wird. Die Auswähleingänge des Multiplexers 312 und der Lese/Schreibsignaleingang zum Speicher 304 werden vom Zwischenkreis 132 über die Leitung 318 gespeist; dieses Signal wird durch den Inverter 320 invertiert und dem Wähleingang des Multiplexers 310 sowie dem Lese/Schreibeingang des Speichers 302 zugeführt. Wenn also eine der Bänke 302 und 304 des C/C-Speichers in Schreib-Mode ist, ist die andere in Lese-Mode. Die Datenausgänge der Speicherbänke 302 und 304 werden einem Zweikanalmultiplexer 324 zugeführt, dessen Wähleingang ebenfalls mit der Leitung 318 verbunden ist.

Das signal auf Leitung 318 ändert seinen Zustand, wenn ein Projektionsverarbeitungszyklus begonnen wird. Auf diese Weise werden der Schreibtakt und die Adressendaten vom Korrektor zuerst der Speicherbank 302 zugeführt, welche in Schreib-Mode ist, und die Daten vom Korrektor auf der Leitung 306 werden in die Speicherbank 1 eingeschrieben. Gleichzeitig werden die Adressendaten auf den Leitungen 314 über den Multiplexer 312 der Speicherbank 304 zugeführt, die in Lese-Mode ist, und bewirken, daß die gewünschten Daten am Ausgang des Speichers 304 erscheinen. Der Multiplexer 324 leitet diese Daten dem Ausgang 326 des C/C-Speichers zu.

Wenn eine Projektion beendet ist, ändert das Signal auf der Leitung 318 seinen Zustand. Das oben beschriebene Verfahren wird während der folgenden Projektion umgekehrt ausgeführt, wobei die Daten vom Korrektor in Bank 2 gespeichert werden, während der Faltungsrechner die Daten, die vorher in Bank 1 gespeichert worden sind, abfragt.

Die Unschärfen beseitigende Funktion, mit der die Daten vom Korrektor gefaltet werden, kann von einem Techniker, der das Tomographiesystem bedient, variiert werden, und zwar abhängig davon, was er im endgültigen Bild hervorheben will, wie dies oben beschrieben ist. Die Daten, die repräsentativ für die Unschärfen beseitigende Funktion sind, werden ein Kernal genannt. Die verschiedenen Kernals, die einem Techniker zur Verfügung stehen, werden im Speicher des Computers 140 gespeichert. Vor der Ausführung eines tomographischen Abtastvorganges lädt der Computer über den Zwischenkreis 132 die gewählten Kernals in den Faltungsrechner.

Bei der dargestellten Ausführungsform besteht der Kernal aus 256 24-bit Worten. Der Kernal wird in einem RAM 340 durch einen Computer 130 und einen Zwischenkreis 132 gespeichert, wie dies nachfolgend beschrieben ist. Die Adressendaten für den Speicher 340 werden durch den Multiplexer 342 gewählt. Wenn ein neuer Kernal geladen werden soll, führt der Zwischenkreis dem Wähleingang des Multiplexers 342 ein Signal zu, das bewirkt, daß Adressenleitungen 344 vom Zwischenkreis mit den Adressenleitungen des Speichers 340 verbunden werden. Dieses Signal wird auch dem

11

Lese/Schreibeingang des Speichers 340 zugeführt und bewirkt, daß der Speicher in Schreib-Mode ist. Jedes Datenstück des Kernals wird dann in den Kernal-speicher auf den Leitungen 346 durch den Computer über den Zwischenkreis eingeschrieben. Nachdem dies erfolgt ist, geht die Leitung 345 in ihren Originalzustand zurück. Dementsprechend wählt der Multiplexer 342 die Adressendaten vom Faltungsrechnerkreis auf der Leitung 344, um sie dem Kernalspeicher 340 zuzuführen, und die im Speicher 340 gespeicherten Daten werden entsprechend den Bedürfnissen während des Faltungsprozesses aufgerufen.

Da die Daten von allen 256 Detektoren einer Projektion in einer Bank des C/C-Speichers gespeichert sein müssen, bevor der Faltungsrechner diese Daten abfragen kann, beginnt der Faltungsrechner nicht zu arbeiten, bevor die erste Projektion beendet ist. Wenn die erste Projektion beendet ist und das letzte Stück der Detektordaten in dem Korrektor verarbeitet und in dem C/C-Speicher geladen worden ist, bewirkt der Zwischenkreis 132, daß ein Faltungsrechner-bereit-Signal auf Leitung 350 in ein hohes Niveau übergeht und die Operation des Faltungsrechner-Interpolators startet. Der Zwischenkreis 132 schaltet auch den Zustand auf der Leitung 318 um, erlaubt dem Korrektor, die Daten der nächsten Projektion in die andere Bank des C/C-Speichers einzuschreiben und stellt die Daten der vorhergehenden Projektion dem Faltungsrechner zur Verfügung, wie dies oben beschrieben ist.

Nachdem die Daten gefaltet worden sind, bildet der Interpolator die interpolierten Werte zwischen den gemessenen Daten. Aufgrund der Struktur des Pipeline-Prozessors des Faltungsrechners und Interpolators ist der Interpolationsprozeß nicht beendet, bevor mehrere Taktperioden nach der letzten Faltung abgeschlossen sind. Wenn der Interpolationsprozeß beendet ist, sendet der Interpolator ein Interpolation-beendet-Signal auf der Leitung 352 zur Faltungsrechner-Steuerlogik 316. Beim Empfang dieses Signals sendet die Schaltungsrechner-Steuerlogik ein Faltung-beendet-Signal zum Zwischenkreis 132, das anzeigt, daß der Faltungsrechner die Verarbeitung der korrigierten Daten der vorhergehenden Projektion beendet hat. Der Zwischenkreis führt dann das Faltungsrechner-bereit-Signal auf ein niedriges Niveau zurück. Bei der beschriebenen Ausführungsform wird das gesamte Faltungs- und Interpolationsverfahren von 256 Datenstücken einer Projektion in ungefähr 10,6 ms durchgeführt. Da eine Projektion jede 13,9te ms aufgenommen wird, ist der Faltungsrechner während etwa 3,3 ms nach der Verarbeitung von Daten von einer Projektion leer, bevor der Korrektor den C/C-Speicher mit allen Daten der nächsten Projektion geladen hat.

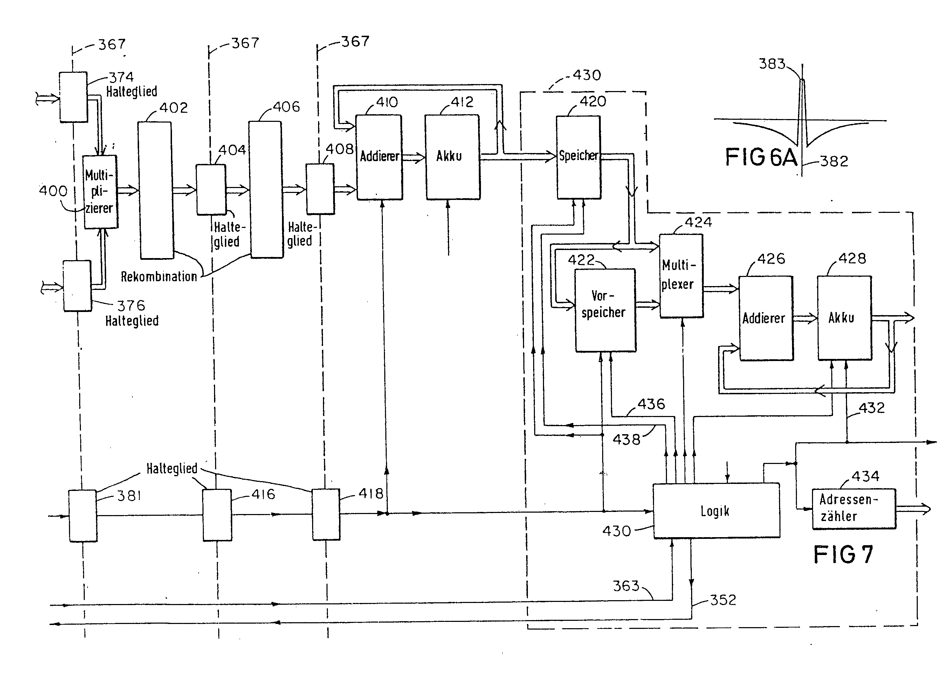

Ein Faltungsrechner-Taktgeber 368 erzeugt Taktsignale für den Faltungsrechner und den Interpolatorkreis. Bei der bevorzugten Ausführungsform haben die Faltungsrechner-Taktsignale eine Frequenz von 6,25 MHz. Die von jeder der Pipeline-Stufen erzeugten Daten werden in Zwischenhaltekreise zwischen den Stufen durch die Faltungsrechner-Zeitsignale eingetaktet, welche jeder dieser Stufen zugeführt werden. Die Pipeline-Halteschaltung und der Divisionskreis zwischen jeder der Pipeline-Stufen sind durch die vertikalen, gestrichelten Linien 367 in Fig. 7 gezeigt.

Der Faltungsrechner-Taktgeber 368 führt Taktsignale einem 9-bit Datenadressenzähler 360 zu und eine 8-bit-Kernal-Adresse einem Aufwärts/Abwärts-Zähler 366. Diese Zähler werden gemeinsam durch die Signale des Faltungsrechner-Taktgebers 368 weitergeschaltet. Der Wert im Datenadressenzähler 360 wird in den Pipeline-Haltekreis 370 eingegeben, wo er gehalten wird, um die Adresseninformation zum C/C-Speicher 120 zu liefern. Der Wert im Kernal-Adressenzähler 366 wird in den Pipeline-Kreis 372 eingegeben, wo dieser Wert gehalten wird, um die ordnungsgemäße Adresseninformation dem Kernal-Speicher 340 in der zweiten Stufe des Pipeline-Prozessors zuzu-führen. Die entsprechenden Ausgangssignale des Kernal-Speichers 340 und des C/C-Speichers 120 werden durch den nächsten Puls des Faltungsrechner-Taktkreises 368 in die Pipeline-Haltekreise 374 und 376 eingegeben. Diese Daten werden einem Vervielfacher-Array zugeführt, das unten beschrieben ist und das die Faltungsmultiplikation durchführt und die Produkte aufsummiert, um die Faltung zu vervollständigen.

Vor der Faltung von Daten einer Projektion setzt die Faltungsrechner-Steuerlogik 316 zunächst einen Datenadressenzähler 360 und einen Faltungszähler 362 auf Null über Rückstellsignale auf der Leitung 358. Das Rückstellsignal wird ferner über ein ODER-Gatter 364 dem Ladeeingang eines Kernal-Adressenzählers 366 zugeführt. Der Wert des Faltungszählers 362 wird den voreingestellten Eingängen des Kernal-Adressenzählers 366 überlagert, wie dies unten näher erläutert ist. Da der Faltungszähler durch ein Rückstellsignal auf der Leitung 358 auf Null gestellt wird, wird dieser Nullwert in den Kernal-Adressenzähler 366 geladen und stellt den Kernal-Adressenzähler auf Null zurück, damit das Faltungsverfahren begonnen werden kann.

Nachdem das Faltungsrechner-bereit-Signal angestiegen ist und der Faltungsprozeß beginnt, werden der Datenadressenzähler 360 und der Kernal-Adressenzähler 366 gemeinsam durch den Faltungstakt 368 weitergeschaltet, um die passenden Adressendaten dem C/C-Speicher und den Kernal-Speichern zuzuführen. Dieses Verfahren wird 256 mal wiederholt, um jeden der 256 Werte von jedem Detektor zu verarbeiten. Wenn der Wert in dem Datenadressenzähler 360 255 erreicht (wobei Null die erste Stufe ist), geht das Ausgangssignal des Zählers 360 in ein hohes Niveau über und bildet ein Faltung-Ende-Signal (EOC) 379, das anzeigt, daß die Faltung für einen Datenpunkt beendet ist. Das EOC-Signal 379 wird dem Rückstelleingang des Datenadressenzählers 360 zugeführt, welcher durch den nächsten Taktimpuls auf Null zurückgestellt wird, und wird ferner in eine Leitung 380 eingespeist, von wo es durch die Pipeline-Halteschaltung getaktet wird und evtl. den Faltungsrechner-Akkumulator zurücksetzt und dessen Ausgangssignal in den Interpolator einspeist, wie dies unten beschrieben ist.

Das EOC-Signal wird einem 9-bit-Faltungszähler 362 zugeführt und erhölt, folgend auf jede Faltung, den Wert in diesem Zähler um eins. Das EOC-Signal wird weiterhin über ein ODER-Gatter 364 dem Ladeeingang des Kernal-Adressenzählers 366 zugeführt. Dadurch wird der Kernal-Adressenzähler 366 in dem Sinne beeinflußt, daß er entsprechend einem Wert voreingestellt wird, der gleich dem Wert im Faltungszähler 362 ist. Aufgrund der Voreinstellung des Kernal-Adressenzählers 366 werden die die Bildschärfe erhöhende Funktion und die Daten durch einen zusätzlichen Datenpunkt für jede folgende Faltung ergänzt. Wenn jede Faltung beendet ist, was durch den Datenadressenzähler 360 und den Detektor 378 bestimmt wird, führt diese Ergänzung dazu, daß der entsprechende gefaltete Datenpunkt vom Faltungsrechner erzeugt wird. Auf diese Weise wird jeder der gewünschten gefalteten Datenpunkte erzeugt.

Das aktuelle Faltungs-Kernal ist 511 bits lang, wobei nur 256 dieser bits mit den Daten im C/C-Speicher 120 gefaltet werden, um einen gefalteten Datenpunkt zu erzeugen. Zur Erzeugung des ersten gefalteten Punktes wird die die Bildschärfe erhöhende Funktion, die in der Fig. 7A gezeigt ist, den Daten derart zugeordnet, daß die positive Spitze 383 der die Bildschärfe erhöhenden Funktion mit dem ersten Datenpunkt multipliziert wird, der zweite Wert der die Bildschärfe erhöhenden Funktion mit dem zweiten Datenpunkt multipliziert wird usw. Zur Erzeugung des zweiten gefalteten Datenpunktes wird die die Bildschärfe erhöhende Funktion um einen Wert verschoben, so daß die positive Spitze 383 mit dem zweiten Datenpunkt multipliziert wird. Die die Bildschärfe erhöhende Funktion wird aufeinanderfolgend in dieser Weise verschoben, um die restlichen gefalteten Datenpunkte zu erzeugen. Zur Ausführung der Faltungen auf diese Weise arbeitet der Faltungsrechner in der folgenden Art.

Die verschiedenen, die Bildschärfe erhöhenden Funktionen sind alle symmetrisch. Dies ist in der Fig. 6A gezeigt, in der eine die Bildschärfe erhöhende Funktion schematisch dargestellt ist. Aus dieser Figur geht hervor, daß diese Funktion symmetrisch zur Linie 382 liegt. Um die Erfordernisse für die Speicherung auf einem Minimum zu halten, wird nur eine Hälfte der die Bildschärfe erhöhenden Funktion in dem Computer und im Kernal-Speicher 340 gespeichert, wobei die positive Spitze im ersten (Null-) Platz des Kernal-Speichers gespeichert wird und die darauffolgenden Punkte (entweder nach rechts oder nach links) in darauffolgenden Speicher-plätzen gespeichert werden. Wie oben beschrieben ist, wird der Kernal-Adressenzähler 366 auf die Faltungsnummer für den Beginn einer Faltung gesetzt. Der Kernal-Adressenzähler zählt dann nach unten, bis er Null erreicht, wobei die entsprechenden Daten vom C/C-Speicher und die Werte der die Bildschärfe erhöhenden Funktion für die Faltung zur Verfügung stehen. Wenn der Zähler Null erreicht, wird die positive Spitze vom Kernal-Speicher erzeugt, und der entsprechende Datenpunkt wird vom C/C-Speicher entsprechend dem Datenadressenzähler erzeugt. Wenn der Kernal-Adressenzähler Null erreicht, schaltet ein Null-Ausgangssignal des Zählers das Flip-Flop 384 um, dessen Ausgangssignal dem Aufwärts/Abwärts-Eingang des Kernal-Adressenzählers zugeführt wird. Das Flip-Flop 384 ändert seinen Zustand und wirkt auf den Kernal-Adressenzähler 366 ein, so daß dieser bei aufeinanderfolgenden Taktimpulsen aufwärts zählt. Der Zähler 366 fährt mit der Aufwärtszählung fort, bis der Zähler 366 und das Flip-Flop 384 am Ende der Faltung des Datenpunktes durch das Signal des ODER-Gatters 364 zurückgestellt werden.

Wenn z.B. die 13te Faltung durchgeführt wird, wird der Kernal-Adressenspeicher auf 12 voreingestellt (weil bei Null die erste Faltung erfolgt), nach 12 Pulsen erzeugt der C/C-Speicher den 13ten Datenpunkt und der Kernal-Speicher die positive Spitze an seinem Ausgang. Wenn der Kernal-Speicher von seinem voreingestellten Wert aus auf Null zurückzählt, werden die richtigen Werte der die Bildschärfe erhöhenden Funktion links von der positiven Spitze (welche dieselben Werte sind wie diejenigen, die rechts liegen) in der richtigen Ordnung erzeugt, so daß die dem 13ten Datenpunkt vorhergehenden Daten multipliziert werden können. Nachdem der Kernal-Adressenzähler seinen Nullzustand erreicht, schaltet er seine Richtung um und zählt aufwärts, um zu erreichen, daß die entsprechenden Werte der die Bildschärfe erhöhenden Funktion mit den Datenpunkten, die auf den 13ten Datenpunkt folgen, multipliziert werden.

Nachdem 256 separate Faltungen durchgeführt worden sind, geht der 9te bit des Faltungszählers 362 auf der Leitung 363 auf hohes Niveau über. Dieses Signal wird dem Interpolatorkreis zugeführt, um anzuzeigen, daß der letzte Datenpunkt vom Faltungsrechner verarbeitet worden ist.

In der Fig. 7 ist der verbleibende Teil des Faltungsrechnerkreises gezeigt. Von den Haltegliedern 374 und 376, die beide in den Fig. 6 und 7 gezeigt sind, werden die Kernaldaten, die für die die Bildschärfe erhöhende Funktion repräsentativ sind, und die korrigierten Detektordaten des C/C-Speichers 120 den Eingängen eines Multiplizierers 400 zugeführt. Der Multiplizierer 400 ist ein 23 mal 15-bit Multiplizierer. Bei der dargestellten Ausführungsform weist der Multiplizierer 400 4×4 Multiplizierkreise auf, deren Ausgänge in einem Wallace-Baum kombiniert sind. Die ersten Teilprodukte des Multiplizierers sind in der ersten Stufe des Rekombinationskreises 402 aufsummiert. Das Ausgangssignal des Rekombinationskreises 402 wird in die Pipeline-Verriegelungsschaltung 404 durch den Faltungstakt eingetaktet. Die Teilprodukte in den Haltegliedern 404 werden der zweiten Stufe eines Rekombinationskreises 406 zugeführt. Der Ausgang der zweiten Stufe des Rekombinationskreises ist das Endprodukt, welches in die Pipeline-Verriegelungsschaltung 408 eingetaktet wird.

Der in den Pipeline-Verriegelungsschaltungen 408 gespeicherte Wert ist ein 32-bit Wert, der das Produkt eines Stückes von Detektordaten, die mit einem Wert der die Bildschärfe erhöhenden Funktion multipliziert sind, darstellt. Für jede vollständige Faltung werden 256 dieser Werte summiert, um jeden gefalteten Datenpunkt zu erzeugen. Die Daten in den Halteschaltungen 408 werden einem Eingang eines Addierers 410 zusammen mit dem Zeichen-bit zugeführt, welches am Addierer/Subtrahierer-Eingang liegt. Das Ausgangssignal des Addierers 410 ist einem Akkumulatorregister 412 zugeführt. Der Wert, der im Akkumulatorregister 412 gespeichert ist, wird dem zweiten Eingang des Addierers 410 zugeführt. Vor dem Beginn der Faltung für jeden Datenpunkt wird das Akkumulatorregister 412 durch das EOC-Signal 379 vom Zähler 360 auf Null gestellt, das über Pipeline-Halteschaltungen zugeführt wird. Das erste Produkt, das in die Halteschaltungen 408 eingetaktet wird, wird durch den Addierer 410 zu dem Nullwert im Akkumulator 412 addiert und in den Akkumulator 412 durch den Faltungstakt eingetaktet, welcher am Takteingang des Akkumulators 412 liegt. Aufeinanderfolgende Produkte, welche in der Halteschaltung 408 gespeichert sind, werden durch den Addierer 410 zu der Untersumme addiert, die in Akkumulatorregister 412 gespeichert ist und die neue Untersumme wird in den Akkumulator 412 durch den Faltungstakt eingetaktet. Nach 256 Additionen und Akkumulationen dieser Art ist der Wert, der im Akkumulator 412 gespeichert ist, der gewünschte gefaltete Datenpunkt.

Das EOC-Signal 379, das vom Detektor 378 erzeugt wird und dem letzten Datenpunkt folgt, wird durch die Halteschaltungen jeder Stufe der Pipeline getaktet. Auf diese Weise wird das EOS-Signal durch die Stufen der Pipeline einen Schritt hinter den letzten Daten, die bei jeder Faltung verarbeitet werden, getaktet. Demgemäß erscheint das EOC-Signal 379, nach dem der letzte Wert zu der Summe im Akkumulator 412 addiert worden ist, in der Halteschaltung 418 beim nächsten Taktimpuls vom Faltungstaktgeber 368. Dieser Impuls wird dem Addierer 410 zugeführt und setzt den Addiererausgang auf Null, so daß der Akkumulator 412 durch den nächsten Taktimpuls zur Vorbereitung der nächsten Faltung gelöscht wird. Der Ausgang der Halteschaltung 418 wird weiterhin dem Takteingang eines Faltungsrechner-Ausgangsspeichers 420 im Interpolator zugeführt. Die Daten im Akkumulator 412 werden den Dateneingängen des Faltungsrechner-Ausgangsspeichers zugeführt und diese Daten werden in den Speicher 420 und den Interpolator durch das EOC-Signal in der Halteschaltung 418 eingetaktet.

Interpolator

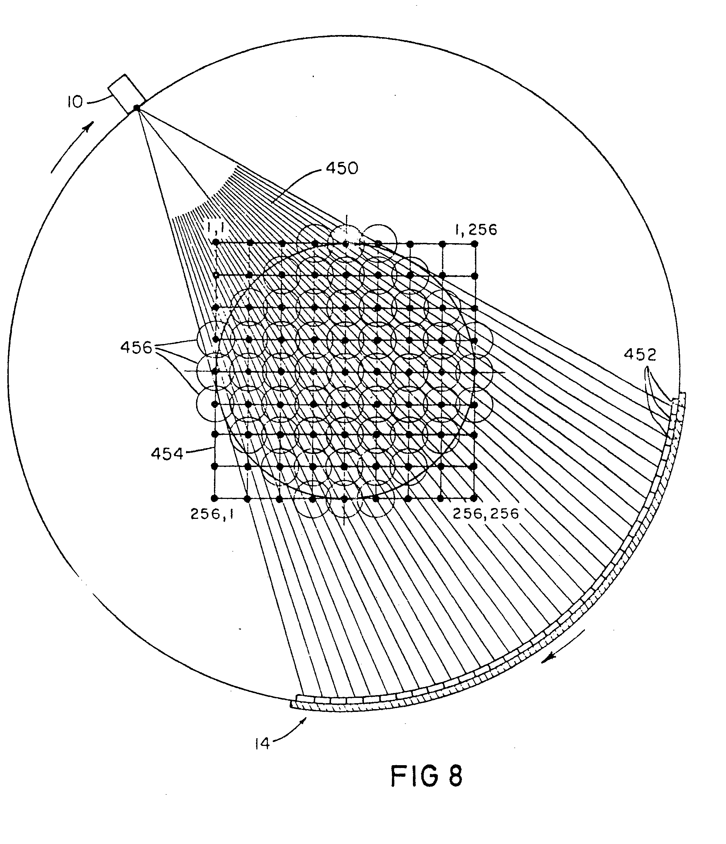

Das Ausgangssignal des Faltungsrechnerkreises wird dem Interpolationskreis zugeführt. Nachfolgend ist erläutert, warum die Interpolation notwendig ist und wie sie durchgeführt wird. Bei der Bildrekonstruktion wird der abgetastete Körper in der Weise betrachtet, daß er in kleine Bereiche gleichförmiger Dichte aufgeteilt ist. Die Bereiche werden als Pixel (picture elements) bezeichnet. Bei der beschriebenen, bevorzugten Ausführungsform ist der kreisförmige Bereich, der von der Röntgenröhre und dem Detektor-Array abgetastet wird, so aufgeteilt, daß der vom Scanner überstrichene Kreis von einem Quadrat mit 256×256 Pixeln eingeschlossen wird. Dies ist schematisch in der Fig. 8 gezeigt.

Aufgrund der Geometrie des Systems verlaufen Strahlen, die von der Röntgenröhre 10 ausgesandt werden und auf dem Detektor-Array 14 auftreffen, nicht immer durch das Zentrum eines Pixels und häufig verläuft mehr als ein Strahl durch einen Pixel. In der Fig. 8 treffen die Strahlen 450 (in Wirklichkeit kleine Fächer von Röntgenstrahlung und keine einzelnen Strahlen) auf jedem der individuellen Detektoren 452 im Detektor-Array 14 auf. Das Gitter 454 zeigt die Pixel 456, in die der abgetastete Körper im Bildspeicher aufgeteilt ist. Die Punkte im Gitter 454 bezeichnen die Zentren jedes Pixels. Der Rekonstruktions-Algorithmus geht davon aus, daß die gefalteten Daten, die zur Erzeugung des Wertes jedes Pixels benutzt werden, das Ergebnis eines Strahls der Röntgenstrahlenquelle durch das Zentrum jenes Pixels sind. Wie aus der Fig. 8 hervorgeht, ist diese Annäherung nicht für alle Pixel richtig. Ferner ist das Pixel-Array 454 fest in bezug auf den Patienten und die Beziehung zwischen den Pixel-Mittelpunkten und den Strahlen 450 ändert sich bei jeder Aufnahme, wenn die Röntgenröhre und das Detektor-Array um das feste Pixel-Array 454 rotieren.

Der Interpolator berechnet Daten entsprechend sieben zusätzlichen Plätzen zwischen jedem der Detektoren im Detektor-Array 14. Dies erfolgt mittels einer linearen Interpolation. Auf diese Weise bestehen die interpolierten Datenpunkte zwischen zwei aneinander angrenzenden Detektoren, die unmittelbar an den ersten Detektor angrenzen, aus dem gefalteten Ausgangssignal des ersten Detektors mit einem Gewicht von 7/8 und dem gefalteten Ausgangssignal des zweiten Detektors mit einem Gewicht von 1/8 usw. für jeden der Zwischenbereiche zwischen Detektoren. Zur Durchführung der Rückprojektion berechnet der Bildrekonstruktionskreis den interpolierten Datenpunkt, der von einem Strahl geschnitten wird, der durch das Zentrum jedes Pixels verläuft, und dieser Datenpunkt wird zur Berechnung der Dichte jenes Pixels herangezogen.

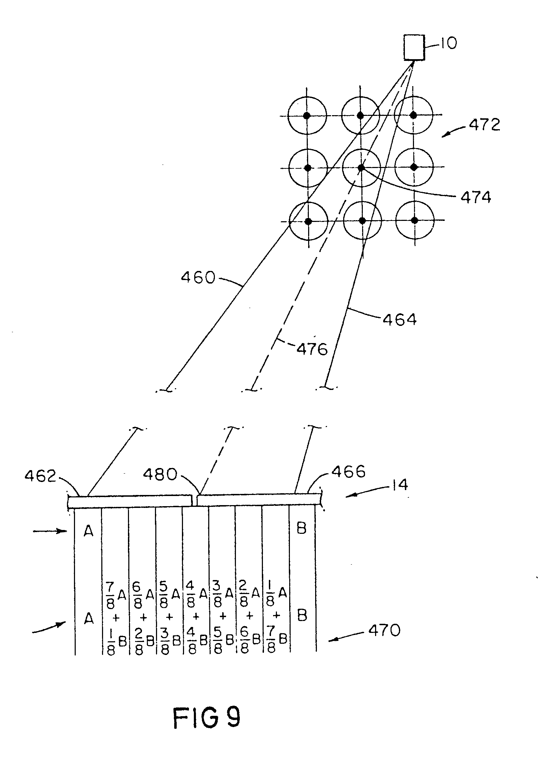

Dies ist schematisch in der Fig. 9 gezeigt. Hier verläuft die Röntgenstrahlenenergie der Röntgenröhre 10 längs eines fächerförmigen Pfades entsprechend der Linie 464 und trifft auf einem Detektor 466 auf. Wenn die gefalteten Daten, die dem Detektor 462 entsprechen, mit A und die gefalteten Daten für den Detektor 466 mit B bezeichnet werden, berechnet der Interpolator sieben Zwischenwerte, welche eine lineare Interpolation zwischen den Werten A und B darstellen, wie dies durch die Werte 470 in der Fig. 9 gezeigt ist.

Wenn die Pixel liegen, wie dies das Gitter 472 wiedergibt, ergibt sich, daß der Dichte des Pixels 474 entsprechende Daten in den Röntgenstrahlen der Linien 460 und 464 enthalten sind. Beim Rekonstruktionsprozeß wird für jedes Pixel der geeignete Wert aus den interpolierten Datenpunkten durch Berechnung des Schnittes eines imaginären Strahls mit der Ebene des Detektor-Arrays 14 gewählt, wie dies durch die gestrichelte Linie 476, die durch das Zentrum des Pixels 474 verläuft, gezeigt ist. Der Punkt 480, in dem der Strahl 476 die Ebene des Detektor-Arrays schneidet, bestimmt, welcher interpolierte Datenpunkt zur Rekonstruktion des Pixels 474 während der Rückprojektion benutzt wird. Der Rückprojektionsprozeß ist genauer nachfolgend beschrieben.

Diese spezielle Methode der Erzeugung interpolierter Daten zwischen den aktuellen Daten jeder Projektion hat den Vorteil, daß sie einfacher und schneller ist als die Durchführung der Interpolation in dem Zeitpunkt, in dem jeder der interpolierten Werte erforderlich ist. Der Bildspeicher enthält 256×256 individuelle Plätze, einen für jeden Pixel. Um eine "fliegende" Interpolation durchzuführen, wenn die in jedem Platz des Bildspeichers gespeicherten Daten aufdatiert werden, wäre es erforderlich, daß 256×256 Rechnungen durchgeführt werden. Die vorliegende Methode erfordert, obgleich mehrere Speicher für die Speicherung der zusätzlichen interpolierten Daten erforderlich sind, nur 8×256 Rechenvorgänge, woraus eine beachtliche Zeitersparnis resultiert.

Der Interpolatorkreis der bevorzugten Ausführungsform ist in der gestrichelten Umrandung 430 in Fig. 7 gezeigt. Wenn ein gefalteter Datenpunkt am Ausgang des Faltungsrechners erzeugt wird, wird er in einen Faltungsrechner-Ausgangsspeicher 420 in dem Interpolator eingetaktet. Bei der Berechnung des nächsten gefalteten Datenpunktes wird der Wert im Speicher 420 in einen Faltungsrechner-Ausgangs-Vorspeicher 422 eingetaktet. Auf diese Weise enthalten die Speicher 420 und 422 zu einer bestimmten Zeit die gefalteten Daten, die die Ausgangssignale zweier benachbarter Detektoren repräsentieren. Die in den Speichern 420 und 422 gespeicherten Werte werden zwei Eingängen eines Zweikanal-Multiplexers 424 zugeführt. Das Ausgangssignal des Multiplexers 424 ist dem ersten Eingang eines Addierkreises 426. zugeführt. Das Ausgangssignal des Addierkreises 426 ist den Eingängen eines Akkumulators 428 zugeführt und die im Akkumulator 428 gespeicherten Werte werden den zweiten Eingängen des Addierers 426 zugeleitet. Eine Interpolator-Steuerlogik 430 speist den Wähleingang zum Multiplexer 424 und bestimmt, welches der zwei Detektorausgangssignale dem Addierer 426 zugeführt wird. Die Interpolator-Steuerlogik 430 führt auch das Taktsignal dem Akkumulator 428 zu.

Wie oben in Verbindung mit Fig. 9 erläutert ist, sind für jedes Detektorausgangssignal die interpolierten sieben Werte proportional 1/8, 2/8, 3/8 usw. des ersten Detektorwertes und 7/8, 6/8, 5/8 usw. des zweiten Detektorwertes. Durch Taktung des Akkumulators 428 bewirkt die Interpolator-Steuervorrichtung 430, daß der Addierer 426 und der Akkumulator 428 sieben Additionen der Werte ausführt, die dem Addierer 426 durch den Multiplexer 424 zugeführt werden. Durch selektive Schaltung des Multiplexers 424 während dieser sieben Additionen werden die interpolierten Werte, die durch verschiedene Bewichtung der in den Speichern 422 und 420 gespeicherten Daten gebildet sind, im Akkumulator 428 akkumuliert. Nachdem sieben Additionen durchgeführt worden sind, erzeugt die Interpolator-Steuerlogik 430 einen Puls auf der Leitung 432, der den Wert im Akkumulator 428 in den Faltungsrechner/Bildrekonstruktor- (C/I)-Speicher 122 eintaktet. Dieses Signal löscht auch den Akkumulator 428 zur Vorbereitung der nächsten Interpolation. Dieses Signal wird ferner dem Takteingang eines Adressenzählers 434 zugeführt, welcher umgeschaltet wird, um die Adressendaten dem C/I-Speicher zuzuführen. Der Interpolator braucht nur sieben Additionen für jeden der acht interpolierten Datenpunkte durchzuführen oder eine Gesamtheit von 64 Operationen für jeden neuen gefalteten Datenpunkt des Faltungsrechners. Da der Faltungsrechner 256 Faltungs-Taktperioden braucht, um jeden gefalteten Datenpunkt zu erzeugen, ist der Interpolator während einer Periode, die dem Abschluß jeder Interpolation folgt, leer, bis der nächste gefaltete Datenpunkt zur Verfügung steht, was durch das EOC-Signal 379 angezeigt wird, das dem "Daten-fertig"-Eingang der Interpolator-Steuerlogik 430 zugeführt wird.

Wenn die Daten des ersten Detektors im Faltungsrechner-Ausgangsspeicher 420 sind, stehen offensichtlich keine Daten von einem Detektor zur Verfügung, der diesem Detektor in dem vorhergehenden C.O.Speicher 422 vorhergeht. (Die Monitor-Detektordaten werden nicht in dieser Art und Weise bearbeitet.) Wenn der erste Datenpunkt im Faltungsrechner-Ausgangsspeicher 420 ist, führt die Interpolator-Steuerlogik dem Speicher 422 auf der Leitung 436 ein Löschsignal zu, um irgendwelche Daten, die im Speicher 422 sein können, zu löschen und auf diese Weise unnormale Resultate von solchen Daten zu vermeiden. In ähnlicher Weise stehen dann, wenn die Daten des letzten Detektors im vorhergehenden Faltungsrechner-Ausgangsspeicher 422 sind, keine Daten von einem nächsten Detektor im Faltungsrechner-Ausgangsspeicher 420 zur Verfügung; die Interpolator-Steuerlogik erzeugt einen Puls, um den Speicher 420 auf der Leitung 438 zu löschen. Diese interpolierten Datenpunkte werden dann berechnet. Bei der dargestellten Ausführungsform werden nur vier interpolierte Datenpunkte vor dem ersten Detektor und vier interpolierte Datenpunkte nach dem letzten Detektor benutzt.

Ein Signal, das anzeigt, wenn die letzte Faltung durchgeführt worden ist, wird auf der Leitung 363 geliefert, und zwar durch den Faltungszähler 362, wie dies oben beschrieben ist. Dieses Signal wird der Interpolator-Steuerlogik 430 zugeführt. Folgend auf die letzte Faltung müssen noch zwölf Interpolationen durch die Interpolations-Steuerlogik 430 durchgeführt werden. Wenn diese Interpolationen beendet sind, erzeugt die Interpolations-Steuerlogik 430 ein Interpolation-beendet-Signal auf der Leitung 352, welches der Faltungsrechner-Steuerlogik 316 zugeführt wird, und der Faltungs- und Interpolationsprozeß der vorliegenden Projektion ist beendet.

Bildrekonstruktor

Die bekannten Methoden der Bildrekonstruktion haben in bezug auf die Abtastzeit viel Zeit erfordert, um die gefalteten Daten des Detektor-Arrays, die während jeder Projektion erzeugt werden, zu verarbeiten. Einige dieser Verfahren haben erfordert, daß die Daten einer großen Zahl von Projektionen zur Verfügung standen, bevor die Verarbeitung begann.

Die Bildrekonstruktorstufe des Tomographieprozessors, die hier beschrieben ist, schließt einen Pipeline-Prozessor ein, der in der Lage ist, eine Rückprojektion von Daten in praktisch der Zeit durchzuführen, in der diese durch die Röntgenröhre und das Detektor-Array geliefert werden und in der sie durch den oben beschriebenen Verarbeitungskreis verarbeitet werden.

Bei der Bildrekonstruktion müssen zwei Funktionen ausgeübt werden. Als erstes muß für jeden Platz im Bildspeicher der entsprechende Datenpunkt im C/I-Speicher plaziert werden, was der Prozeß der Zuordnung jedes Pixels zu dem entsprechenden gefalteten und interpolierten Datenpunkt ist. (Jedes Pixel hat einen entsprechenden Platz im Bildspeicher, in dem Daten gespeichert werden, die repräsentativ für die Dichte des abgetasteten Körpers in jenem Punkt sind.) Als zweites müssen die plazierten Daten mit einem Bewichtungsfaktor multipliziert werden, der in der unten beschriebenen Weise bestimmt wird. Nachdem diese Rechenvorgänge durchgeführt worden sind, werden die entsprechend bewichteten Daten in den zugeordneten Platz im Bildspeicher dadurch eingegeben, daß sie zu den vorher in jenem Platz im Bildspeicher akkumulierten Daten addiert werden. Nachdem die Abtastung beendet und all die Daten jeder der Projektionen geliefert worden sind, repräsentieren die Werte, die in jedem Platz des Bildspeichers gespeichert sind, die Dichte der entsprechenden Stellen in dem abgetasteten Körper. Diese Daten werden dann benutzt, um das endgültige Bild zu erzeugen.

Die beschriebene Ausführungsform hat einen Bildspeicher mit 256×256 Speicherplätzen. Für jede der 360 Projektionen muß jeder dieser Plätze mit einem entsprechend bewichteten und plazierten Datenpunkt aus dem C/I-Speicher 122 beaufschlagt werden. Dies heißt, daß 256×256×360 oder 23 592 960 Plazierungen und Bewichtungen für jedes endgültige Bild durchgeführt werden müssen. Jede dieser Plazierung und Bewichtung schließt eine Vielzahl von arithmetischen Funktionen ein, die ausgeführt werden müssen.

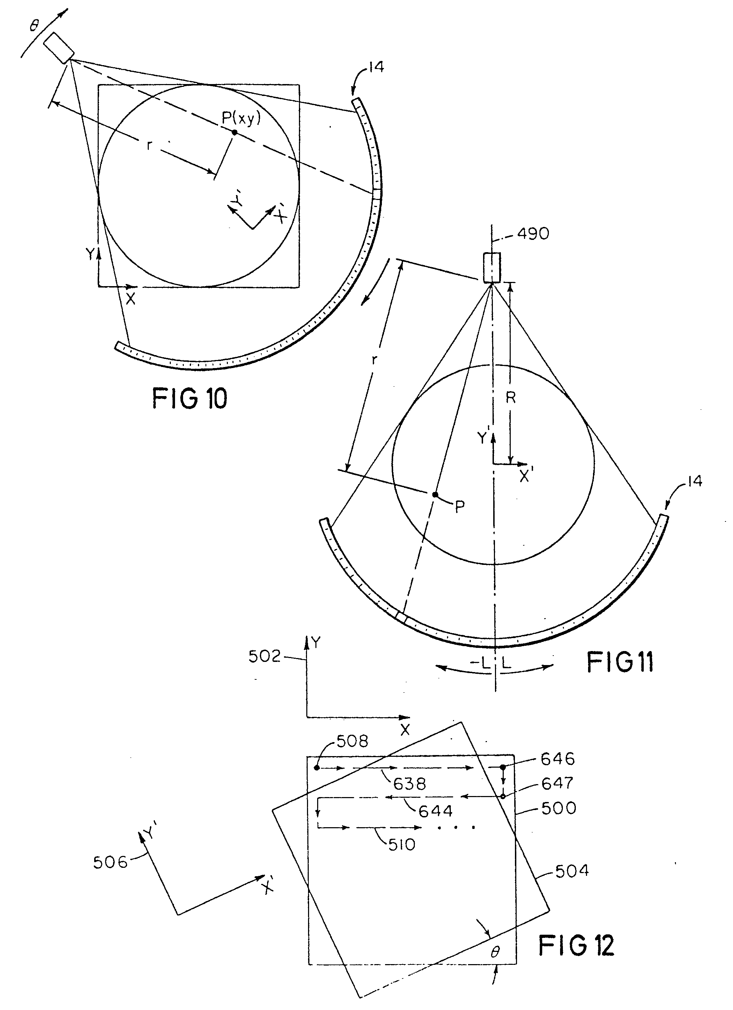

Gemäß Fig. 10 wird die Funktion, die jeden Punkt P mit den Koordinaten x und y im Bildspeicher den Daten eines bestimmten Detektors im Detektor-Array 14 für jeden Projektionswinkel zuordnet, die Zuordnungsfunktion oder Lagefunktion genannt. Sie ist gegeben durch:

$$L = C_1 \tan^{-1} \frac{(y \cdot \sin\theta - x \cdot \cos\theta)}{(R - y \cdot \cos\theta - x \cdot \sin\theta)} \qquad (24)$$

wobei x und y die Koordinaten im Bildspeicher, $\theta$ der Projektionswinkel, R der Abstand der Röntgenröhre vom Zentrum der Rotation, $C_1$ eine Konstante und L der Wert der Lagefunktion ist, welcher den Winkel der entsprechenden interpolierten Daten wiedergibt.

Wie beschrieben müssen die Daten vor dem Rückprojektionsprozeß durch Multiplikation mit $1/r^2$ bewichtet werden, wobei r der Abstand der Röntgenröhre 10 vom Pixel, das rückprojiziert wird, ist. Die Funktion, die den Wert von $1/r^2$ ausdrückt, wird die Bewichtungsfunktion genannt und ist gegeben durch:

$$W = C_2 \frac{1}{(y \sin\theta)^2 + (R - y \cos\theta + x \sin\theta)^2} \qquad (25)$$

wobei $C_2$ eine Konstante und W der Wert der Bewichtungsfunktion ist und die verbleibenden Terme der oben gegebenen Definition entsprechen.

Diese beiden Funktionen sind vergleichsweise kompliziert und zeitraubend in der Berechnung. Beide erfordern verschiedene Multiplikationen, Emittlung von trigonometrischen Funktionen, und eine Division, eine sehr zeitraubende Operation. Zusätzlich sind die Rechenvorgänge, die für jeden Projektionswinkel erforderlich sind, unterschiedlich, weil die Relationen Funktionen des Projektionswinkels $\theta$ und der Patientenkoordinaten x und y sind.

Es wurde gefunden, daß die Bewichtungs- und Zuordnungsfunktionen wesentlich schneller und einfacher berechnet werden können, wenn vor der Berechnung dieser Werte eine Koordinatentransformation durchgeführt wird und die Bewichtungs- und Zuordnungsfunktionen in den Koordinaten des Projektionssystems und nicht in den Koordinaten des Patientensystems berechnet werden. Dies erfordert einen besonderen Schritt für jeden Platz (Pixel) im Bildspeicher—die Stufe der Umwandlung der Bildspeicheradresse, die im Patientenkoordinatensystem vorliegt, ins Projektionskoordinatensystem. Diese Transformation ist jedoch verhältnismäßig leicht durchzuführen und führt zu einer großen

**0 004 258**

Vereinfachung bei der Berechnung der Bewichtungsund Plazierungsfunktionen. Wenn eine solche Koordinatentransformation einmal durchgeführt ist, werden die Plazierungs- und Bewichtungsfunktionen in den Koordinaten des Projektionssystems wie nachfolgend beschrieben berechnet.

Aus der Fig. 11 geht hervor, daß für jeden Pixel P die vom Detektor empfangene Strahlung, die durch diesen Pixel geht, eine Funktion des Winkels zwischen jenem Punkt und der Röntgenröhrenachse 490 ist. Der Winkel kann einfach im Projektionskoordinatensystem durch folgende Plazierungsfunktion ausgedrückt werden:

$$L = C_3 \tan^{-1} \frac{(x')}{(y'-R)} \qquad (26)$$

wobei L die Adresse der interpolierten Daten, $C_3$ eine Konstante, $x'$ und $y'$ die Pixel-Koordinaten im Projektionskoordinatensystem und R der Abstand zwischen der Röntgenröhre und dem Zentrum der Rotation sind. Zwei sehr wichtige Dinge resultieren aus der Berechnung der Lagefunktion im Projektionskoordinatensystem an Stelle der Berechnung im Patientenkoordinatensystem. Als erstes ist nun die Lagefunktion eine Funktion von nur zwei Variablen und nicht von drei. Als zweites ist die Lagefunktion identisch für alle Projektionswinkel.

In ähnlicher Weise ist auch die Berechnung der Bewichtungsfunktion stark vereinfacht, wenn die Bewichtungsfunktion im Projektionskoordinatensystem ausgedrückt wird. Wie oben beschrieben, erfordert die Bewichtungsfunktion, daß die Daten für jeden Punkt des Bild-Arrays mit $1/r^2$ multipliziert werden, wobei r der Abstand der Röntgenröhre zur Lage des Bild-Arrays ist. Im Hinblick auf Fig. 12 kann der Wert von $r^2$ einfach berechnet werden, indem der Satz des Pythogoras benutzt wird und die Bewichtungsfunktion wird zu:

$$W = \frac{1}{x'^2 + (y'+R)^2} \qquad (27)$$

Die Bewichtungsfunktion ist, wenn sie im Patientenkoordinatensystem ausgedrückt ist, ebenfalls eine Funktion von zwei Variablen und nicht von dreien und ist dieselbe für alle Projektionswinkel.

Gemäß Fig. 12 entspricht der mit 500 bezeichnete Bereich dem Bildspeicher, der im Patientenkoordinatensystem orientiert ist, das durch die x- und y-Achsen 502 verkörpert ist. Jeder Platz im Bildspeicher entspricht einem Pixel, in die der abgetastete Querschnittsbereich unterteilt ist. Im allgemeinen sind die Röntgenröhre 10 und das Detektor-Array 14 unter einem Winkel $\theta$ zum Patientenkoordinatensystem orientiert. Dies ist in der Fig. 12 gezeigt, wo der Kasten 504 und die Achsen 506 dem Projektionskoordinatensystem entsprechen. Die Koordinaten im Projektionskoordinatensystem-Rahmen sind mit $x'$ und $y'$ bezeichnet. Es ist gezeigt, daß für jeden der verschiedenen Projektionswinkel $\theta$ eine unterschiedliche Koordinatentransformation zwischen dem Patientenkoordinatensystem und dem Projektionskoordinatensystem gemacht werden muß.

Bei der vorliegenden Erfindung werden die Plätze im Bildspeicher aufeinanderfolgend beaufschlagt, während die gefalteten und interpolierten Daten beliebig zugänglich sind. Im Hinblick auf Fig. 12 beginnt der Bildrekonstruktor beim ersten Speicherplatz im Bildspeicher 508 und wird schrittweise durch deden der Speicherplätze des Speichers durchgeschaltet, wie dies durch die gestrichelte Linie gezeigt ist. Während der systematischen schrittweisen Durchschaltung durch den Bildspeicher können die entsprechenden Projektionskoordinaten $x'$ und $y'$ von jedem der Plätze im Bildspeicher leicht erzeugt werden. Wenn die Projektionskoordinaten des ersten Speicherplatzes 508 im Bildspeicher einmal bestimmt sind, können die Projektionskoordinaten aller anderen Plätze im Bildspeicher berechnet werden, wenn der Prozessor schrittweise durch diese Plätze hindurchschaltet, und zwar durch einfaches Erhöhen oder Erniedrigen der vorhergehenden Koordinaten mit $\Delta x'$ und $\Delta y'$ Werten, welche für jeden Projektionswinkel konstant sind. Auf diese Weise wird die Koordinatentransformationsfunktion ein Prozeß der iterativen Beaufschlagung der $x'$ und $y'$-Werte entsprechend den folgenden Funktionen:

$$x' = x' \pm \Delta x' = x' \pm \delta \cos \theta \qquad (28a)$$

$$y' = y' \pm \Delta y' = y' \pm \delta \sin \theta \qquad (28b)$$

Für Inkremente längs der x-Achse im Bildspeicher und

$$x' = x' \pm \delta \sin \theta \qquad (28c)$$

$$y' = y' \pm \delta \cos \theta \qquad (28d)$$

für Inkremente längs der y-Achse im Bildspeicher, wobei $\delta$ der Abstand zwischen Pixel-Zentren im Bild-Array ist. Jedes Inkrement von $x'$ und $y'$ wird durch eine Addition erzeugt, welche sehr schnell durch

17

relativ einfache hardware durchgeführt werden kann. Die Projektionskoordinaten des ersten Platzes 508 im Bildspeicher können für jeden Projektionswinkel durch eine einfache trigonometrische Berechnung bestimmt werden.

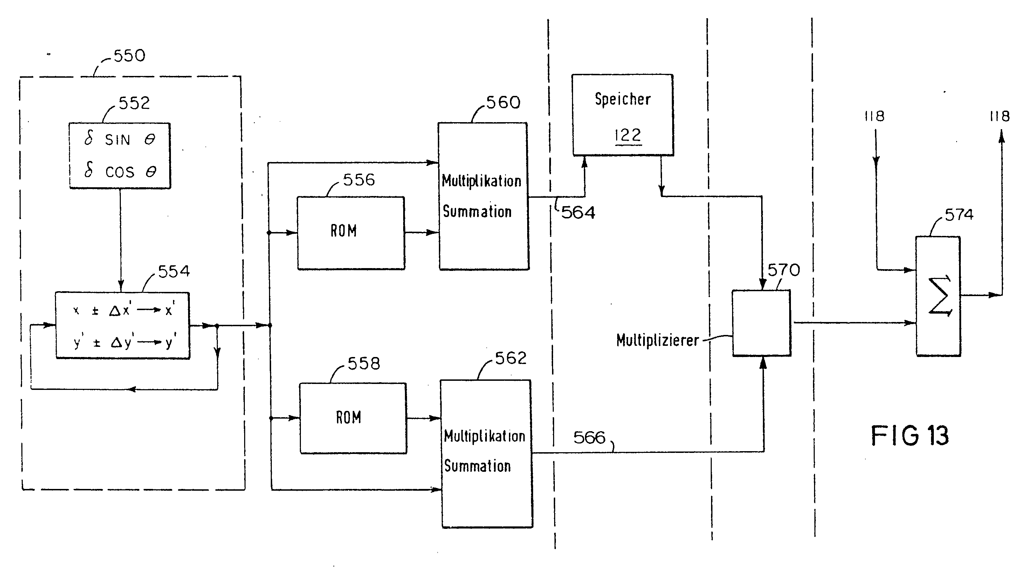

Ein Blockdiagramm zeigt die Stufen, die im Bildrekonstruktionsprozeß der vorliegenden Erfindung erforderlich sind (Fig. 13). Wenn die gefalteten und interpolierten Daten für eine ganze Abtastung vom Faltungsrechner und vom Interpolationskreis verarbeitet worden sind und im C/I-Speicher gespeichert sind, kann der Bildrekonstruktionskreis seine Operation mit diesem Satz von Daten beginnen. Die erste Stufe im Rekonstruktionsprozeß ist die oben beschriebene Koordinatentransformation. Dies ist im Block 550 in Fig. 13 gezeigt. Vor dem Beginn der Bildrekonstruktion der Daten jeder Projektion werden $\delta \sin \theta$ und $\delta \cos \theta$ berechnet. Diese Werte brauchen nur einmal pro Projektion beaufschlagt zu werden und hierzu können sie leicht durch den Hilfsrechner 130 berechnet und in Halteregister geladen werden, bevor sie vom Bildrekonstruktor angefordert werden. Dieser Prozeß ist durch den Block 552 in Fig. 13 dargestellt. Während des Bildrekonstruktionsprozesses werden die x' und y'-Werte in Übereinstimmung mit den Gleichungen (28) gebildet, wie dies durch den Block 554 gezeigt ist.

Die transformierten x' und y'-Koordinaten werden zwei ROM's 556 und 558 zugeführt. In diesen Speichern werden Daten gespeichert, die repräsentativ für die Bewichtungsfunktion und für die Lagefunktion sind. Wie oben erläutert, sind diese Funktionen identisch für alle Projektionen und deshalb können sie einfach und schnell mittels in den ROM's gespeicherten Tabellen berechnet werden. In Abhängigkeit von der gewünschten Genauigkeit und Geschwindigkeit können alle Werte der Bewichtungs- und der Lagefunktionen in den ROM's gespeichert werden. Alternativ können die ROM's komprimierte Datentabellen enthalten, durch die Bewichtungs- und Lagefunktionen schnell berechnet werden können. Beispiele für solche komprimierte Funktionen sind z.B. stückweise lineare Näherungen und Taylor-Reihen-Entwicklungen. Die dargestellte Ausführungsform des Bildkonstruktors, die unten beschrieben ist, berechnet die Bewichtungs- und Lagefunktionen durch Speicherung der Werte dieser Funktionen in vielen diskreten Punkten und durch Interpolation zwischen diesen Punkten mittels zweidimensionaler, stückweise linearer oder quadratischer Entwicklung mit Kreuztermen der ersten Ordnung. Jeder der ROM's 556 und 558 erzeugt in Abhängigkeit von den x' und y'-Koordinaten, die den Adresseneingängen davon zugeführt werden, an seinem Ausgang die Bewichtungsfunktions- oder Lage-funktionswerte entsprechend der Lage, die durch die bits höherer Ordnung bestimmt ist. Die Entwicklungskoeffizienten für die Bewichtungs- und Lagefunktion sind einer Multiplikations- und Summationslogik zugeführt, wo die x' und y'-Werte und die Kreuzterme mit den entsprechenden Termen Multipliziert und die Produkte summiert werden, um die Lagefunktionswerte 564 und die Bewichtungsfunktionswerte 566 zu erzeugen.

Das Ausgangssignal des Multiplikations- und Summationskreises 560 ist der Wert der Lagefunktion und gibt an, welcher der 2048 interpolierten Werte dem Platz im Bildspeicher, der gerade verarbeitet wird, entspricht. Mit anderen Worten, die Lagefunktion bestimmt den speziellen interpolierten Datenwert, der am nächsten einem Strahl der Röntgenstrahlenquelle 10 entspricht, welcher durch das Zentrum des Pixels verläuft, das dem Platz im Bildspeicher, der bearbeitet wird, zugeordnet ist, wie dies oben in Verbindung mit Fig. 9 beschrieben ist.

Die Lagefunktion wird dem Adresseneingang des C/I-Speichers 122 zugeführt. Dementsprechend erzeugt der C/I-Speicher 122 an seinem Ausgang die zugeordneten interpolierten Daten. Diese Daten sind einem Eingang eines Multiplizierers 570 zugeführt. Der Multiplikations- und Summationskreis 562 erzeugt Daten an seinem Ausgang, die der Bewichtungsfunktion entsprechen, mit der die Daten an dem Punkt im Bildspeicher, die gerade verarbeitet werden, multipliziert werden müssen. Die Bewichtungsfunktion wird einem zweiten Eingang des Multiplizierers 570 zugeführt.

Der Multiplizierer 570 multipliziert entsprechend den zwei oben beschriebenen Signalen die gefalteten Daten mit der Bewichtungsfunktion, um einen Wert 572 zu erzeugen, der den entsprechend bewichteten Daten an seinem Ausgang zugeordnet ist. Dieser bewichtete Datenwert wird einem Eingang eines Summationskreises 574 zugeführt. Das andere Eingangssignal des Summationskreises sind die Daten des Bildspeichers, die dem während den vorangehenden Projektionen akkumulierten Wert entsprechen. Der Summationskreis 574 summiert die bewichteten Daten des Multiplizierers 570 mit den vorher im Bildspeicher 118 gespeicherten Daten und das resultierende Ausgangssignal des Summationskreises 574 wird den Dateneingängen des Bildspeichers zugeführt, wo es gespeichert wird und die vorher gespeicherten Daten ersetzt.

Die oben beschriebene Folge von Verarbeitungsschritten wird für jeden Platz im Bildspeicher wiederholt. Nachdem alle Plätze im Bildspeicher aufdatiert worden sind, stehen die gefalteten und interpolierten Daten der nächsten Projektion für den Bildrekonstruktor zur Verfügung und der obige Ablauf wird wiederholt. Dies erfolgt für alle Daten jeder Projektion, um die endgültigen Werte im Bildspeicher 118 zu erzeugen, die repräsentativ für die Dichte des abgetasteten Objekts sind.


Bildrekonstruktor-Koordinatentransformation

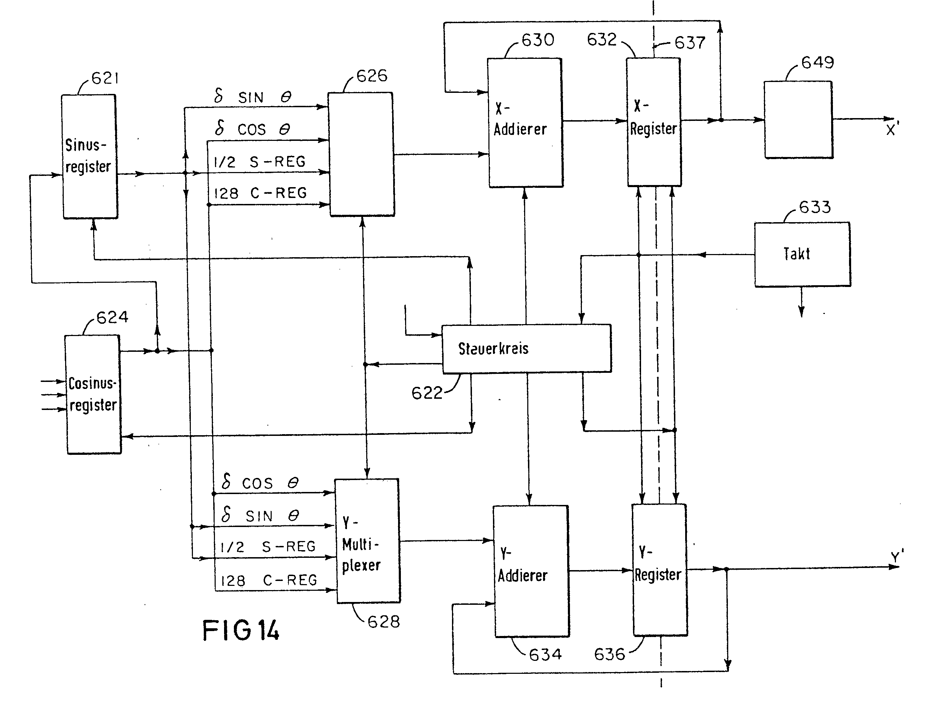

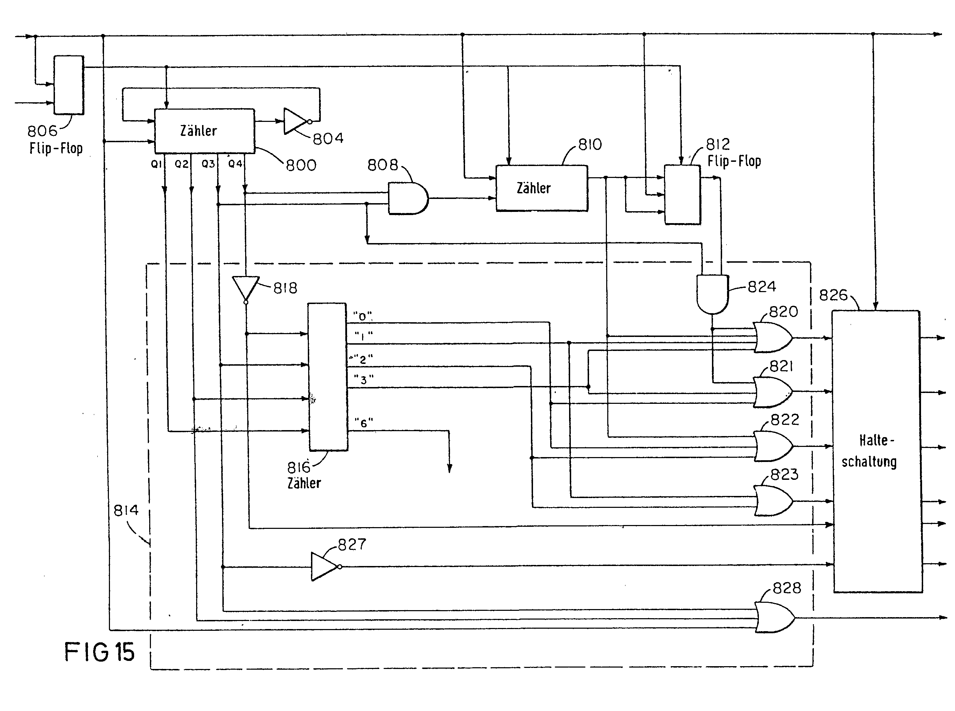

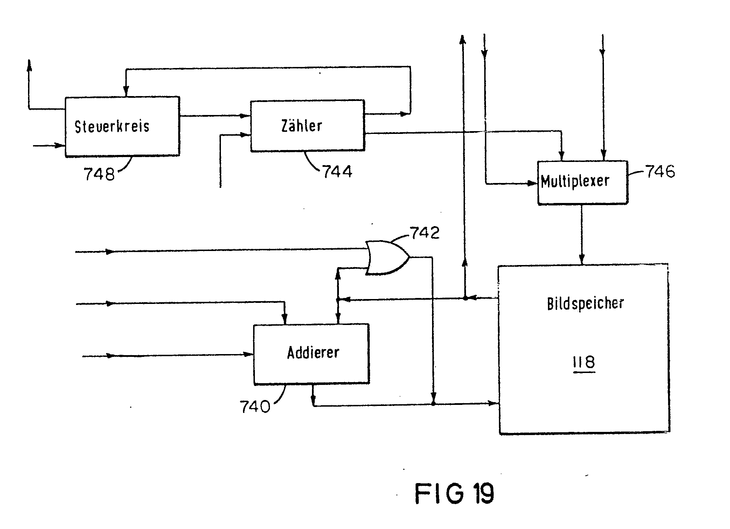

Die Fig. 14 bis 18 zeigen den detaillierten Aufbau einer bevorzugten Ausführungsform des Bildrekonstruktors. Fig. 15 zeigt den Kreis, der die Koordinatentransformationsfunktion im Bildrekonstruktor bildet, um die entsprechenden x' und y'-Koordinaten im Projektionskoordinatensystem zu

**0 004 258**

erzeugen. Für jede Projektion datiert der Bildrekonstruktor aufeinanderfolgend jede Lage in einem Bildspeicher auf. Beim Zugriff zu den Plätzen im Bildspeicher löst der Koordinatentransformationskreis die Gleichung (28), um die Projektionskoordinaten jedes Speicherplatzes zu bilden. Wenn die Koordinatentransformation durchgeführt worden ist, werden alle Rückprojektionsoperationen, die vom Bildrekonstruktorkreis gemäß Fig. 18 durchgeführt werden, im Projektionskoordinatensystem ausgeführt.

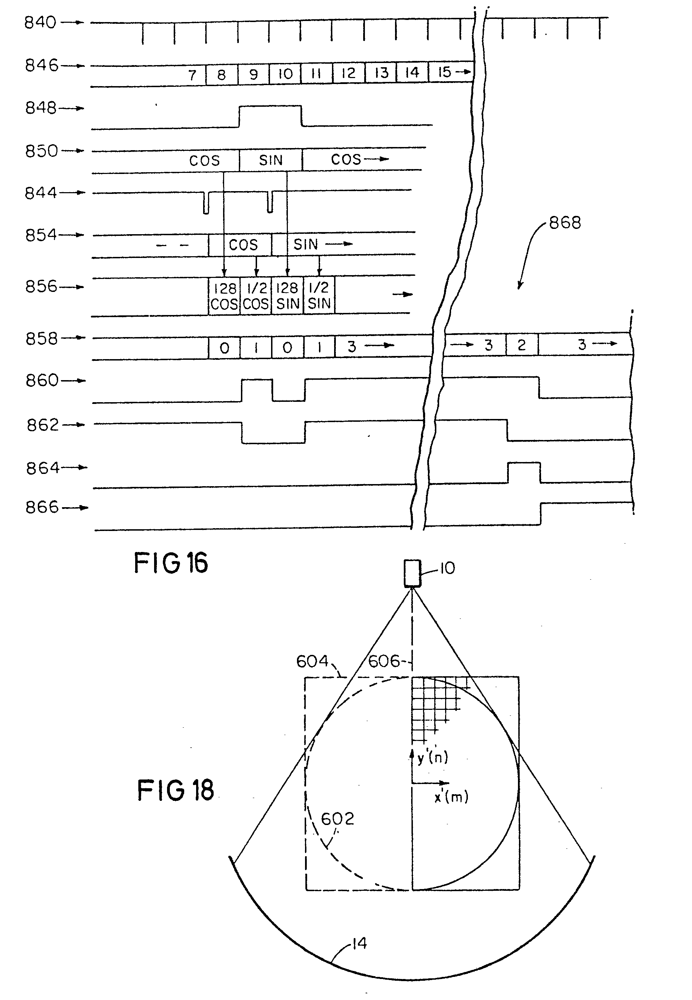

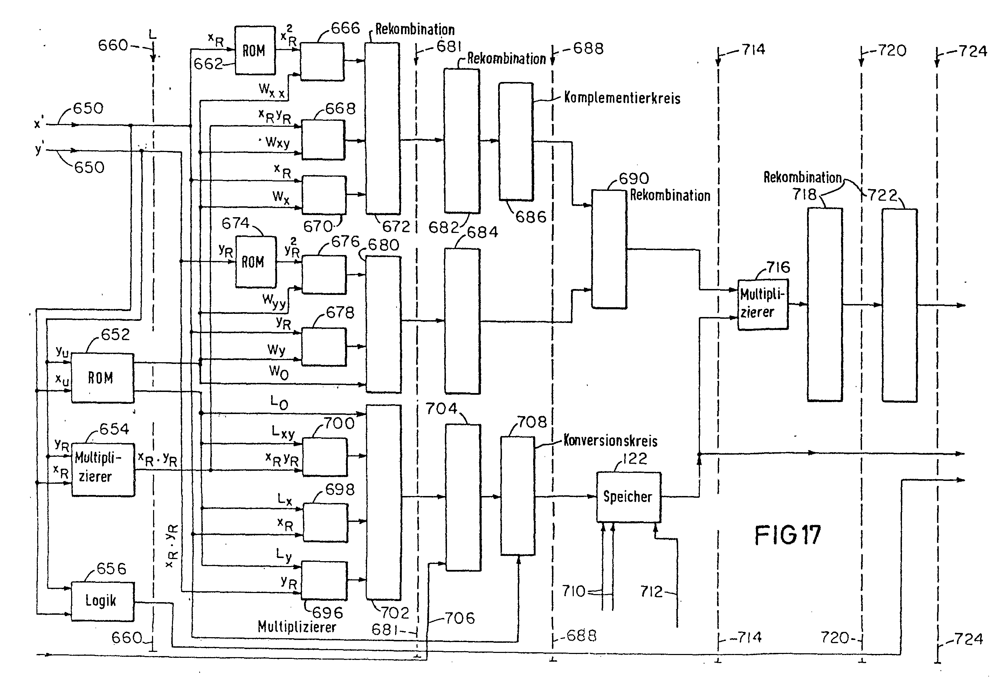

In Verbindung mit der Fig. 14 ist die Wirkungsweise des Koordinatentransformationskreises beschrieben. Vor dem Beginn eines Rekonstruktionsprozesses werden die $\delta \sin \theta$ und $\delta \cos \theta$-Werte der Gleichung (28) über das Zwischenglied 132·in ein Sinusregister 621 und ein Cosinusregister 624 geladen und die Projektionskoordinaten des ersten Platzes im Bildspeicher werden in zwei Register geladen, die mit X-Register 632 und Y-Register 636 bezeichnet sind. Die Art und Weise, in welcher diese Anfangswerte geladen werden, ist unten im Detail im Zusammenhang mit den Fig. 16 und 17 beschrieben. Die Sinus- und Cosinus-Daten der Register 621 und 624 werden zwei Eingängen zweier 4-zu-1-Multiplexer zugeführt, die als X-Multiplexer 626 und Y-Multiplexer 628 bezeichnet sind. Das Ausgangssignal des X-Multiplexers 626 wird einem ersten Eingang eines Addierers zugeführt, der mit X-Addierer 630 bezeichnet ist. Das Ausgangssignal des X-Addierers 630 wird einem X-Register 632 zugeführt und das Ausgangssignal des X-Registers 632 dem zweiten Eingang des X-Addierers 630. Das X-Register 632 wird durch ein 6,25 MHz-Taktsignal vom Bildrekonstruktor-(IR)-Takt 633 getaktet und durch Zufuhr des entsprechenden Signals zu dem Addier/Subtrahier-Eingang des X-Addierers 630 kann der im X-Register 632 akkumulierte Wert durch den Wert, der am Ausgang des X-Multiplexers 626 liegt, erhöht oder erniedrigt werden. In ähnlicher Weise wird das Ausgangssignal des Y-Multiplexers 628 einem ersten Eingang eines Addierers zugeführt, der als Y-Addierer 634 bezeichnet ist. Das Ausgangssignal des Y-Addierers 634 wird einem Y-Register 636 zugeführt und das Ausgangssignal des Y-Registers 636 dem zweiten Eingang des Y-Addierers 634. Die Addier/Subtrahier-Signale zu den X- und Y-Addierern 630 und 634 werden in einem Koordinatentransformationssteuerkreis 622 erzeugt, der unten näher beschrieben ist.

Fur den Beginn des Koordinatentransformationsprozesses für eine Projektion lädt die Steuereinrichtung 622 die Projektionskoordinaten des Anfangspunktes im Bildspeicher (Punkt 508 in Fig 12) in die X- und Y-Register 632 und 636. Wenn die Anfangswerte in die X- und Y-Register geladen sind, wird die Koordinatentransformation dadurch durchgeführt, daß die entsprechenden erhöhten Werte zu den vorher akkumulierten Werten in den X- und Y-Registern addiert werden, während jeder der Plätze im Bildspeicher aufeinanderfolgend adressiert wird. Gemäß Fig. 12 bewirkt zur Erzeugung der Koordinaten der Plätze in der ersten Zeile des Bildspeichers, die durch die gestrichelte Linie 638 in Fig. 12 dargestellt ist, der Steuerkreis 622, daß der X-Multiplexer 626 den $\delta \cos \theta$-Wert dem X-Addierer 630 und daß der Y-Multiplexer 628 den $\delta \sin \theta$-Wert dem Y-Addierer 634 zuführt. Die X- und Y-Addiererausgänge werden in die X- und Y-Register durch ein Taktsignal des I.R.Taktgebers 633 eingetaktet, um die nächsten x'- und y'-Werte zu erzeugen, die nach den Gleichungen (28a) und (28b) um entsprechende Werte erhöht sind. Dieses Verfahren wird fortgesetzt, bis der letzte Platz in der ersten Zeile des Bildspeichers (Punkt 646 in Fig. 12) adressiert worden ist. Die Koordinatentransformationssteuereinrichtung 622 bewirkt dann, daß der X-Multiplexer 626 den $\delta \sin \theta$-Wert dem X-Addierer 630 und der Y-Multiplexer 628 den $\delta \cos \theta$-Wert dem Y-Addierer 634 zuführt und führt ein Subtraktionssignal dem Addier/Subtrahiereingang des Y-Addierers 634 zu. Der nächste Taktimpuls bewirkt, daß die entsprechenden Werte zu den x'- und y'-Koordinaten, die in den X- und Y-Registern gespeichert sind, addiert werden, und zwar entsprechend dem Inkrement zwischen dem letzten Platz in der ersten Reihe des Bildspeichers und dem letzten Platz in der zweiten Reihe des Bildspeichers. Die entsprechenden Punkte in Fig. 12 sind die Punkte 646 und 647. Darauffolgend bewirkt der Steuerkreis 622, daß die X- und Y-Multiplexer den $\delta \cos \theta$ und den $\delta \sin \theta$-Wert den X- und Y-Addierern entsprechend wieder zuführen und daß Subtraktionssignale an den Addier/Subtrahier-Eingängen beider Addierer liegen. Die folgenden Taktsignale des I.R.-Taktgebers 633 erzeugen die Projektionskoordinaten der Plätze in der zweiten Zeile des Bildspeichers, der Zeile 644 in Fig. 12. Dieses Verfahren wird wiederholt, bis jeder Platz im Bildspeicher adressiert worden ist.

Das Cosinusregister 624 ist ein Zweibank-, Zweitorspeicher. Dieser Speicher ist im wesentlichen der gleiche wie der Zweibank-C/C und C/I-Speicher mit der Ausnahme, daß jede Bank nur zwei Plätze hat. Die $\delta \sin \theta$-Daten und $\delta \cos \theta$-Daten werden in die zwei Register einer Bank des Speichers 624 geladen, während die vorher geladenen $\delta \sin \theta$-Daten und $\delta \cos \theta$-Daten in der anderen Bank am Ausgang des Speichers 624 zur Verfügung stehen. Der Steuerkreis 622 führt ein Eingangssignal dem Speicher 624 zu, welches auswählt, ob die Sinus- oder Cosinusdaten der Ausgangsbank am Ausgang erscheinen. Das Ausgangssignal des Sinus-Registers 621 wird dem vierten Eingang sowohl des X- als auch des Y-Multiplexers zugeführt, und zwar um einen bit verschoben, um Werte zu erzeugen, die gleich sind der Hälfte der Werte im Sinusregister 622.

Für den Beginn eines Koordinatentransformationszyklusses für eine bestimmte Projektion lädt der Koordinatentransformationskreis die Projektionssystemkoordinaten des ersten Platzes im Bildspeicher in die X- und Y-Register 632 und 636. Der Steuerkreis 622 steuert dies, indem er entsprechende Signale den Sinus- und Cosinus-Registern, den X- und Y-Multiplexern un den X- und Y-Addierern und Registern zuführt, um die Werte zu erzeugen, die gegeben sind durch:

19

$$x' = -127 - 1/2 \cdot \delta \cos \theta - 127 - 1/2 \cdot \delta \cos \theta$$

$$y' = 127 - 1/2 \cdot \delta \cos \theta - 127 - 1/2 \cdot \delta \sin \theta$$
(29)

und die in jedem der X- und Y-Register akkumuliert werden sollen. Diese Werte entsprechen den Projektionskoordinaten des Zentrums des Pixels, das dem Anfangsplatz im Bildspeicher zugeordnet ist, wobei das Zentrum des Pixels das Ergebnis der Addition oder Subtraktion der $1/2\ \delta \cos \theta$ oder $1/2\ \delta \sin \theta$-Faktoren ist.

In Fig. 15 ist der Steuerkreis 622 im Detail gezeigt. Der Steuerkreis wird durch einen Vier-bit-Folgezähler 800 fortgeschaltet. Das 6,25 MHz Taktsignal des I.R.-Taktgebers 633 wird dem Takteingang des Zählers 800 zugeführt. Das Ausgangssignal des Zählers 800 wird über einen Inverter 804 einem Aktivier-Eingang des Zählers zugeführt. Dieses Ausgangssignal geht in ein hohes Niveau über, wenn der Zähler 15 erreicht. Der 1111-Zustand disaktiviert den Zähler und verhindert, daß er durch das Taktsignal weitergeschaltet wird, bevor er zurückgesetzt ist. Ein I.R.-Aktivierungssignal vom Zwischenkreis 132 wird einem Flip-Flop 806 zugeführt, welches durch einen Takt 633 weitergeschaltet wird und das I.R.-Aktivierungssignal mit dem Bildrekonstruktortakt synchronisiert. Das synchronisierte I.R.-Aktivierungssignal vom Flip-Flop 806 wird einem Voreinstell-Aktivierungseingang des Zählers 800 zugeführt. Die Voreinstelleingänge des Zählers 800 sind so verbunden, daß der Wert 7 (0111) durch den Voreinstell-Aktivierungseingang in den Zähler geladen wird.

Vor dem Beginn eines Bildrekonstruktionszyklusses ist das I.R.-bereit-Signal vom Zwischenkreis 132 auf hohem Niveau, hält den Zähler 800 in dem 7-Zustand und verhindert, daß er durch das Signal des Taktgebers 633 weitergeschaltet wird. Für den Beginn eines Bildrekonstruktionszyklusses bewirkt der Zwischenkreis 132, daß das I.R.-bereit-Signal auf sein niedriges Niveau übergeht. Der Zähler 800 beginnt dann mit der Zählung durch die Zustände 7 bis 15. Wenn der Zähler den 15-Zustand erreicht, verhindert das Ausgangssignal, das durch den Invertierer 804 invertiert wird, daß der Zähler 800 weiterzählt und er verbleibt in dem 15-Zustand, bis das I.R.-bereit-Signal wieder auf sein hohes Niveau zurückgeht, was am Ende des Bildrekonstruktionszyklusses für eine bestimmte Projektion der Fall ist. Auf diese Weise definiert der Zähler 800 verschiedene Zustände am Beginn der Bildrekonstruktion für jede Projektion und während dieser anfänglichen Zustände werden die Anfangswerte in das X-Register und Y-Register 632 und 636 geladen.

Die Q3- und Q4-Ausgangssignale des Zählers 800 werden einem UND-Gatter 808 zugeführt. Das Ausgangssignal des UND-Gatters 808 bewirkt, daß der Eingang eines 7-bit-Zählers 810 aktiviert wird. Der Zähler 810 wird durch das Signal des I.R.-Taktgebers 633 getaktet. Wenn der Zähler 800 den Zustand 12 erreicht, geht das Ausgangssignal des UND-Gatters 808 auf ein hohes Niveau und der Zähler 810 wird aktiviert. Der Zähler 810 wird dann durch jeden der folgenden Taktpulse des I.R.-Taktgebers 633 weitergeschaltet. Der Zähler 810 zählt die Plätze in jeder Reihe des Bildspeichers und erzeugt ein Signal an seinem Ausgang, wenn er den Zustand 255 erreicht. Dieses Signal bezeichnet das Ende einer Zeile (EOL) im Bildspeicher. Das EOL-Signal ist einem Flip-Flop 812 zugeführt, welches durch den I.R.-Taktgeber 633 getaktet wird und seinen Zustand am Ende jeder Zeile ändert. Das Ausgangssignal des Flip-Flop 812 gibt an, ob eine ungerade oder gerade Zeile rekonstruiert wird.

Die Ausgangssignale des Zählers 800, das EOL-Signal und das ungerade/gerade-Zeile-Signal werden der Kombinationslogik zugeführt, die in der gestrichelten Umrandung 814 ist und die diese Signale decodiert, um die Ausgangssignale des Steuerkreises 622 zu bilden. Die drei letzten signifikanten bits vom Zähler 800 werden dem Decoder 816 zugeführt und der vierte bit wird durch den Inverter 818 invertiert und einem Decoder 816 zugeleitet. Der Decoder 816 ist ein 10-Zustände-Decoder. Aufgrund der Inversion des vierten bit des Zählers 800 durch den Inverter 818 gehen die vier Ausgangssignale zum Decoder 816 von den Zuständen 15 bis 7, während der Zähler durch die Zustände 7 bis 15 geht. Der Decoder 816 hat zehn Ausgangssignale, von denen eines entsprechend einem decodierten BCD-Eingang, der von Null bis 9 geht, auf hohem Niveau ist. Für Werte, die größer als 9 sind, sind alle zehn Ausgangssignale des Decoders 816 niedrig. Die "0"-bis "3"-Ausgangssignale des Decoders 816 werden ODER-Gattern 820 bis 823 zugeführt, wie dies in der Fig. 16 dargestellt ist, um die unten beschriebenen Ausgangssignale zu erzeugen. Zusätzlich werden das EOL-Signal und das ungerade/gerade-Zeile-Signal des Flip-Flops 812 einer Kombinationslogik 814 zugeführt. Das ungerade/gerade-Zeile-Signal wird durch den dritten bit des Zählers 800 über ein UND-Gatter 824 durchgeschaltet.

Die Kombinationslogik 814 erzeugt die folgenden Signale. Das ODER-Gatter 820 bildet das Addier/Subtrahier-Signal, das dem X-Addierer 630 zugeführt wird. Das ODER-Gatter 621 erzeugt das Addier/Subtrahier-Signal, das dem Y-Addierer 634 zugeführt wird. Das ODER-Gatter 822 erzeugt den kleinsten signifikanten bit des 2-bit-Auswahlsignals, das den X- und Y-Multiplexern 626 und 628 zugeführt wird. Das ODER-Gatter 623 erzeugt das Wählsignal, das dem Cosinus-Register 624 zugeführt wird. Das vom Inverter 818 invertierte Q4-Ausgangssignal des Zählers 800 erzeugt das Rückstellsignal, das die Rückstellung der X- und Y-Register 632 und 636 bewirkt. Das Q3-Ausgangssignal vom Zähler 800 wird durch den Inverter 827 invertiert, um den signifikantesten bit des 2-bit-Wählsignals zu erzeugen, das den X- und Y-Multiplexern 626 und 628 zugeführt wird. Die oben beschriebenen Signale werden Halteschaltungen 826 zugeführt, in die sie durch ein Taktsignal vom I.R.-Taktgeber 633 zur Weiterleitung an den Rest des Koordinatentransformationskreises gemäß Fig. 14 eingetaktet werden. Das Taktsignal für das Sinus-Register 622 wird erzeugt, indem das Taktsignal vom Taktgeber 802 mit den Q2- und Q3-Ausgangssignalen des Zählers 800 im ODER-Gatter 828 getastet wird.

Das "6"-Ausgangssignal des Decoders 816 wird dem Bildspeichersteuerkreis 748, der unten beschrieben ist, zugeführt und bewirkt, daß der Bildspeichersteuerkreis einen Zyklus durch den Bildspeicher startet. Die Verwendung des "6"-Ausgangssignals vom Decoder 816 kompensiert die Verzögerung, die durch die Taktung der Daten durch die verschiedenen Pipeline-Stufen des Bildrekonstruktionskreises und des Bildspeichers verursacht ist.

Im Zusammenhang mit den Kurven gemäß Fig. 16 ist die Wirkungsweise des Koordinatentransformationskreises der Fig. 14 im Detail beschrieben. Die Kurve 840 ist repräsentativ für das Signal vom I.R.-Taktgeber 633. Das Taktsignal ist hier als schmaler Puls gezeigt, aber es können auch andere Taktsignale, wie Rechteckwellen, benutzt werden. Für den Beginn eines Bildrekonstruktionsprozesses bewirkt das Zwischenglied 132, daß das I.R.-bereit-Signal auf hohes Niveau geht. Dementsprechend geht das Ausgangssignal des Flip-Flop 806 auf niedriges Niveau. Der Zähler 800, der vorher im 7-Zustand durch das Ausgangssignal des Flip-Flops 806 gehalten worden ist, wird nun durch die folgenden Taktimpulse schrittweise erhöht. Der Zähler 800 geht durch seine einzelnen Zustände, bis er 15 erreicht, wo er durch das invertierte Ausgangssignal gehalten wird, das dem Aktivierungseingang des Zählers 800, wie oben beschrieben, zugeführt wird. Die Zählerzustände sind in der Zeile 846 gezeigt. (Man beachte, daß wegen der 1-bit-Verzögerung in den Signalen 848 bis 862 aufgrund der Halteschaltung 826 die Zählerzustände um einen bit gegenüber dem aktuellen Auftreten der bits versetzt sind, um die vorliegende Erklärung zu erleichtern.)

Das vom Gatter 823 gewählte Eingangssignal des Cosinus-Registers ist in Zeile 848 und die resultierenden Daten, die am Ausgang des Cosinus-Registers 624 erscheinen, $\delta \cos \theta$ oder $\delta \sin \theta$, sind in Zeile 850 dargestellt. Das Taktsignal, das dem Sinus-Register 621 über das ODER-Gatter 828 zugeführt wird, ist durch die Kurve 844 dargestellt. Das Ausgangssignal des Cosinus-Registers 624 ist dem Eingang des Sinus-Registers 621 zugeführt, so daß das Sinus-Register-Taktsignal 852 die Daten, die am Ausgang des Cosinus-Registers erscheinen, und die durch die Kurve 850 dargestellt sind, in das Sinus-Register eintaktet. Das resultierende Ausgangssignal vom Sinus-Register 621 ist durch die Kurve 854 dargestellt.

Die Wahleingangssignale der X- und Y-Multiplexer 626 und 628 wählen die Multiplexereingänge, wie dies durch die Linie 858 dargestellt ist. Die Multiplexereingänge sind mit "0" bis "3" bezeichnet, wie dies die Fig. 14 zeigt. Während der Periode, während der die X- und Y- Register initiiert werden, wählen die X- und Y-Multiplexer alternativ die Eingänge entsprechend dem 128-fachen des Cosinus-Registerausgangssignals und dem halbfachen des Sinus-Registerausgangssignale, wie dies in der Fig. 16 dargestellt ist. Die X- und Y-Addier/Subtrahier-Signale werden durch einen Steuerkreis 622 in Übereinstimmung mit den Kurvenformen 860 und 862 erzeugt. Das Ergebnis dieser Operation ist, daß die entsprechenden Projektionskoordinaten des Anfangsplatzes im Bildspeicher, die durch die Gleichung (29) gegeben sind, in den X- und Y-Registern 632 und 636 während der ersten vier I.R.-Taktperioden, die dem Hochgehen des I.R.-bereit-Signals folgen, akkumuliert werden.

Auf die X- und Y-Register-Initiierung folgend erhöht der Koordinatentransformationskreis die Werte in den X- und Y-Registern um entsprechende Werte, wie dies oben beschrieben ist. Am Ende einer Zeile geht das EOL-Signal vom Zähler 810 auf hohes Niveau und bewirkt eine Anzeige; beim Beginn der folgenden Zeile wechselt der ungerade/gerade-Ausgang des Flip-Flops 812 in Abhängigkeit davon, ob die Zeile ungerade oder gerade ist. Die EOL- und ungerade/gerade-Signale sind durch die Kurven 864 und 866 wiedergegeben. Die Wirkungsweise des Steuerkreises 622 am Ende der ersten Zeile ist durch die Kurve in dem mit 868 bezeichneten Bereich gezeigt und die Multiplexeradressenwerte und X- und Y-Addier/Subtrahier-Signale, die dargestellt sind, resultieren aus einer richtigen Addition von Werten, wie dies oben beschrieben ist.

Bildrekonstruktor-Rückprojektor

Die Fig. 17 stellt im Detail eine bevorzugte Ausführungsform des Rückprojektionskreises des Bildrekonstruktors dar. Für eine wirtschaftliche und schnelle Berechnung der Werte der Lage- und Bewichtungsfunktionen werden zweidimensionale Gebilde benutzt, um diese Funktionen über den Bildbereich zu erzeugen. Diese Gebilde sind in 512 Unterbereiche, Zellen genannt, unterteilt, welche in einem rechteckigen Array angeordnet sind; 32 Zellen parallel zum zentralen Röntgenstrahl und 16 Zellen senkrecht zu dieser Richtung.

Dies ist in der Fig. 18 gezeigt. Hier ist dargestellt, daß der kreisförmige Bereich 602, der dem endgültigen Bild entspricht, umgeben ist von einem quadratischen Bereich 604. Während Daten in den Ecken des Quadrates 604 evtl. eliminiert werden, da sie außerhalb des endgültigen Bildes liegen, ist der Rückprojektionskreis wesentlich einfacher ausgebildet, derart, daß der gesamte quadratische Bereich 604 während jeder Projektion abgetastet wird. Der Bereich 604 ist in 1024 quadratische Unterbereiche unterteilt, die Zellen genannt sind, wobei 32 Zellen auf einer Seite liegen. Aufgrund der Tatsache, daß sowohl die Bewichtungsfunktion als auch die Lagefunktion symmetrisch für ein positives und negatives x zum Zentralstrahl 606 der Röntgenstrahlenquelle 10 liegen, sind die Werte dieser Funktionen identisch für positive und negative x. Deshalb brauchen die Lageund Bewichtungswerte nur für die 512 Zellen gespeichert zu werden, die aus dem 16×32 Zellen-Array bestehen, das rechts der Zentrallinie 606 liegt.

Bei der Berechnung der Bewichtungsfunktion für einen Platz, der die Projektionskoordinaten x' und y' hat, wird jede dieser Koordinaten in einen am meisten signifikanten Teil von 5 bits und einen am wenigsten signifikanten Teil, der die verbleibenden bits enthält, geteilt. Die fünf am meisten signifikanten bits der x-Koordinate und die vier am meisten signifikanten bits der y-Koordinate identifizieren allein, welche der 512 Zellen des Punktes innen liegt. Die am meisten signifikanten Teile der x'- und y'-Koordinaten sind mit m und n bezeichnet. Die verbleibenden am wenigsten signifikanten bits der x/y-Koordinaten geben an, wo in einer speziellen Zelle (m, n) der Punkt gelegen ist; diese weniger signifikanten bits, die Reste genannt, sind mit $x_R$ und $y_R$ bezeichnet. Die Bewichtungsfunktion wird nach der folgenden Gleichung berechnet:

$$W=W_o(m, n)+W_x(m, n) \cdot x_R+W_y(m, n) \cdot y_R+W_{xy}(m, n) \cdot x_R \cdot y_R+W_{xx}(m, n) \cdot x_R{}^2+W_{yy}(m, n) \cdot X_R^2 \qquad (30)$$

wobei m und n die Koordinaten der Zelle mit m zwischen 1 und 16 und n zwischen 1 und 32 und die W-Werte die Ausdehnungskoeffizienten für die Zelle (m, n) sind. Die Gleichung (30) erfordert, daß sechs konstante Bildzellen gespeichert werden, nämlich $W_o$ (m, n), $W_x$ (m, n), $W_y$ (m, n), $W_{xy}$ (m, n), $W_{xx}$ (m, n) und $W_{yy}$ (m, n). Diese Konstanten werden in einem großen ROM mit 512 Plätzen und 60 bits pro Platz gespeichert.

Die Lagefunktion wird nach folgender Gleichung berechnet:

$$L=L_o(m, n)+L_x(m, n) \cdot x_R+L_y(m, n) \cdot y_R+L_{xy}(m, n) \cdot x_R y_R \qquad (31)$$

wobei L der Wert der Lagefunktion, m und n die Koordinaten der Zelle und die L-Werte die Ausdehnungskoeffizienten für die Zelle m, n sind. Die Lagefunktion ist eine flachere und weniger schnell sich ändernde Funktion als die Bewichtungsfunktion und demgemäß sind nur Terme erster Ordnung und ein Kreuzterm für die gewünschte Genauigkeit erforderlich. Die vier Ausdehnungskoeffizienten für jede Zelle $L_o$ (m, n), $L_x$ (m, n), $L_y$ (m, n) und $L_{xy}$ (m, n) werden in einem ROM mit 512 Plätzen und 36 bits für jeden Platz gespeichert.

Die Projektionskoordinaten x' und y' des Koordinatentransformationskreises der Fig. 13 bestehen je aus 18-bit-Werten. Diese x'- und y'-Werte werden dem Rückprojektionskreis, der in der Fig. 18 gezeigt ist, zugeführt. Die Daten sind ferner in "obere" und "untere" Gruppen geteilt. Die oberen Gruppen schließen die fünf am meisten signifikanten bits für die Zeichen- und Größeninformation ein und entsprechen den oben beschriebenen Koordinaten m und n der Zellen. Diese Werte sind mit $x_U$ und $y_U$ bezeichnet. Die unteren Gruppen entsprechen den oben beschriebenen Resten und schließen die zwölf am wenigsten signifikanten bits ein. Die Reste sind bezeichnet mit $x_R$ und $y_R$.

Fünf bits des $y_U$-Wertes und vier bits des $x_U$-Wertes (die Zeichenbits sind nicht eingeschlossen) werden dem ROM 652 zugeführt. ROM 652 ist ein 512×96 Speicher. Aufgrund der Symmetrie der Bewichtungs- und Lagefunktionen für positive und negative Werte ist es nicht erforderlich, daß das Zeichen der x'-Koordinate bei der Durchführung der Bewichtungs- und Lagefunktionen zur Verfügung steht. ROM 652 enthält die Werte der Koeffizienten der Bewichtungs- und Lagefunktion für jede der Zellen. Entsprechend den $x_U$- und $y_U$-Werten, die seinen Adresseneingängen zugeführt werden, erzeugt das ROM 652 an seinen Ausgängen 60 bits von Bewichtungsfunktionskoeffizienten und 36 bits von Lagefunktionskoeffizienten.

Die acht am meisten signifikanten bits jedes der $x_R$-und $y_R$-Werte sind einem Multiplizierer 654 zugeführt. Der Multiplizierer 654 ist ein 8×8-Multiplizier, der einen 8-bit-Wert an seinem Ausgang erzeugt, der gleich ist $x_R$ mal $y_R$, was zur Berechnung der Kreuzterme in den Bewichtungs- und Lagefunktionen benutzt ist. Die Bereich überschreitenden bits jedes der x'- und y'-Werte sind einer Logik ORI 656 zugeführt, die angibt, was außerhalb des rekonstruierten Bildes liegt. Wenn ein eine Bereichsüberschreitung angebender bit positiv ist, geht der ORI-bit-Ausgang der Logik 656 hoch und zeigt an, daß der verarbeitete Wert außerhalb des endgültigen Bildes, das vom Tomographieprozessor erzeugt wird, liegt. Der ORI-bit geht durch den Bildrekonstruktor und wird einem Bildspeicher zugeführt, wie dies unten beschrieben ist, so daß diese außerhalb des endgültig rekonstruierten Bildes liegenden Werte nicht angezeigt werden. Die Ausgänge des ROM 652, des Multiplizierer-arrays 654, der ORI-bit-Logik 656 und die $x_R$- und $y_R$-Werte werden in Pipeline-Halteschaltungen, die durch gestrichelte Linien 660 dargestellt sind, eingetaktet, wo diese Werte für die Verarbeitung durch die nächste Stufe des Pipeline-Prozessors gehalten werden.

Die Multiplizier-Arrays und die Wallace-Baum-Rekombinationskreise, die unten beschrieben sind, sind ähnlich jenen, die oben in Verbindung mit dem Faltungsrechnerkreis dargestellt und beschrieben sind.

Die nächsten drei Stufen des Pipeline-Prozessors führen die Multiplikation der x'- und y'-Werte mit den Koeffizienten, die im ROM 652 gespeichert sind, aus, um die Lage- und Bewichtungsfunktionen in Übereinstimmung mit den Gleichungen (30) und (31) zu berechnen.

22

Der $x_R$-Wert wird einem Quadrier-ROM 662 zugeführt, der ein 8-Bit-Ausgangssignal, das gleich dem Quadrat seines Eingangssignals ist, erzeugt. Das $x_R 2$-Ausgangssignal des ROM 662 ist einem Eingang eines Multiplizier-Arrays 666 zugeführt. Das andere Eingangssignal des Multiplizier-Arrays 666 ist ein Sieben-bit-Wert vom ROM 652, welcher der $W_{xx}$-Koeffizient des Terms zweiter Ordnung $x_R 2$ in der Bewichtungsfunktion ist. Das Multiplizier-Array 666 ist ein 7×8-Multiplizier-Array und erzeugt entsprechend seinen Eingangssignalen den $W_{xx} . x_R^2$ Term der Gleichung (30).

Das 8-bit-Ausgangssignal $x_R . y_R$ des Multiplizierers 654 ist dem einen Eingang eines 7×8-Multiplizier-Arrays 668 zugeführt. Der $W_{xy}$-Koeffizient des ROM 652 ist dem zweiten Eingang des Multiplizierers 668 zugeführt, welcher den entsprechenden Term an seinem Ausgang erzeugt. Der 12-bit-Wert $x_R$ ist dem einen Eingang eines 10×12-Multiplizier-Arrays 670 zugeführt. Ein 10-bit-Wert $W_x$ des ROM 652 ist dem anderen Eingang des Multiplizier-Arrays 670 zugeführt, welches den entsprechenden Term an seinem Ausgang erzeugt. Die drei Ausgänge der Multiplizier-Arrays 666, 668 und 670 sind einem Bewichtungsfunktionsrekombinationskreis 672 eines ersten Niveaus zugeführt, welcher die ersten Stufen der Rekombination seiner Eingänge erzeugt.

Der $y_R$-Wert ist einem zweiten Quadrier-ROM 674 zugeführt, welches an seinem Ausgang einen $y_R^2$-Wert erzeugt. Der $y_R^2$-Wert ist einem Eingang eines 8×8-Multiplizier-Arrays 676 zugeführt, welches ihn mit einem 8-bit-Wert $W_{yy}$ von dem ROM 652 multipliziert. Ein 12×12-Multiplizier-Array 678 multipliziert die 12-bit-Werte $y_R$ und $W_y$, die an seinen Eingängen liegen, um ein Ausgangssignal zu erzeugen, das repräsentativ für die entsprechenden Terme der Bewichtungsfunktion in Gleichung (30) ist.

Die Ausgangssignale der Multiplizier-Arrays 676 und 678 werden einem zweiten Bewichtungsfunktions-Rekombinationskreis 680 eines ersten Niveaus zugeführt. Ferner wird dem Bewichtungsfunktions-Rekombinationskreis 680 ein 16-bit-Wert $W_0$ zugeführt, der gleich ist dem Koeffizienten der O-ten Ordnung der Bewichtungsfunktion für die in Frage stehende Bild-Array-Zelle.

Das Ausgangssignal der Bewichtungsfunktions-Rekombinationskreise 672 und 680 der ersten Ordnung wird in die Pipeline-Haltekreise 681 eingetaktet, wo er gehalten wird, um durch Rekombinationskreise 682 und 684 der zweiten Ordnung verarbeitet zu werden. Aufgrund der Charakteristiken der Bewichtungsfunktion sind die drei Terme der Gleichung (30), die dem Rekombinationskreis 672 des ersten Niveaus zugeführt werden, immer negativ und die zwei Terme, die dem Rekombinationskreis 680 des ersten Niveaus zugeführt werden, immer positiv. Diese Terme werden entsprechend dem ersten und dem zweiten Niveau der Rekombination verarbeitet, und zwar positive und negative Terme separat und das Ausgangssignal des Rekombinationskreises 682 des zweiten Niveaus wird einem Komplementierkreis 686 zugeführt, um das Ausgangssignal des Rekombinationskreises 682 des zweiten Niveaus umzukehren. Die Ausgangssignale des Komplementierkreises 686 und des Rekombinationskreises 684 werden im Pipeline-Haltkreis, der durch die gestrichelte Linie 688 gekennzeichnet ist, gehalten und werden einem Bewichtungsfunktions-Rekombinationskreis 690 eines dritten Niveaus zugeführt. Das Ausgangssignal des Rekombinationskreises 690 ist der Wert der Bewichtungsfunktion.

Die Lagefunktion wird in derselben Weise wie die Bewichtungsfunktion berechnet. Wegen der geringeren Komplexität der Lagefunktion sind nur zwei Niveaus der Rekombination und zwei Pipeline-Stufen erforderlich. Die gefalteten Daten, die im C/I-Speicher 122 gespeichert sind, werden von der Lagefunktion während des Pipeline-Rechenschritts aufgerufen, die für den Rekombinationskreis des dritten Niveaus der Bewichtungsfunktionsberechnung erforderlich ist.

Zur Berechnung der Lagefunktion werden acht bits des $y_L$-Wertes und ein 8-bit-Wert $L_y$ einem 8×8-Multiplizier-Array 696 zugeführt und alle zwölf bits des $x_R$-Wertes und ein 10-bit-Wert $L_x$ werden einem 10×12-Multiplizier-Array 698 zugeführt, um die Terme erster Ordnung der Lagefunktion zu erzeugen. Die unterschiedlichen bit-Zahlen der $x'$ und $y'$-Werte und -Koeffizienten der ersten Ordnung resultieren aus der Tatsache, daß die erste Ableitung der Lagefunktion in einer Richtung größer ist als in der anderen; die beschriebene Zahl von bits ist erforderlich, damit die endgültige Lagefunktion die erforderliche Präzision hat.

Der $x_R . y_R$-Wert des Multiplizier-Arrays 654 wird dem einen Eingang eines 5×8-Multiplizier-Arrays 700 zugeführt. Ein 5-bit-Wert $L_{xy}$ vom ROM 652 wird dem anderen Eingang des Multiplizier-Arrays 700 zugeführt. Die Ausgangssignale der Multiplizier-Arrays 696, 698 und 700 und ein 13-bit-Wert $L_0$, der gleich ist dem Term der O-ten Ordnung der Gleichung (31), werden einem Lage-Rekombinationskreis 702 eines ersten Niveaus zugeführt.

Die Ausgangssignale des Lage-Rekombinationskreises 702 des ersten Niveaus werden in eine Pipeline-Halteschaltung 681 eingetaktet und einem Rekombinationskreis 704 eines zweiten Niveaus zugeführt. Zusätzlich wird eine Versetzungskonstante, die vom Computer 130 über den Zwischenkreis 132 geliefert wird, auf der Leitung 706 dem Lage-Rekombinationskreis 704 des zweiten Niveaus zugeführt. Diese Konstante kann benutzt werden, um zu der Lagefunktion einen Wert zu addieren, der mechanische Verlagerungen des Detektor-Arrays kompensiert. Der Koeffizient $L_0$ der O-ten Ordnung des ROM 652 hat einen konstanten Faktor, der dem Abstand zweier Detektoren entspricht. Auf diese Weise kann die Versetzungskonstante positive und negative Verlagerungen kompensieren, ohne daß es notwendig ist, eine Addition für die Verlagerungskonstante durchzuführen.

23

# O 004 258

Das Ausgangssignal des Lage-Rekombinationskreises 704 des zweiten Niveaus wird einem Konversionskreis 708 zugeführt. Auch das x'-bit wird dem Konversionskreis 708 zugeführt. Wie oben beschrieben, ist die Lagefunktion für positive und negative Werte von x' symmetrisch. Der Zeichen-bit des x'-Wertes des Koordinatenkonversionskreises wird in den oben beschriebenen Berechnungen nicht benutzt, aber er wird dem Konversionskreis 708 zugeführt, um ein Ausgangssignal des ordnungsgemäßen Zeichens zu bilden. Wie oben im Zusammenhang mit der Erklärung des Koordinatentransformationskreises beschrieben, wird der x'-Wert in einer Zeichen/Größe-Repräsentation berechnet. Der Konversionskreis 708 wandelt diese Zeichen/Größe-Repräsentation in eine zweite komplementäre Präsentation um. Dieser Wert kann dann umgekehrt werden oder nicht, indem die bits des Ausgangssignals des Rekombinationskreises 704 des zweiten Niveaus invertiert werden oder nicht, entsprechend dem Wert des Zeichen-bits. Das Ausgangssignal des Konversionskreises 708 ist der Lagefunktionswert und er wird in den Pipeline-Haltekreis 688 eingetaktet.

Es sollte beachtet werden, daß der Zeichenwert x', obwohl er nicht in den ersten zwei Stufen des Pipeline-Prozessors gemäß Fig. 15 verarbeitet wird, trotzdem in die Haltekreise 660 und 681 zwischen diesen Stufen eingetaktet wird, so daß diese Werte durch den Pipeline-Prozessor synchron mit den entsprechenden Daten, die in jeder dieser Stufen verarbeitet werden, hindurchwandern. Die Versetzungskontante ändert sich nicht und braucht deshalb nicht durch die Pipeline-Haltekreise getaktet zu werden.

Der Lagefunktionswert im Verriegelungskreis 688 wird dem C/I-Speicher 122 zugeführt. Der C/I-Speicher 122 arbeitet in genau derselben Weise wie der C/C-Speicher 120, die im Detail beschrieben und in der Figur gezeigt sind, mit der Ausnahme, daß die Bänke des C/I-Speichers 2048 18-bit-Worte enthalten. Entsprechend dem Lagefunktionswert, der seinen Leseadressen-Eingängen zugeführt wird, erzeugt der C/I-Speicher 122 an seinem Ausgang den entsprechenden Datenpunkt aus den 2048 gefalteten und interpolierten Datenpunkten, die darin gespeichert sind. Die Daten, die vom Bildrekonstruktor zur Durchführung jeder Rückprojektion angefordert werden, werden in den C/I-Speicher 122 während der vorhergehenden Projektion durch den Faltungsrechner 114 über Daten- und Adressenleitungen 710 geladen, wie dies gezeigt ist. Die Bank, die durch den Bildrekonstruktor jeweils abgefragt wird, ist durch das Signal bestimmt, das dem C/I-Speicher 122 auf der Leitung 712 vom Zwischenkreis 132 zugeführt wird.

Der Bewichtungsfunktionswert des Bewichtungsfunktions-Rekombinationskreises 690 des dritten Niveaus und die Lagedaten des C/I-Speichers 122 werden in einen Pipeline-Haltekreis 714 eingetaktet, wo die Daten den Eingängen eines $17 \times 16$-Multiplizier-Arrays 716 zugeführt werden. Dieses Multiplizier-Array und die darauffolgenden Wallace-Baum-Rekombinationsstufen sind ähnlich jenen, die oben beschrieben sind. Der Bewichtungsfunktionswert ist gleich $1/r^2$ und ist immer positiv. Deshalb ist das Zeichen des Produktes der Bewichtungsfunktion und der Lagedaten allein bestimmt durch das Zeichen der Lagedaten. Das Zeichen-bit der Lagedaten braucht daher nicht dem Multiplizier-Array 716 zugeführt zu werden. Die Ausgänge des Multiplizier-Arrays 716 werden einem Rekombinationskreis 718 eines ersten Niveaus zugeführt, welcher die ersten Stufen der Wallace-Baum-Rekombinationslogik beinhaltet. Die Ausgangssignale des Rekombinationskreises 718 werden in Pipeline-Verriegelungskreise eingetaktet, die durch gestrichelte Linien 720 gekennzeichnet sind, wo sie für die Verarbeitung durch die Stufe in dem Rekombinationskreis 722 des zweiten Niveaus in der nächsten Pipeline-Stufe gehalten werden.

Das Ausgangssignal des Rekombinationskreises 722 des zweiten Niveaus wird in den Haltekreis, der durch die Linie 724 gekennzeichnet ist, eingetaktet. Diese Daten repräsentieren die Größe des Wertes, der im Bildspeicher gespeichert werden muß. Die Zeichen-bits werden direkt vom C/I-Speicher über Haltekreise 714, 720 und 722 geliefert. Das ORI-bit von der ORI-Logik 656 wird ebenfalls dem Bildspeicher zugeführt; dieses bit wird durch die Verriegelungskreise zwischen jeder Stufe des Pipeline-Prozessors getaktet, so daß es durch die Pipeline-Stufen synchron mit den Daten, zu denen es gehört, wandert.

Bildspeicher

Der Bildspeicher und der zugeordnete Kreis sind in der Fig. 19 dargestellt. Die Daten des Multiplizier-Arrays 760 werden einem Eingang eines Addierkreises 740 zugeführt. Das Zeichen-bit der Daten des C/I-Speichers 122 wird dem Addier-Subtrahier-Eingang des Addierers 740 zugeführt. Der zweite Eingang des Addierers 740 empfängt Daten vom Bildspeicher 118. Wenn der Bildrekonstruktor durch die Plätze im Bildspeicher 118 geht, werden die vorher in jedem Platz des Bildspeichers akkumulierten Daten dem zweiten Eingang des Addierers 740 zugeführt. Zu diesen Daten werden die Daten der jeweils gegebenen Projektion addiert oder subtrahiert und die resultierende Summe des Addierers 740 ersetzt die vorhergehenden Daten, die in den jeweils aufgerufenen Plätzen des Bildspeichers 118 akkumuliert sind. Das ORI-bit der jeweiligen Projektion wird dem einen Eingang eines ODER-Gaters 742 zugeführt. Das ORI-bit, das im Bildspeicher 118 gespeichert ist, wird einem zweiten Eingang des ODER-Gatters 742 zugeführt und das Ausgangssignal des ODER-Gatters 742 wird wie das ORI-bit, das den neuen Daten des Addierers 740 zugeordnet ist, im Bildspeicher 118 gespeichert. Auf diese Weise wird dann, wenn der Bildrekonstruktor bestimmt, daß ein Platz im Bildspeicher außerhalb des rekonstruierten Bildes liegt, ein positiver ORI-bit, der diese Kondition bezeichnet, im Speicher 118 gespeichert und dieser positive ORI-bit wird bei jeder folgenden Aufdatierung der Bildspeicherdaten, die

24

in jenem Platz akkumuliert sind, in Abhängigkeit von der Operation des ODER-Gatters 742 aufrecht erhalten.

Die Adresseninformation für den Bildspeicher 118 wird von einem 16-bit-Speicheradressenzähler 744 erzeugt. Während einer Abtastung werden die Werte des Zählers 144 dem einen Eingang eines 16-Kanal-2-zu-1-Multiplexers 746 zugeführt, welcher die Eingangssignale den Adresseneingängen des Bildspeichers 118 zuführt. Nachdem eine Abtastung beendet ist, werden die Daten im Bildspeicher 118, die repräsentativ für das endgültige Bild sind, vom Computer 130 oder einer anderen entsprechenden Einrichtung gelesen, wobei ein Signal dem Wähleingang des Multiplexers 746 und Adressendaten den zweiten Eingängen des Multiplexers 746 zugeführt werden. Zum Lesen der gespeicherten Daten wird der Wähleingang umgeschaltet, so daß die Adresseninformation den Adresseneingängen des Bildspeichers 118 zugeführt wird und die Daten an den Datenausgängen dementsprechend erzeugt werden.

Der Adressenzähler 744 wird durch einen Bildspeichersteuerkreis 748 gesteuert. Für den Beginn der Bildrekonstruktion für eine Projektion führt der Computer-zwischenkreis 132 dem Steuerkreis 622 des vorher beschriebenen Koordinatentransformationskreises ein Signal zu. Der Steuerkreis 622 berechnet die Projektionskoordinaten der ursprünglichen Lage im Bildspeicher, wie dies oben in Verbindung mit Fig. 13 beschrieben ist. Nachdem dies getan ist, führt der Steuerkreis 622 einer Bildspeichersteuervorrichtung 748 ein Startsignal zu und fährt fort, in der beschriebenen Weise den Bildspeicher durchzuschalten. Entsprechend dem Startsignal des Koordinatentransformationskreises befähigt die Steuerschaltung 748 den Zähler 744, der dann mit dem 6,25 MHz-Taktsignal vom I.R.-Taktgeber 633 beaufschlagt wird, so daß der Zähler 744 dem Bildspeicher 118 die entsprechenden Adressendaten zuführt.

Wie oben beschrieben, enthält der Koordinatentransformationskreis nur einen Zwei-Leitungszähler 640. Das Ende des Bildrekonstruktionsprozesses wird durch einen Zähler 744 bestimmt. Wenn der Zähler 744 den letzten Platz im Bildspeicher 118 erreicht hat, führt er der Bildspeichersteuereinrichtung 748 ein dafür repräsentatives Signal zu. Entsprechend diesem Signal sperrt die Bildspeichersteuereinrichtung den Zähler 744 und sendet ein I.R.-Erledigt-Signal an den Zwischenkreis 132. Entsprechend diesem I.R.-Erledigt-Signal schaltet der Zwischenkreis das I.R.-Bereit-Signal auf ein niedriges Niveau zurück, das anzeigt, daß der Bildrekonstruktionsprozess für die vorliegende Projektion komplett ist.

Zwischenkreis

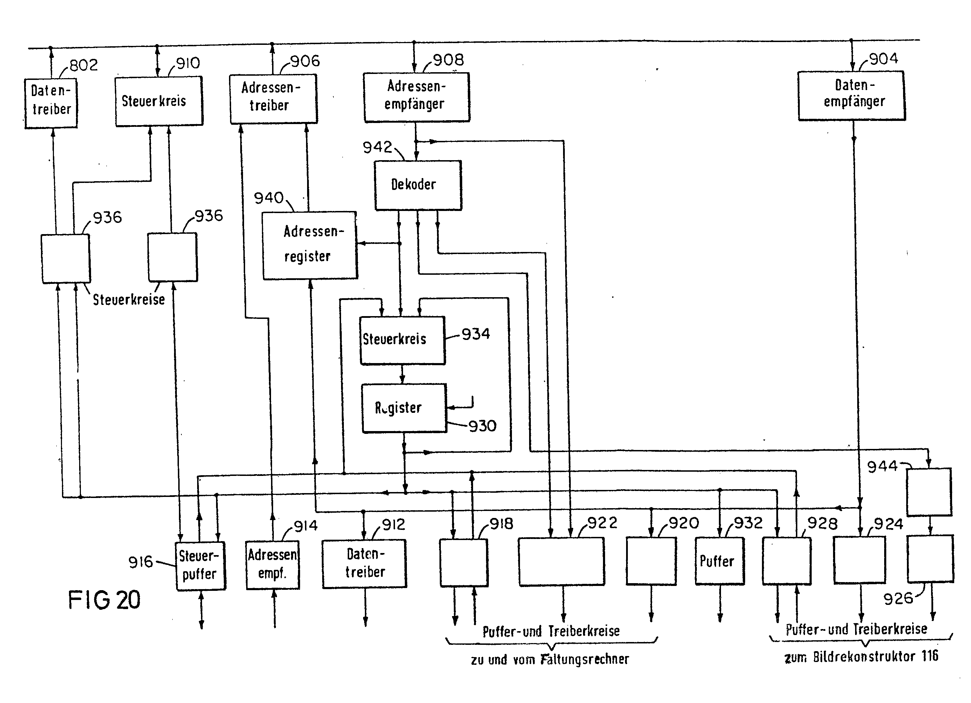

Die Fig. 20 zeigt ein Blockdiagramm des Zwischenkreises 132. Der hier vorgesehene Computer hat eine Zweirichtungsleitung für den I/O-Zwischenkreis.

In der Fig. 20 erfolgt die Kommunikation zu und vom Computer 130 über eine Computer-I/O-Leitung 900. Diese Leitung beinhaltet Zweirichtungs-Datenleitungen, Zweirichtungs-Adressenleitungen und verschiedene Kontroll-leitungen, die für eine ordnungsgemäße Operation des I/O-Kreises des Computers nötig sind. Mit der Leitung 900 sind Datentreiber 902 und Datenempfänger 904 zum Aussenden von Daten und zum Empfangen von Daten vom Computer, Adressentreiber 906 und Empfänger 908 zum Senden und Empfangen von Adressendaten vom Computer und ein Leitungssteuerkreis 910, welcher die verschiedenen Signale von der Leitung 900 empfängt und die einspeist, die für eine ordnungsgemäße Ubertragung zum Computer erforderlich sind, verbunden.

Der Zwischenkreis enthält separate Treiber und Empfänger für die Daten und andere notwendige Informationen, die übertragen werden zu und empfangen werden von dem Korrektor, Faltungsrechner und den Bildkonstruktor-abschnitten des Prozessors.

Daten werden zum Korrektor 112 über Datentreiber 912 übertragen. Die Adressendaten vom Korrektor, die die Adresse der erforderlichen Daten in dem Speicher des Computers 130 bezeichnen, werden dem Zwischenkreis 132 vom Korrektor über Adressenempfänger 914 zugeführt. Steuersignale zu und vom Korrektor gehen durch Steuerpuffer 916. Diese Signale sind im Detail in Verbindung mit der Fig. 6 und der Wirkungsweise des Korrektors näher erläutert.

Die Daten zu und vom Faltungsrechner 114 gehen durch Puffer- und Treiberkreise 918 bis 922. Das Faltungsrechner-Bereit-Signal zum Faltungsrechner und das Faltungsrechner-Erledigt-Signal vom Faltungsrechner geht durch Steuerpuffer 918. Die Kernal-Daten, die die die Bildschärfe erhöhende Funktion repräsentieren, werden vom Computer 130 in den Kernal-Speicher des Faltungsrechners vor dem Beginn einer tomographischen Abtastung geladen und diese Daten werden dem Kernal-Speicher durch Datentreiber 920 zugeführt. Die Adresseneingänge des Kernal-Speichers werden Adressentreibern 922 zugeführt. Der Wähleingang des Kernal-Speicher-Adressenmultiplexers wird über Adressen-Treiber 922 zugeführt, und zwar in Abhängigkeit von einem Signal vom Decoder 942.

Für jede neue Projektion muß der Computer dem Bildrekonstruktor eine neue Rekonstruktionskonstante zuführen. Diese Bildrekonstruktionskonstanten werden über Datentreiber 924 zugeführt. Dem Bildrekonstruktor wird angezeigt, welche Konstante jeweils angewandt wird, und zwar über Adressentreiber 926. Das I.R.-Breit-Signal zu und das I.R.-Erledigt-Signal vom Bildrekonstruktor geht durch Kontrollpuffer 928.

Ein Adressendecoder 942 empfängt Adressendaten von der Computerleitung 900 über Adressenempfänger 908. Entsprechend den jeweiligen Adressendaten bildet der Adressendecoder 942 ein Takt-

25

0 004 258

signal, um das Adressenregister 940 in einen Basiszustand zu bringen, das die Basisadresse der Korrekturdaten, die im Computer gespeichert sind, vom Computer 130 über Datenempfänger 902 lädt. Der Adressendecoder 942 führt ferner einem Bildrekonstruktor-Adressengenerator 944 Signale zu. Entsprechend der ordnungsgemäßen Adresseninformation zeigt der Adressengenerator 944 an, welche von verschiedenen Bildrekonstruktionskonstanten jeweils vom Computer geliefert werden und bildet die entsprechenden Signale, um die Adressentreiber 926 zu treiben.

Informationen, die den Zustand—arbeitend oder leer—der verschiedenen Abschnitte des Prozessors anzeigen, sind in einem Zustandsregister 930 enthalten. Diese Zustands-bits schließen folgendes ein. Ein Korrektor-Bereit-bit zeigt den Zustand des Korrektors an. Dieser bit wird dem Korrektor über Puffer 916 zugeführt und die Überführung dieses bits vom niedrigen in das hohe Niveau bewirkt, daß der Korrektor beginnt, Daten einer vollständigen Projektion zu verarbeiten. Ein Faltungsrechner-Bereit-bit zeigt den Zustand des Faltungsrechners an. Dieses bit wird dem Faltungsrechner über Puffer 918 zugeführt und die Überführung dieses bits vom niedrigen in das hohe Niveau bewirkt, daß der Faltungsrechner beginnt, die vorher korrigierten, im C/C-Speicher gespeicherten Daten zu verarbeiten. Ein I.R.-Bereit-Signal zeigt den Zustand des Bildrekonstruktors an. Dieses bit ist dem Bildrekonstruktor über Puffer 932 zugeführt und die Überführung vom niedrigen in das hohe Niveau bewirkt, daß der Bildrekonstruktor beginnt, die gefalteten Daten, die im C/I-Speicher 122 gespeichert sind, zu verarbeiten. Ein C/C-Speicher-Zustands-bit steuert den Zustand des Zweibank-C/I-Speichers und ein C/I-Zustands-bit steuert ebenfalls den Zustand des Zweibank-C/I-Speichers. Ein Bildrekonstruktor-Speicher-Zustands-bit steuert den Zustand des Zweibank-Speichers 624 im Bildrekonstruktor, der die Sinus- und Cosinusdaten enthält. Diese Speicher-Zustands-bits werden den zugeordneten Speichern über Puffer 932 zugeführt.

Der Zustand jedes der Zustands-bits im Zustandsregister 930 wird durch einen Zustandsregister-Steuerkreis 934 gesteuert. Die Eingangssignale des Zustandsregister-Steuerkreises 934 sind das Korrektur-Erledigt-Signal des Korrektors, das Faltungs-Erledigt-Signal des Faltungsrechners, das I.R.-Erledigt-Signal des Bildrekonstruktors, die Ausgangssignale der Statusregister und das Signal vom Decoder 942, welches bewirkt, daß die Basisadresse vom Computer 130 in das Basis-Adressenregister 940 geladen wird. Dementsprechend steuert eine kombinierte Logik 934 im Zustandsregister Signale, welche in das Zustandsregister 930 getaktet werden, um den Zustand der bits im Zustandsregister 930 in der folgenden Weise zu ändern. Entsprechend einem Korrektur-Erledigt-oder Faltungs-Erledigt-Signal von der Korrektur- oder Faltungsrechnerstufe wartet die Zustandsregister-Steuervorrichtung (wenn nötig) bis die folgende Stufe nicht bereit ist, was durch den entsprechenden Faltungsrechner-Bereit- oder I.R.-Bereit-bit vom Zustandsregister angezeigt wird und liefert dem Zustandsregister ein Signal, welches beim nächsten Taktpuls den Zustands-bit der Erledigt-Stufe auf nicht bereit setzt, den Zustands-bit der folgenden Stufe auf bereit setzt, um die Stufe zu starten und den Zustands-bit des C/C- oder C/I-Speichers zwischen den Einheiten ändert, damit der Zustand umgeschaltet wird. Entsprechend einem I.R.-Erledigt-Signal vom Bildrekonstruktor ändert die Zustandsregister-Steuervorrichtung den I.R.-Bereit-bit auf nicht bereit und den Zustands-bit des Bildrekonstruktor-speichers. Der Korrektor-Bereit-bit wird gesetzt, um den Korrektor entsprechend dem Laden einer Basisadresse in das Register 940 zu starten, wie dies durch ein Signal vom Adressendecoder 942 angezeigt wird.

Die Korrektor-Bereit- und I.R.-Bereit-Signale des Zustandsregisters 930 werden einem Unterbrechungssteuerkreis 936 zugeführt. Entsprechend den Änderungen in diesen Signalen vom hohen in den niedrigen Zustand liefert der Unterbrechungssteuerkreis 936 ein Signal, um den Steuerkreis 910 zu beeinflussen, den Computer 130 zu unterbrechen. Der Unterbrechungssteuerkreis 936 erzeugt ferner einen Unterbrechungsvektor, der anzeigt, ob der Korrektor oder Bildrekonstruktor die Datenverarbeitung beendet hat. Dieser Unterbrechungsvektor wird dem Computer über Datentreiber 902 zugeführt. Der Computer 130 muß, folgend auf jeden Bildrekonstruktionszyklus für die Daten von einer Projektion, neue Bildrekonstruktionskonstanten dem Bildrekonstruktor zuführen, wie dies oben beschrieben ist. In ähnlicher Weise muß der Computer 130 für jeden neuen Satz von Projektionsdaten, die vom Korrektor 112 verarbeitet werden, die Basisadresse des nächsten Datenblocks liefern. Wenn die Kernal-Daten in den Faltungsrechner 115 geladen worden sind, arbeitet der Faltungsrechner, ohne daß er weitere Daten vom Computer 130 braucht; deshalb braucht dem Computer 130 keine Unterbrechung vom Faltungsrechner 114 signalisiert zu werden.

Wie im Detail oben im Zusammenhang mit dem Korrektorkreis beschrieben ist, werden die Daten, die vom Korrektor verarbeitet werden, vom Computer über DMA gelesen. Durch den DMA-Steuerkreis 938 gehen Steuersignale zu und vom Faltungsrechner, die einen DMA-Zyklus anfordern und anzeigen, daß der Computerspeicher zur Verfügung steht. Diese Steuersignale bewirken, daß die entsprechenden Signale auf der Computerleitung 900 über die Leitungssteuereinrichtung 910 geliefert werden.

Die Adressendaten vom Korrektor 112, die die Daten, die während eines DMA-Zyklusses gelesen werden, anzeigen, werden auf der Computerleitung 900 über die Adressentreiber 906 geliefert. Ein Basisadressenregister 940 wird vom Computer geladen und enthält eine Basisadresse, die den Startpunkt im Computerspeicher des Datenblocks, der vom Korrektor anzufordern ist, anzeigt. Die bits unterer Ordnung der Adresse, die vom Computer geliefert wird, werden vom Korrektor berechnet und den Adressentreibern 906 über Korrektor-Adressenempfänger 914 zugeführt.

26

# 0 004 258

Patentansprüche

1. Computer-Tomograph zur Durchstrahlung eines Körpers (21) für die Erzeugung eines Querschnittsbildes aus Bilddaten, die repräsentativ für die Dichte des Körpers (21) in einer Querschnittsebene sind, mit einer Quelle (10) für die Erzeugung der den Körper (21) durchdringenden Strahlung (12), einem Detektor (14) zum Empfangen der Strahlung, die in der betreffenden Querschnittsebene durch den Körper (21) längs einer Vielzahl von Pfaden verläuft, und zur Bildung von für die empfangene Strahlungsintensität repräsentativen Ausgangssignalen, einer Einrichtung (16, 18) zur gemeinsamen Rotation der Strahlenquelle (10) und des Detektors (14) in bezug auf den Körper (21) und zur periodischen Aussendung der Strahlung (12) während der Rotation für die Abtastung unter einer Vielzahl von Projektionen mit einer entsprechenden Vielzahl von Projektionswinkeln, sowie einem Tomographieprozessor zur Bildung eines Bildes der durchstrahlten Schicht aus den Ausgangssignalen des Detektors (14), bestehend aus einem Korrektor (112) zur Korrektur der Ausgangssignale des Detektors (14) für die Bildung von korrigierten Daten, die repräsentativ für Linienintegrale der Dichte des Körpers (21) längs der von der Strahlung (12) durchsetzten Pfade sind, einem ersten Speicher (120) für die Speicherung der korrigierten Daten und zum Zwecke der Weiterverarbeitung der Daten zu einem späteren Zeitpunkt, einem Faltungsrechner (114) für die Faltung der korrigierten Daten mit einer die Bildschärfe erhöhenden Funktion, um eine Vielzahl von gefalteten Datenpunkten zu erzeugen, einem zweiten Speicher (122), der mit dem Faltungsrechner (114) verbunden ist, um die gefalteten Datenpunkte zu speichern für die Verarbeitung derselben zu einem späteren Zeitpunkt, einem Rekonstruktor (116), der mit dem zweiten Speicher (122) gekoppelt ist und dessen Daten so verarbeitet, daß die gefalteten Datenpunkte der Projektionen durch eine entsprechende Zuordnung zu ihren Plätzen im Querschnittsbild sowie durch Bewichtung mit einem vorgegebenen Bewichtungsfaktor mit nachfolgender Aufsummierung in einem Summationskreis (574, 740) rückprojiziert werden, sowie einem Bildspeicher (118) mit einer Vielzahl von Speicherplätzen, die mit einer entsprechenden Vielzahl von Bereichen des Körpers (21) in der entsprechenden Querschnittsebene übereinstimmt und in denen die rückprojizierten Datenpunkte abgespeichert werden, dadurch gekennzeichnet, daß der Korrektor (112), Faltungsrechner (114) und Rekonstruktor (116) sowie die als Zwischenspeicher ausgebildeten Speicher (118, 120, 122) des Tomographieprozessors mit einer Steuereinrichtung (130, 132) verbunden sind, welche die Prozessorstufen so steuert, daß jede Stufe die ankommenden Daten einer Projektion sofort verarbeitet und nach ihrer Verarbeitung der jeweils nachfolgenden Prozessorstufe zuführt, und daß der Summationskreis (574, 740) zwei Eingänge besitzt, von denen der erste Eingang mit dem zweiten Speicher (122) und der zweite Eingang mit dem Bildspeicher (118) in Verbindung steht, derart, daß der Summationskreis die vom zweiten Speicher angelieferten Datenpunkte einer Projektion mit den im Bildspeicher (118) bereits vorher abgespeicherten Datenpunkten aufsummiert und die auf diese Weise aufsummierten Datenpunkte den Dateneingängen des Bildspeichers zuführt, in welchem jeweils diese Datenpunkte die vorher abgespeicherten Datenpunkte ersetzen.

2. Tomograph nach Anspruch 1, dadurch gekennzeichnet, daß der erste und zweite Speicher (120, 122) jeweils ein Zweibankspeicher ist, der eine erste Speicherbank (302), eine zweite Speicherbank (304) und Mittel (310, 312) zur Speicherung von Daten in einer der beiden Speicherbänke (302, 304) jedes Speichers und zur Weiterverarbeitung von Daten von der jeweils anderen Speicherbank (302, 304) des betreffenden Speichers entsprechend einem Selektionssignal enthält, das von der Steuereinrichtung (132) geliefert wird und bestimmt, in welcher Bank Daten gespeichert werden und von welcher Bank Daten weiterverarbeitet werden, derart, daß jeweils korrigierte Datenpunkte einer Projektion in den einen Bänken der beiden Speicher abgespeichert und korrigierte Datenpunkte von einer früheren Projektion, die in den jeweils anderen Speicherbänken abgespeichert sind, gleichzeitig davon weiterverarbeitet werden.

3. Tomograph nach Anspruch 2, dadurch gekennzeichnet, daß der Rekonstruktor (116) Mittel (570) zum Multiplizieren der gefalteten Datenpunkte mit einem Bewichtungsfaktor vor der Speicherung der gefalteten Datenpunkte in dem entsprechenden Platz des Bildspeichers (118) aufweist.

4. Tomograph nach Anspruch 3, dadurch gekennzeichnet, daß der Korrektor (112) Mittel (Fig. 5) zur Durchführung von Rechenvorgängen für die Kompensation von Meßfehlern zwischen den Ausgangssignalen des Detektors und der aktuellen Intensität der davon empfangenen Strahlung und für die Kompensation der Strahlungsaufhärtung im Körper aufweist.

5. Tomograph nach Anspruch 3, dadurch gekennzeichnet, daß die Quelle (10) ein fächerförmiges Strahlenbündel (12), das durch den Körper (21) dringt, erzeugt, und daß der Detektor (14) eine Vielzahl von Einzeldetektoren aufweist, von denen jeder die Strahlungsintensität erfaßt und ein entsprechendes Ausgangssignal erzeugt, die längs eines Kreises angeordnet sind, dessen Mittelpunkt in dem Fokus liegt, wobei jeder Detektor unter einem Detektorwinkel in bezug auf eine Linie angeordnet ist, die durch die Quelle (10) und die Rotationsachse (20) verläuft.

6. Tomograph nach Anspruch 5, dadurch gekennzeichnet, daß der Korrektor (112) Mittel (262) zur Multiplikation der Ausgangssignale jedes der Einzeldetektoren mit dem Cosinus des Detektorwinkels zwischen dem betreffenden Einzeldetektor und dem zentralen Detektor längs des Kreises aufweist.

27

**0 004 258**

7. Tomograph nach Anspruch 5 oder 6, dadurch gekennzeichnet, daß der Bewichtungsfaktor, mit dem der Rekonstruktor (116) die gefalteten Datenpunkte multipliziert, gleich ist dem Reziprokwert des Quadrates des Abstandes der Quelle (10) vom Bereich des Körpers (21), der dem Platz im Bildspeicher (118) entspricht, in dem die bewichteten und gefalteten Datenpunkte gespeichert werden.

8. Tomograph nach Anspruch 1, dadurch gekennzeichnet, daß die Steuereinrichtung (130) einen programmierbaren Digitalprozessor aufweist.

9. Tomograph nach Anspruch 8, dadurch gekennzeichnet, daß die Steuereinrichtung (130) folgende Baugruppen enthält: Korrektor-Aktiviermittel (132, 272), die entsprechend einem Korrektor-Aktiviersignal arbeiten, um den Korrektor (112) zu veranlassen, mit der Korrektur der Ausgangssignale des Detektors (14) für eine Projektion zu beginnen und mit der Korrektur der Ausgangssignale des Detektors (14) aufzuhören, wenn alle Ausgangssignale einer Projektion korrigiert sind, Faltungsrechner-Aktiviermittel, die entsprechend einem Faltungsrechner-Aktiviersignal arbeiten, um zu bewirken, daß der Faltungsrechner (114) mit der Faltung korrigierter Datenpunkte einer Projektion beginnt und mit der Faltung korrigierter Datenpunkte aufhört, wenn alle korrigierten Datenpunkte einer Projektion gefaltet sind, und Rekonstruktor-Aktiviermittel (132), die entsprechend einem Rekonstruktor-Aktiviersignal arbeiten, um den Rekonstruktor (116) in dem Sinne zu beeinflussen, daß eine Rückprojektion der gefalteten Datenpunkte einer Projektion beginnt und daß eine Rückprojektion der gefalteten Datenpunkte einer Projektion beendet wird, wenn alle gefalteten Datenpunkte einer Projektion rückprojiziert sind, wobei die Korrektor-, Faltungsrechner- und Rekonstruktor-Aktiviersignale von der Steuereinrichtung (30) erzeugt werden.

10. Tomograph nach Anspruch 1, dadurch gekennzeichnet, daß er einen Interpolator (430) enthält, der auf die gefalteten Datenpunkte des Faltungsrechners (Fig. 6, 7) anspricht, um interpolierte Datenpunkte für eine Projektion zu erzeugen, die jeweils zwischen Paaren von gefalteten Datenpunkten liegen und die gebildet werden, indem eine lineare Interpolation zwischen dem betreffenden Paar von gefalteten Datenpunkten erfolgt, und daß der zweite Speicher (122) Mittel zur Speicherung der interpolierten Datenpunkte einer Projektion und zur Weiterverarbeitung dieser gespeicherten, interpolierten Datenpunkte zu einem späteren Zeitpunkt aufweist, und wobei der Rekonstruktor (116) sowohl die gefalteten Datenpunkte als auch die interpolierten Datenpunkte einer Projektion rückprojiziert, indem er den entsprechenden der gefalteten Datenpunkte und der interpolierten Daten-punkte für jeden Platz im Bildspeicher (118) bestimmt und diesen zu dem Wert, der in diesem Platz im Bildspeicher vorher gespeichert ist, aufaddiert.

11. Tomograph nach den Ansprüchen 5 bis 7, dadurch gekennzeichnet, daß der Rekonstruktor (116) enthält: Adressenmittel (550) zur sequentiellen Erzeugung von Adressendaten, um die Plätze im Bildspeicher (122) während jeder Projektion abzufragen, Lagemittel (556), die in Abhängigkeit von Daten, die von den Adressendaten abgeleitet sind, arbeiten, um zu bestimmen, welche der gefalteten Datenpunkte einer Projektion mit den jeweils abgefragten Bildspeicherplätzen korrespondieren, und Mittel (570) zur Multiplikation der entsprechenden Datenpunkte, die durch die Lagemittel (556) bestimmt worden sind, mit dem Bewichtungsfaktor, um die bewichteten Datenpunkte zu erzeugen.

12. Tomograph nach Anspruch 11, dadurch gekennzeichnet, daß das fächerförmige Strahlenbündel (12) aus Röntgenstrahlung besteht.

13. Tomograph nach Anspruch 11, dadurch gekennzeichnet, daß zur Umwandlung der im Patientenkoordinatensystem vorliegenden Bildspeicheradressendaten in das Projektionskoordinatensystem, dessen Koordinaten durch das Projektionssystem aus Strahlenquelle (10) und Detektor (14) festgelegt sind, der Rekonstruktor (116) Transformationsmittel (550) aufweist, welche die Adressendaten in für die Projektionskoordinaten des Körperbereiches repräsentative Daten umwandelt.

14. Tomograph nach Anspruch 13, dadurch gekennzeichnet, daß die Transformationsmittel (550) eine Einrichtung (552) zur Erzeugung von für jeden Winkel ($\theta$) zwischen einer Koordinate des Projektionskoordinatensystems und der entsprechenden Koordinate des Patientenkoordinatensystems repräsentativen Projektionswinkeldaten aufweist, welche die Projektionskoordinaten des ersten Speicherplatzes (508) im Bildspeicher (118) für den betreffenden Projektionswinkel einmal bestimmt (Fig. 12 und 13) und der eine Einrichtung (554) zugeordnet ist, die bei schrittweiser Durchschaltung des Prozessors durch die Speicherplätze des Bildspeichers (118) zu den bereits bestimmten Projektionsdaten des ersten Bildspeicherplatzes der betreffenden Projektion im Schritttakt des Prozessors nacheinander gleiche Inkrementwerte hinzuaddiert, deren Größe für eine Projektion jeweils gleich ist, aber vom Projektionswinkel abhängt, so daß auf diese Weise die Projektionskoordinaten aller anderen Bildspeicherplätze dieser Projektion bestimmbar sind.

15. Tomograph nach Anspruch 11, dadurch gekennzeichnet, daß wenigstens ein logarithmischer Analog-Digitalwandler (106) zur Bildung von den integrierten Detektorausgangssignalen entsprechenden digitalen Daten vorgesehen ist.

16. Tomograph nach Anspruch 11, dadurch gekennzeichnet, daß dem Detektor (14) Integratoren (202) vom selbst-nullabgleichenden Typ nachgeschaltet sind, um Drift-Fehler zu reduzieren.

17. Tomograph nach Anspruch 16, gekennzeichnet durch Mittel (108) zur Umschaltung jedes Integrators (202) auf Selbst-Nullabgleich vor der Emission jedes Strahlungsimpulses durch die Strahlen-quelle (10).

28

18. Tomograph nach Anspruch 15, gekennzeichnet durch einen Multiplexer (104) zum Multiplexen der integrierten Detektorausgangssignale, wobei die integrierten Detektorausgangssignale seriell mindestens einem logarithmischen Konverter (106) für die Umwandlung in digitaler Form zugeführt werden.

19. Tomograph nach Anspruch 18, dadurch gekennzeichnet, daß der Multiplexer (104) die integrierten Detektorausgangssignale dem Konverter (106) in einer Folge zuführt, die der Ordnung entspricht, in der die Detektoren in dem linearen Array (14) angeordnet sind, beginnend mit Detektoren, die im Zentrum des Arrays (14) liegen, und fortschreitend bis zum Ende des Arrays (14).

20. Tomograph nach Anspruch 11, dadurch gekennzeichnet, daß der Korrektor (112) direkt mit dem ersten Speicher (120) und der erste Speicher (120) direkt mit dem Faltungsrechner (114) gekoppelt ist.

21. Tomograph nach den Ansprüchen 3 und 13, dadurch gekennzeichnet, daß an die Multiplikationsmittel (570) Mittel (558, 562) zur Speicherung und Zuführung des von den Transformationsmitteln (550) gebildeten Bewichtungsfaktors angeschlossen sind.

22. Tomograph nach Anspruch 1, dadurch gekennzeichnet, daß die Körperbereiche, die den Bildspeicherplätzen zugeordnet sind, längs orthogonaler Reihen und Spalten angeordnet sind, so daß das Bildspeicher-Koordinatensystem ein zweidimensional kartesisches Koordinatensystem ist.

23. Tomograph nach Anspruch 14, dadurch gekennzeichnet, daß die Einrichtung (552) als Projektionswinkeldaten Daten erzeugt, die repräsentativ für $\delta$ sin $\theta$- und $\delta$ cos $\theta$-Werte sind, und zu diesem Zweck einen programmierbaren Digitalprozessor (Fig. 14) enthält.

24. Tomograph nach Anspruch 23, dadurch gekennzeichnet, daß der Digitalprozessor (Fig. 14) während einer Projektion die $\delta$ sin $\theta$- und $\delta$ cos $\theta$-Werte der vorhergehenden Projektion liefert.

25. Tomograph nach Anspruch 11, dadurch gekennzeichnet, daß die Lagemittel ein ROM (556) aufweisen, in dem Daten gespeichert sind, die repräsentativ für die Lagefunktion sind, welche jeden Platz im Bildspeicher (118) einem der Strahlenpfade für jeden Projektionswinkel zuordnet.

26. Tomograph nach Anspruch 11, dadurch gekennzeichnet, daß an den Mitteln (570) zum Multiplizieren Bewichtungsfaktormittel (558, 562) angeschlossen sind, die die Bewichtungsfaktoren in Abhängigkeit von Daten der Transformationsmittel (550) bilden.

27. Tomograph nach Anspruch 26, dadurch gekennzeichnet, daß die Bewichtungsfaktormittel (558, 562) ein ROM (558) beinhalten, dem die Projektionskoordinaten-Daten von den Transformationsmitteln (550) zugeführt werden und das in Abhängigkeit davon an seinem Ausgang Daten liefert, die repräsentativ für den Bewichtungsfaktor sind.

**Claims**

1. A computer tomograph for the irradiation of a body (21) to produce a cross-sectional image composed of elements of image data representative of the density of the body (21) in a cross-sectional plane; with a source (10) for the generation of radiation (12) which penetrates the body (21); with a detector (14) for the reception of the radiation which passes through the body (21) along a plurality of paths in the cross-sectional plane in question and the formation of output signals representative of the received radiation intensity; with a device (16, 18) for the common rotation of the radiation source (10) and of the detector (14) relative to the body (21) and for the periodic transmission of radiation (12) during the rotation for scanning at a plurality of projections with a corresponding plurality of projection angles; and with a tomography processor for the formation of an image of the irradiated layer from the output signals of the detector (14), composed of a corrector (112) for the correction of the output signals of the detector (14) to form corrected data elements which are representative of line integrals of the density of the body (21) along the paths travelled by the radiation (12); with a first store (120) for the storage of the corrected data elements and for the further processing of the data at a later point in time; with a convolution computer (114) for the convolution of the corrected data elements with a function which increases the image definition to produce a plurality of convoluted data points; with a second store (122) connected to the convolution computer (114) to store the convoluted data points for the processing thereof at a later point in time; with a reconstructor (116) coupled to the second store (122) to process the data thereof in such manner that the convoluted data points of the projections are back-projected by an appropriate assignment to their positions in the cross-sectional diagram and by weighting with a predetermined weighting factor followed by addition in a summation circuit (574, 740); and with an image store (118) having a plurality of store positions which conforms with a corresponding plurality of zones of the body (21) in the corresponding cross-sectional plane and in which the back-projected data points are stored; characterised in that the corrector (112), the convolution computer (114) and the reconstructor (116), together with the stores (118, 120, 122) of the tomography processor, which are designed as an intermediate store, are connected to a control device (130, 132) which controls the processor stages in such manner that each stage immediately processes the incoming data of a projection and then supplies this data to the following processor stage, and that the summation circuit (574, 740) possesses two inputs, the first of which is connected to the second store (122) and the second of which is connected to the image store (118) in such manner that the summation circuit adds the data points of a projection which have been supplied by the second store to the data points already stored in the image store (118) and conducts the data points which have been added in this way to the data inputs of the image store in which these data points replace the previously stored data points.

2. A tomograph as claimed in claim 1, characterised in that the first and second stores (120, 122) are each in the form of a two-bank store having a first storage bank (302), a second storage bank (304), and means (310, 312) for storing data in one of the two storage banks (302, 304) of each store and for further processing data from the particular other storage bank (302, 304) of the store in question in accordance with a selection signal which is supplied by the control device (132), and which determines the bank in which any data is to be stored and by which bank the data is further processed in such manner that corrected data points of a projection are stored in one of the banks of the two stores and corrected data points from an earlier projection which are stored in the particular other storage banks are simultaneously further processed.

3. A tomograph as claimed in claim 2, characterised in that the reconstructor (116) possesses means (570) for multiplying the convoluted data points with a weighting factor prior to the storage of the convoluted data points in the appropriate position of the image store (118).

4. A tomograph as claimed in claim 3, characterised in that the corrector (112) possesses means (Fig. 5) for carrying out calculating processes for the compensation of measuring errors between the output signals of the detector and the current intensity of the radiation received, and for compensation of radiation hardening in the body.

5. A tomograph as claimed in claim 3, characterised in that the source (10) produces a fan-like group of beams (12) which penetrates the body (21), and that the detector (14) possesses a plurality of individual detectors, each of which detects radiation intensity and produces a corresponding output signal, these being arranged along a circular path whose centre lies in the focal point, each detector being arranged at a detector angle relative to a line which passes through the source (10) and the axis of rotation (20).

6. A tomograph as claimed in claim 5, characterised in that the corrector (112) possesses means (262) for multiplying the output signals of each of the individual detectors by the cosine of the detector angle between that individual detector and that detector central along the circular path.

7. A tomograph as claimed in claim 5 or claim 6, characterised in that the weighting factor with which the reconstructor (116) multiplies the convoluted data points is equal to the reciprocal value of the square of the distance of the source (10) from that zone of the body (21) which corresponds to the position in the image store (118) in which the weighted and convoluted data points are stored.

8. A tomograph as claimed in claim 1, characterised in that the control device (130) comprises a programmable digital processor.

9. A tomograph as claimed in claim 8, characterised in that the control device (130) contains the following assemblies:— a corrector-activating means (132, 272) which operate in accordance with a corrector-activator signal to cause the corrector (112) to commence correction of the output signals of the detector (14) for a projection, and to stop the correction of the output signals of the detector (14) when all the output signals of a projection have been corrected; convolution computer activating means which operate in accordance with a convolution computer activating signal to cause the convolution computer (114) to commence the convolution of corrected data points of a projection, and to discontinue convolution of corrected data points when all the corrected data points of a projection have been convoluted; and reconstructor activating means (132) which operate in accordance with a reconstructor activating signal to influence the reconstructor (116) in that a back projection of the convoluted data points of a projection commences and a back projection of the convoluted data points of a projection is terminated when all the convoluted data points of a projection have been back projected, the activating signals for the corrector, the convolution computer, and the reconstructor being produced by the control device (30).

10. A tomograph as claimed in claim 1, characterised in that it contains an interpolator (430) which responds to the convoluted data points of the convolution computer (Fig. 6, 7) to produce interpolated data points for a projection, each of which lie between pairs of convoluted data points, and which are formed in that a linear interpolation is effected between the relevant pair of convoluted data points, and that the second store (122) comprises means for storing the interpolated data points of a projection, and for further processing these stored, interpolated data points at a later point in time, and where the reconstructor (116) back-projects both the convoluted data points and the interpolated data points of a projection in that it determines the appropriate member of the convoluted data points and of the interpolated data points for each position in the image store (118) and adds this to the value which was previously stored in this position in the image store.

11. A tomograph as claimed in claims 5 to 7, characterised in that the reconstructor (116) contains an address means (550) for the sequential generation of address data to interrogate positions in the image store (122) during each projection, position means (556) which operate in dependence upon items of data derived from the address data to determine which of the convoluted data points of a projection correspond to the particular interrogated image store positions, and means (570) for multiplying the corresponding data points which have been determined by the position means (556) with the weighting factor to produce the weighted data points.

30

**0 004 258**

12. A tomograph as claimed in claim 11, characterised in that the fan-like group of beams (12) consists of X-rays.

13. A tomograph as claimed in claim 11, characterised in that conversion of the image store addresses contained in the patient co-ordinate system into the projection co-ordinate system whose co-ordinates are determined by the projection system composed of radiation source (10) and detector (14) is effected by the reconstructor (116), which comprises transformation means (550) to convert the address data into data representative of the projection co-ordinates of the region of the body.

14. A tomograph as claimed in claim 13, characterised in that the transformation means (550) comprise a device (552) for the generation of items of projection angle data which are representative of each angle ($\theta$) between a co-ordinate of the projection co-ordinate system and the corresponding co-ordinate of the patient co-ordinate system, which device determines the projection co-ordinates of the first store position (508) in the image store (118) for the relevant projection angle (Fig. 12 and 13) and which is assigned a device (554) which adds to the already determined projection data of the first image store position of the relevant projection, consecutively and in the element timing of the processor, identical increment values whose quantity is always equal for a projection but is dependent upon the projection angle as the processor is switched through step by step through the store positions of the image store (118) so that it is possible to determine the projection co-ordinates of all the other image store positions of this projection.

15. A tomograph as claimed in claim 11, characterised in that at least one logarithmic analogue-digital converter (106) is provided for the formation of items of digital data corresponding to the integrated detector output signals.

16. A tomograph as claimed in claim 11, characterised in that the detector (14) is followed by integrators (202) of the self-zero compensating type in order to reduce drift errors.

17. A tomograph as claimed in claim 16, characterised by means (108) for switching over each integrator (202) to self-zero compensation prior to the emission of each radiation pulse by the radiation source (10).

18. A tomograph as claimed in claim 15, characterised by a multiplexer (104) which serves to multiplex the integrated detector output signals, the integrated detector output signals being conducted in serial fashion to at least one logarithmic converter (106) for conversion into digital form.

19. A tomograph as claimed in claim 18, characterised in that the multiplexer (104) conducts the integrated detector output signals to the converter (106) in a sequence which corresponds to the order in which the detectors are arranged in the linear array (14) commencing with detectors which are located in the centre of the array (14) and continuing to the end of the array (14).

20. A tomograph as claimed in claim 11, characterised in that the corrector (112) is directly coupled to the first store (120), and the first store (120) is directly coupled to the convolution computer (114).

21. A tomograph as claimed in claims 3 and 13, characterised in that the multiplication means (570) are connected to means (558, 562) for the storage and supply of the weighting factor formed by the transformation means (550).

22. A tomograph as claimed in claim 1, characterised in that those regions of the body which are assigned to the image store positions are arranged along orthogonal rows and columns so that the image store co-ordinate system is a two-dimensional Cartesian co-ordinate system.

23. A tomograph as claimed in claim 14, characterised in that for projection angle data the device (552) produces items of data representative of $\delta \sin \theta$, and $\delta \cos \theta$ values, using a programmable digital processor (Fig. 14).

24. A tomograph as claimed in claim 23, characterised in that the digital processor (Fig. 14) supplies the $\delta \sin \theta$ and $\delta \cos \theta$ values of the previous projection during a projection.

25. A tomograph as claimed in claim 11, characterised in that the position means comprise a ROM (556) which stores items of data which are representative of the position function which assigns each position in the image store (118) to one of the beam paths for each projection angle.

26. A tomograph as claimed in claim 11, characterised in that the multiplication means (570) are connected to weighting factor means (558, 562) which form the weighting factors in dependence upon data from the transformation means (550).

27. A tomograph as claimed in claim 26, characterised in that the weighting factor means (558, 562) contain a ROM (558) which is supplied with the projection co-ordinate data from the transformation means (550) and which in dependence thereupon supplies from its output items of data which are representative of the weighting factor.

**Revendications**

1. Tomographe avec ordinateur pour irradier un corps (21) en vue de l'établissement d'images transversales à partir de données d'image qui sont représentatives de la densité du corps (21) dans un plan transversal, du type comportant une source (10) pour produire un rayonnement traversant le corps (21), un détecteur (14) pour recevoir le rayonnement qui passe dans ladite section transversale à travers le corps, le long d'un grand nombre de voies, et pour former des signaux de sortie représentatifs

**0 004 258**

de l'intensité du rayonnement reçu, un dispositif (16, 18) assurant la rotation simultanée de la source de rayonnement (10) et du détecteur (14) par rapport au corps (21) et l'émission périodique du rayonnement (12) pendant la rotation pour le balayage sous un grand nombre de projections avec un grand nombre correspondant d'angles de projection, ainsi qu'un processeur de tomographie pour former une image de la couche irradiée à partir des signaux de sortie du détecteur (14), constitué par un correcteur (112) pour corriger les signaux de sortie du détecteur (14) pour la formation de données corrigées qui sont représentatives pour l'intégrale linéaire de la densité du corps (21) le long des voies traversées par le rayonnement (12), une première mémoire (120) pour mémoriser les données corrigées et pour le traitement à un instant ultérieur, une calculatrice de convolution (114) pour obtenir la transformation de convolution des données corrigées avec une fonction de la netteté de l'image afin de produire un grand nombre de points de données de convolution, une seconde mémoire (122) reliée à la calculatrice d'intégrale de convolution (114), en vue de mémoriser les points de données obtenus par convolution pour le traitement à un instant ultérieur, un reconstituteur (116) qui est accouplé à la seconde mémoire (122) et dont les données sont traitées de telle manière que les points de données des projections, obtenus par convolution sont reprojetés, par une coordination correspondante avec leurs emplacements dans l'image transversale et par une pondération avec un facteur de pondération donné à l'avance ainsi que par une sommation subséquente dans un circuit de sommations (574, 740), ainsi qu'un tube image à mémoire (118) à grands nombres d'emplacements de mémoires qui concorde à un grand nombre correspondant de plages du corps (21) dans le plan transversal correspondant et dans lesquels sont mémorisés les points de données reprojetés, caractérisé par le fait que le correcteur (112), la calculatrice de convolution (114) et le reconstituteur (116) ainsi que les mémoires (118, 120, 122) du processeur de tomographie et qui sont constituées comme des mémoires intermédiaires, sont reliés à un dispositif de commande (130, 132) qui commande de telle manière les étages du processeur que chaque étage traite immédiatement les données d'entrée d'une projection et les transfère, après traitement, à l'étage suivant du processeur et que le circuit de sommation (574, 740) possède deux entrées dont la première est reliée à la seconde mémoire et la seconde entrée est reliée au tube image à mémoire ou mémoire d'image (118) de manière que le circuit de sommation additionne les points de données d'une projection qui sont fournis par la seconde mémoire et les points de données déjà précédemment mémorisés dans la mémoire d'image (118) et applique les points de données ainsi additionnés aux entrées de données de la mémoire d'image dans laquelle ces points de données remplacent les points d'image précédemment mémorisés.

2. Tomographe selon la revendication 1, caractérisé par le fait que la première et la seconde mémoires (120, 122) sont constituées chacune par une mémoire à deux blocs comportant un premier bloc de mémoire (302), un second bloc de mémoire (304) et des moyens (310, 312) pour mémoriser des données dans l'un des deux blocs de mémoires (302, 304) de chaque mémoire et pour le traitement ultérieur de données provenant de l'autre bloc de mémoire (302, 304) de la mémoire correspondante en fonction d'un signal de sélection qui est fourni par le dispositif de commande (132) et détermine dans quel bloc des données doivent être mémorisées et par quel bloc la poursuite du traitement de données doit être assurée, la réalisation étant telle que des points de données corrigés d'une projection sont mémorisés dans l'un des deux blocs des deux mémoires, alors que des points de données corrigés d'une projection antérieure et qui sont mémorisés dans l'autre des deux blocs de mémoire, sont en même temps soumis au traitement ultérieur.

3. Tomographe selon la revendication 2, caractérisé par le fait que le reconstituteur ou reconstructeur (116) comporte des moyens (570) pour multiplier les points de données ayant subi la transformation de convolution, avec un facteur de pondération, avant la mémorisation, dans l'emplacement correspondant du tube image à mémoire (118), des points de données qui ont subi la transformation de convolution.

4. Tomographe selon la revendication 3, caractérisé par le fait que le correcteur (112) comporte des moyens (figure 5) pour réaliser des opérations de calcul en vue de la compensation des erreurs de mesure entre les signaux de sortie du détecteur et l'intensité actuelle ou réelle du rayonnement reçu par celui-ci, et en vue de compenser le durcissement du rayonnement dans le corps.

5. Tomographe selon la revendication 3, caractérisé par le fait que la source (10) produit un faisceau de rayons (12) en forme d'un éventail, traversant le corps (21), et que le détecteur (14) comporte un grand nombre de détecteurs individuels dont chacun saisit l'intensité du rayonnement et fournit un signal de sortie correspondant, lesdits détecteurs individuels étant disposés le long d'un cercle dont le centre se situe au foyer, chaque détecteur étant disposé sous un angle de détecteur par rapport à une ligne qui passe par la source (10) et par l'axe de rotation (20).

6. Tomographe selon la revendication 5, caractérisé par le fait que le correcteur (112) comporte des moyens (262) pour multiplier les signaux de sortie de chacun des détecteurs individuels avec le cosinus de l'angle de détection entre le détecteur individuel concerné et le détecteur central, le long du cercle.

7. Tomographe selon la revendication 5 ou 6, caractérisé par le fait que le facteur de pondération avec lequel le reconstituteur (116) multiple les points de données ayant subi la convolution, est égal à la valeur inverse du carré de la distance de la source (10) de la partie du corps (21) qui correspond à

**0 004 258**

l'emplacement dans le tube image à mémoire (118) dans lequel sont mémorisés les points de données pondérés et ayant subi la transformation de convolution.

8. Tomographe selon la revendication 1, caractérisé par le fait que le dispositif de commande (130) comporte un processeur numérique programmable.

9. Tomographe selon la revendication 8, caractérisé par le fait que le dispositif de commande (130) comporte les sous-ensembles suivants: des moyens (132, 272) pour activer le correcteur, opérant en fonction d'un signal d'activation du correcteur pour faire en sorte que le correcteur (112) commence à opérer avec la correction des signaux de sortie du détecteur (14) pour une projection et cesse d'opérer avec la correction des signaux de sortie du détecteur lorsque tous les signaux de sortie d'une projection sont corrigés, des moyens pour activer la calculatrice de convolution, et qui opèrent, en fonction d'un signal d'activation de la calculatrice de convolution pour provoquer que la calculatrice de convolution (114) commence avec la convolution de points de données corrigés d'une projection et cesse la convolution de points de données corrigés lorsque tous les points de données corrigés d'une projection ont subi la convolution, des moyens (132) pour activer le reconstituteur, et qui opèrent avec un signal d'activation du reconstituteur pour influencer le reconstituteur (116) dans ce sens qu'une reprojection des points de données d'une projection, qui ont subi la convolution, commence et qu'une reprojection des points de données d'une projection et qui ont subi la convolution, cesse lorsque tous les points de données d'une projection, qui ont subi la convolution, sont reprojetés, les signaux d'activation pour le correcteur, pour la calculatrice de convolution et pour le reconstituteur étant produits par le dispositif de commande (30).

10. Tomographe selon la revendication 1, caractérisé par le fait qu'il comporte un interpolateur (430) qui est sensible aux points de données de la calculatrice de convolution (figures 6, 7) qui ont subi la convolution, en vue de produire des points de données interpolés pour une projection, qui se situent entre des paires de points de données ayant subi la convolution et qui sont formés par une interpolation linéaire entre la paire considérée de points de données ayant subi la convolution, et que la seconde mémoire (122) comporte des moyens pour mémoriser les points de données interpolés d'une projection et pour le traitement ultérieur, à un instant ultérieur de ces points de données mémorisés et interpolés, le reconstituteur (116) reprojetant aussi bien des points de données ayant subi la convolution que les points de données interpolés d'une projection en déterminant pour chaque emplacement dans le tube image à mémoire (108) le point de donnée correspondant parmi les points de données qui ont subi la convolution et le point de donnée correspondant parmi les points de données interpolés, et en ajoutant à celui-ci à la valeur qui a été précédemment mémorisée dans cet emplacement du tube image à mémoire.

11. Tomographe selon les revendications 5 à 7, caractérisé par le fait que le reconstituteur (116) comporte: des moyens d'adresses (550) pour produire de façon séquentielle des données d'adresses pour interroger les emplacements du tube image à mémoire (122) pendant chaque projection, des moyens de position (556) qui opèrent en fonction de données qui sont dérivées des données d'adresses, en vue de déterminer ceux des points de données d'une projection, qui ont subi la transformation de convolution, qui correspondent aux emplacements du tube image à mémoire qui ont été interrogés, et des moyens (570) pour multiplier les points de données correspondants et qui ont été déterminés par les moyens de position (556), avec le facteur de pondération, en vue de fournir les points de données pondérés.

12. Tomographe selon la revendication 11, caractérisé par le fait que le faisceau de rayons en forme d'éventail (12) est constitué par un rayonnement X.

13. Tomographe selon la revendication 11, caractérisé par le fait que pour transformer les données d'adresses de la mémoire d'image présentes dans le système de coordonnées du patient, en le système de coordonnées de projection dont les coordonnées sont déterminées par le système de projection constitué par la source de rayonnement (10) et le détecteur (14), le reconstituteur (116) comportant des moyens de transformation (550) qui transforment les données d'adresses en des données représentatives des coordonnées de projection de la partie du corps concernée.

14. Tomographe selon la revendication 13, caractérisé par le fait que les moyens de transformation (555) comportent un dispositif (552) pour fournir des données d'angles de projection représentatives pour chaque angle ($\theta$) entre une coordonnée du système de coordonnées de projection et des coordonnées correspondantes du système de coordonnées du patient, et qui détermine les coordonnées de projection du premier emplacement de mémoire (508) dans le tube image à mémoire (118) pour l'angle de projection concerné (figures 12 et 13), dispositif auquel est associé un dispositif (554) qui, lors de la commutation pas-à-pas du processeur à travers les emplacements de mémoire du tube image à mémoire (18), additionne aux données des projections, déjà déterminées, du premier emplacement du tube image à mémoire de la projection concernée, des valeurs d'incréments identiques, cette addition se faisant à la cadence du processeur, et les valeurs d'incréments étant identiques pour une même projection mais dépendent de l'angle de projection en sorte que les coordonnées de projection de tous les autres emplacements du tube image à mémoire de cette projection puissent être déterminés de cette manière.

15. Tomographe selon la revendication 11, caractérisé par le fait qu'il est prévu au moins un

33

convertisseur analogique/numérique (106), opérant par voie logarithmique, pour former à partir des signaux de sortie intégrés du détecteur des données numériques correspondantes.

16. Tomographe selon la revendication 11, caractérisé par le fait qu'en aval du détecteur (14) sont prévus des intégrateurs (202) du type à compensation automatique du zéro, pour réduire les erreurs de dérive.

17. Tomographe selon la revendication 16, caractérisé par des moyens (108) pour commuter chaque intégrateur (202) pour la compensation automatique du zéro, avant l'émission de chaque impulsion de rayonnement par la source de rayonnement (10).

18. Tomographe selon la revendication 15, caractérisé par un multiplexeur (104) pour multiplexer les signaux de sortie intégrés des détecteurs, les signaux de sortie intégrés des détecteurs étant appliqués sériellement au moins à un convertisseur logarithmique (106) pour la transformation sous forme numérique.

19. Tomographe selon la revendication 18, caractérisé par le fait que le multiplexeur (104) applique les signaux de sortie intégrés des détecteurs au convertisseur (106) suivant une succession qui correspond à l'ordre dans lequel sont disposés les détecteurs dans la rangée linéaire (14), en commençant par les détecteurs qui se situent au centre de la rangée (14) et se poursuivant jusqu'à l'extrémité de la rangée (14).

20. Tomographe selon la revendication 11, caractérisé par le fait que le correcteur (112) est relié directement à la première mémoire (120) alors que la première mémoire (120) est reliée directement à la calculatrice de convolution (114).

21. Tomographe selon les revendications 3 et 13, caractérisé par le fait qu'au moyen de multiplications (570) sont reliés des moyens (558, 562) pour la mémorisation et l'application du facteur de pondération qui est formé par les moyens de transformation (555).

22. Tomographe selon la revendication 1, caractérisé par le fait que les parties du corps qui sont associées aux emplacements du tube image à mémoire, sont disposées le long de rangées et de colonnes orthogonales, en sorte que le système de coordonnées du tube image à mémoire est un système de coordonnées cartésien bidimensionnel.

23. Tomographe selon la revendication 14, caractérisé par le fait que le dispositif (552) produit, au titre de données d'angles de projection, des données qui sont représentatives pour les valeurs $\delta \sin \theta$ et $\delta \cos \theta$, et qu'il comporte à cet effet un processeur numérique programmable (figure 14).

24. Tomographe selon la revendication 23, caractérisé par le fait que le processeur numérique (14) fournit pendant une projection les valeurs $\delta \sin \theta$ et $\delta \cos \theta$ de la projection antérieure.

25. Tomographe selon la revendication 11, caractérisé par le fait que les moyens de position comportent une mémoire morte (ROM 556), dans laquelle le sont mémorisées des données qui sont représentatives pour la fonction de position qui associe à chaque emplacement du tube image à mémoire (118) à une piste ou voie de rayonnement pour chaque angle de projection.

26. Tomographe selon la revendication 11, caractérisé par le fait qu'aux moyens (570) servant à la multiplication, sont reliés des moyens de facteurs de pondération (558, 562) qui forment les facteurs de pondération en fonction des données de moyens de transformation (550).

27. Tomographe selon la revendication 26, caractérisé par le fait que les moyens de facteurs de pondérations (558, 562) comprennent une mémoire morte (ROM, 558) à laquelle sont appliquées les données de coordonnées de projection provenant des moyens de transformation (550), et qu'en fonction de cela ladite mémoire fournit à sa sortie des données qui sont représentatives du facteur de pondération.

FIG1

FIG 1A

FIG 2

0 004 258

FIG 3

FIG 3A

Multiplexer 206

Gruppe 4-R
I-256 ... I-241
IC-4

Gruppe 1-R
I-160 ... I-145
I-144 ... I-129
IC-1

Gruppe 1-L
I-128 ... I-113
I-112 ... I-97

Gruppe 4-L
I-32 ... I-17
I-16 ... I-1
IC-4

Multiplexer 210
Multiplexer 208

A/D Wandler 216
A/D Wandler 214

Wandler 218

Σ 212
MD 1
MD 2
MD 3
MD 4

Steuerkreis 220
IC-1
IC-2
IC-3
IC-4

222

231
227
225
229

202
200

3

**FIG 4**

- Lesen — 250
- Bewichten — 251
- Offset-.Korrektur — 252
- Addition — 253
- Lesen — 254
- Strahlenaufhärtungskorrektur: Berechnung des Faktors — 255
- Berechnung der im Hinblick auf die Strahlenaufhärtung korrigierten Daten — 256
- Cosinus-Faktor-Korrektur — 257
- Speicherung — 258

FIG 5

FIG 6

FIG 6A

FIG 7

FIG 8

FIG 9

FIG 10

FIG 11

FIG 12

FIG 13

FIG 14

FIG 15

0 004 258

FIG 16

FIG 18

14

FIG 17

Rekombination

Komplementierkreis

Rekombination

Konversionskreis

Multiplizierer

ROM

Logik

Speicher

FIG 19

FIG 20

Datentreiber — 802
Steuerkreis — 910
Adressentreiber — 906
Adressenempfänger — 908
Datenempfänger — 904

942 — Dekoder

940 — Adressenregister

936 — Steuerkreise
936

Steuerkreis — 934

Register — 930

944

916 — Steuerpuffer — 914
Adressen empf. — 912
Datentreiber
918
922
920
Puffer — 932
928
924
926

Puffer- und Treiberkreise
zu und vom Faltungsrechner

Puffer- und Treiberkreise
zum Bildrekonstruktor 116

0 004 258